(19) Europäisches Patentamt
European Patent Office
Office européen des brevets

(11) Publication number: 0 492 136 A2

# (12) EUROPEAN PATENT APPLICATION

(21) Application number: **91119897.6**

(22) Date of filing: **22.11.91**

(51) Int. Cl.5: **C07K 5/06**, C07C 229/16, C07C 235/04, C07C 237/04, C07D 215/48, C07D 211/60, C07D 263/20, A61K 37/02, A61K 31/47, A61K 31/445, A61K 31/42

(30) Priority: **20.12.90 US 630915**

(43) Date of publication of application: **01.07.92 Bulletin 92/27**

(84) Designated Contracting States: **AT BE CH DE DK ES FR GB GR IT LI LU NL SE**

(71) Applicant: **AMERICAN CYANAMID COMPANY**
**1937 West Main Street P.O. Box 60**
**Stamford Connecticut 06904-0060(US)**

(72) Inventor: **Babine, Robert E.**
**30 Frederic Street**
**Nanuet, New York 10954(US)**
Inventor: **Zhang, Nan**
**8-0 Church**
**Valley Cottage, New York 10989(US)**
Inventor: **Schow, Steven R.**
**One Helmshill Road**
**Washingtonville, New York 10992(US)**
Inventor: **Jurgens, Alex Roger**
**831 Willow Grove Road**
**Westfield, New Jersey 07090(US)**

(74) Representative: **Wächtershäuser, Günter, Dr.**
**Tal 29**
**W-8000 München 2(DE)**

## (54) Retroviral protease inhibitors derived from 3-chloro-2-chloromethyl-1-propene.

(57) The invention provides compounds of the formula:

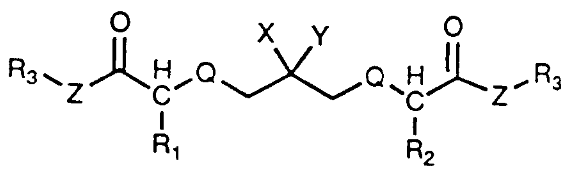

wherein Q, X, Y, Z, $R_1$, $R_2$ and $R_3$ are defined in the specifications useful as inhibitors of HIV protease enzyme.

EP 0 492 136 A2

## BACKGROUND OF THE INVENTION

### 1. FIELD OF INVENTION

The invention relates to novel compounds derived from 3-chloro-2-chloromethyl-1-propene which are useful as inhibitors of retroviral protease enzymes.

### 2.DESCRIPTION OF PRIOR ART

HIV-1 is a retrovirus which is believed to be responsible for the onset of AIDS and AIDS related complex. The life cycle of a retrovirus involves utilizing both the enzymatic functions of the host cell as well the enzymatic functions of virally encoded proteins for viral replication. Inhibitors of essential retroviral enzyme function have potential in the control of infection and preventing the onset of serious life threatening disease caused by the infection (Mitsuka et al., Science, 249,1533-44(1990)).

Retroviruses contain single stranded RNA as their genetic material. After initial retroviral infection, a series of essential viral enzymes (reverse transcriptase, RNase-H, and integrase) are responsible for transcribing the viral RNA into double stranded DNA and integrating this genetic material into the DNA of the host cell. Thus, once infected by a retrovirus, the infected cell's DNA and that of its progeny have viral genetic information associated with it. An infected cell is then capable of producing new viral RNA and protein using the host's enzymatic machinery. The retroviral protein is produced by the host as a series of large polyproteins which must be posttranslationally modified in order to produce a new virus (i.e., viral replication). Some of these modifications are accomplished via the host's enzymes while others are accomplished by virally encoded enzymes. One essential retroviral enzyme is a protease which is responsible for the processing of viral polyproteins into essential viral structural proteins and enzymes.

Of the retroviral proteases, HIV-1 protease has been studied in the greatest detail. This enzyme is responsible for selectively hydrolysing the HIV encoded gag and gag-pol polyproteins into the structural proteins that form the viral core as well as the essential viral enzymes, including the protease. Mutagenasis studies have shown that mutants which lack protease function are non-infectious (Kohl et al., Proc. Nat. Acad. Sci.,85, 4686-90(1988); Peng et al., J.Virol., 63, 2550(1989); Gottlinger et al., Proc. Nat. Acad. Sci., 86, 5781-5(1989); Seelmeier et al., Proc. Nat. Acad. Sci., 85, 6612-6(1988). Included in the studies on HIV-1 protease is the structural characterization of HIV-1 protease by X-ray crystallography. X-ray structures have been determined for both recombinant and synthetic proteins (Navia, et al., Nature, 337, 615-20(1989); Lapatto et al., Nature 342, 299-302 (1989); Wlodawer, et al., Science, 245, 616-21(1989); Miller et al., Science, 246, 1149-52(1989)). One of the groups involved in these structural studies, the NCI, has made their structural data available to the public via the Brookhaven Protein Data Base (PDB). (PDB entries: 1HVP,3HVP, 4HVP). The data 4HVP was provided by Dr. A. Wlodawer prior to its general release. These structural studies have shown that the protein is a C2 symmetrical dimer, and a member of the well known aspartic protease class of hydrolytic enzymes. It is likely that these two features may be characteristic of all retroviral proteases (Lapatto et al., Nature, 342, 299-302(1989); Wu et al., Arch. Bioch. Biophys., 277, 306-11(1990)).

Several groups have reported potent inhibitors of HIV-1 protease. (Dreyer et al., Proc. Nat. Acad. Sci, 86, 9752-6(1989); Meek et al., Nature, 343, 90-2(1990); Dreyer et al., EP 352000 (1990); Tomaselli et al., Biochem., 29, 264-9(1990); McQuade et al., Science, 247, 454-6(1990); Roberts et al., Science, 248, 358-61(1990); Handa et al., EP 346847 (1989); Sigal et al., 337714 (1989); Rich et al., J. Med. Chem., 33, 1285-8(1990), Kempf et al., EP 0,342,541; Fassler et al., EP 0,374,098; Fassler et al., EP 0,374,097; Schramm et al., WO 90/09191; Raddatz et al., EP 0,369,141; Ruger et al., EP 0,372,537; De et al., EP 0,365,992; Fung et al., EP 0,364,804, Vaca et al., EP 0,356,223; Hanko et al., EP 0,361,341). In each of these reports the inhibitors are related in that they are essentially an oligopeptide which consists of a mixture of natural and unnatural amino acids. Compounds having the general formula:

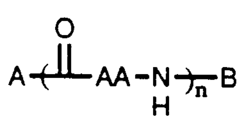

where A = C-terminal group, AA = amino acids(natural or unnatural), n = 1 to 8 and B = N-terminal group, have been reported by the above groups.

Another group, (Erickson et al., Science, 249, 527-33(1990); Kempf et al., J. Med. Chem., 33, 2687-9-

(1990)) has reported two series of compounds, which are pseudosymmetrical inhibitors of HIV-1 protease. These compounds are not related to the oligopeptide compounds above and have the structures shown below. The synthesis of these compounds, however, involves using amino acid derived central units.

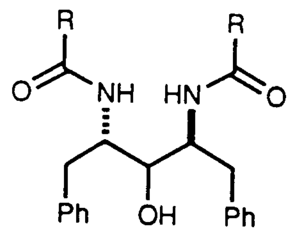

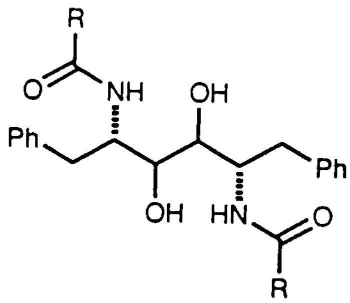

Some of these groups have shown that inhibitors of the HIV-1 protease enzyme are effective at producing non-infectious virus in cell culture. In addition one group has shown that inhibitors of HIV-1 protease are also inhibitors of HIV-2 protease.

## SUMMARY OF THE INVENTION

Compounds of the formula:

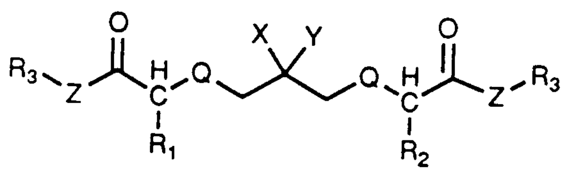

wherein:

Q is the same or different and is selected from a single bond or $NR_9$ or $NR_{10}$; wherein:

$R_9$ = H, $C_1$ - $C_7$ straight or branched alkyl; $-(CH_2)_n$ cyclic $C_3$ - $C_7$ alkyl, n = 0-4; $-(CH_2)_n$-phenyl, n = 0-4; or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; or $R_9$ and $R_2$ taken together are $-(CH_2)_m-$, m = 3 or 4; or $R_9$ and $R_1$ taken together are $-(CH_2)_m-$, m = 3 or 4; or $R_9$ and $R_2$ taken together are

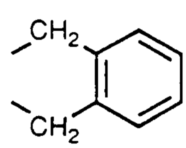

or $R_9$ and $R_1$ taken together are

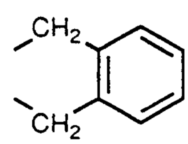

$R_{10}$ = H, $C_1$ - $C_7$ straight or branched alkyl; $-(CH_2)_n$ cyclic $C_3$ - $C_7$ alkyl, n = 0-4; $-(CH_2)_n$-phenyl, n = 0-4; or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; or $R_{10}$ and $R_2$ taken together are $-(CH_2)_m-$, m = 3 or 4; or $R_{10}$ and $R_1$ taken together are $-(CH_2)_m-$, m = 3 or 4; or $R_{10}$ and $R_2$ taken together are

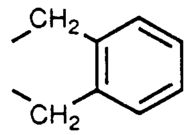

or $R_{10}$ and $R_1$ taken together are

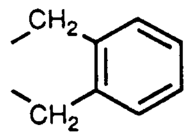

wherein:

Z = O or NH; X = OH and Y = H or $CH_2OH$ or X and Y taken together may be an epoxide $-(CH_2O)-$;

$R_1$ = H, $C_1$ - $C_7$ straight or branched alkyl; $-(CH_2)_n$ cyclic $C_3$ - $C_7$ alkyl, n = 0-4; $-(CH_2)_n$-phenyl, n = 0-4; or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl, or phenyl;

$R_2$ = H, $C_1$ - $C_7$ straight or branched alkyl; $-(CH_2)_n$ cyclic $C_3$ - $C_7$ alkyl, n = 0-4; $-(CH_2)_n$-phenyl, n = 0-4; or $-(CH_2)_n$-substituted phenyl,

where n = 0 - 4 and substituted with F,Cl,Br,I, $C_1$ - $C_4$ alkoxide, $C_1$ - $c_6$ straight, branched or cyclic alkyl or phenyl;

$R_3$ =

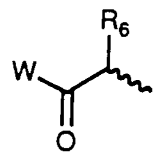

$C_1$ - $C_{10}$ straight or branched alkyl; $-(CH_2)_n$-cyclic $C_3$ - $C_7$ alkyl, n = 0 - 4; or $-(CH_2)_n$ -substituted cyclic $C_3$ - $C_7$ alkyl, where n = 0 - 4 and substituted with $C_1$ - $C_7$ straight, branched or cyclic alkyl, or $NHCOR_4$;

wherein:

$R_4$ = $UR_5$; U = a single bond, oxygen or NH; $R_5$ = $C_1$ - $C_8$ straight or branched alkyl; $-(CH_2)_n$ cyclic $C_3$ - $C_7$ alkyl, n = 0-4; $-(CH_2)_n$-phenyl, n = 0 - 4; $(CH_2)_n$ substituted phenyl, where n = 0 - 4 and substituted with F,Cl,Br,I, $C_1$ - $C_4$ alkoxide, $C_1$ - $C_6$ straight, branched or cyclic alkyl; $-(CH_2)_n$ 1-napthyl, n = 0 - 4; $(CH_2)_n$ 1-substituted napthyl, where n = 0 - 4 and substituted with F,Cl,Br,I, $C_1$ - $C_4$ alkoxide, $C_1$ - $C_6$ straight, branched or cyclic alkyl; $-(CH_2)_n$ 2-napthyl, n = 0 - 4; $-(CH_2)_n$ 2-substituted napthyl, where n = 0 - 4 and substituted with F,Cl,Br,I, $C_1$ - $C_4$ alkoxide, $C_1$ - $C_6$ straight, branched or cyclic alkyl; $-(CH_2)_n$ 2-quinoyl, n = 0 - 4; $-(CH_2)_n$ 2-substituted quinoyl, where n = 0 - 4 and substituted with F,Cl,Br, I, $C_1$ - $C_4$ alkoxide, $C_1$ -$C_6$ straight, branched or cyclic alkyl; $-(CH_2)_n$ heterocycle, where n = 0-4 and heterocycle is defined as a stable 5- to 7-membered mono- or bicyclic or a stable 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and include any bicyclic group in which any of the above defined heterocycles is fused to a benzene ring. The heterocyclic ring may be attached to any heteroatom or carbon atom which results in the creation of a stable structure. Examples of such heterocycles include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, isoxazoyl, isoxazoyl, isoxazolidinyl, morpolinyl, thiazolyl, thiazolidinyl, isothiazolyl, quinuclidinyl, isothiazolidinyl, indolyl, quinolinyl, isoquinolinyl, benzimidazolyl, quinuclidinyl, isothiazolidinyl, indolyl, quinolinyl, isoquinolinyl, benzimidazolyl, thiadiazolyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone and oxadiazolyl; $-(CH_2)_n$ substituted heterocycle, where n = 0-4, heterocycle is as defined above and substituted with F,Cl,Br,I, $C_1$ -$C_6$ alkoxide, $C_1$ - $C_6$ straight, branched or cyclic alkyl;

$R_6$ = $C_1$ - $C_7$ straight or branched alkyl; $(CH_2)_2CONH_2$,$CH_2CONH_2$, $CH_2OH$, or $CH(CH_3)OH$;

W =

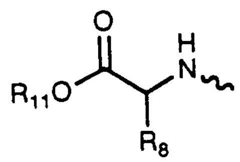

or $OR_7$ or $NHR_7$, wherein: $R_7$ = $C_1$ - $C_8$ straight or branched alkyl; $-(CH_2)_n$ cyclic $C_3$ - $C_7$ alkyl, n = 0-4; $-(CH_2)_n$-phenyl n = 0 - 4; $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; $-(CH_2)_n$ heterocycle, where n = 0-4 and heterocycle is as defined before; $-(CH_2)_n$ substituted heterocycle, where n = 0-4, heterocycle is as defined before and substituted with F,Cl,Br,I, $C_1$ - $C_4$ alkoxide, $C_1$ - $C_6$ straight, branched or cyclic alkyl;
wherein:
$R_8$ = $C_1$ - $C_7$ straight or branched alkyl; $-(CH_2)_n$ cyclic $C_3$ - $C_7$ alkyl, n = 0-4; $-(CH_2)_n$ -phenyl, n = 0 - 4; $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; or $-(CH_2)_n-N=C(NH_2)_2$, n = 0 - 4;
$R_{11}$ = $C_1$ - $C_8$ straight, branched or cyclic alkyl; $-(CH_2)_n$-phenyl, n = 0 - 4, or $(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; are useful as inhibitors of the function of HIV protease.

Novel methods of making the compounds described above are also provided as are novel methods of using the compounds in the inhibition of HIV protease enzyme and pharmaceutical compositions containing the compounds. Also disclosed are novel compounds useful as intermediates for preparing the HIV protease inhibiting compounds of the present invention.

## DESCRIPTION OF THE PREFERRED EMBODIMENTS

The retroviral protease inhibiting compounds of the present invention are prepared as follows.

An important intermediate in the preparation of the compounds of this invention are compounds of the general formula 1 or ester derivatives thereof.

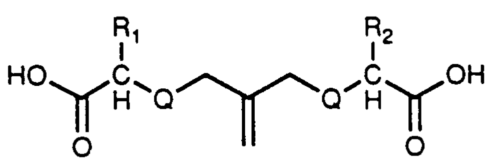

**1**

Compounds of formula 1 are prepared as described below. Commercially available 3-chloro-2-chloromethyl-1-propene is converted into diiodide 2 upon treatment with sodium iodide in acetone.

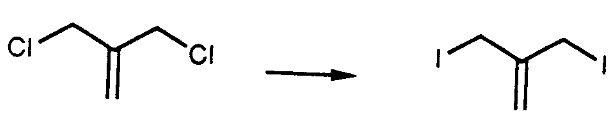

**2**

Commercially available (S)-(-)-4-benzyl-2-oxazolidinone is converted into substituted oxazolidinones of formula 3 upon treatment with base and acid chlorides of the formula $R_1CH_2COCl$ following the methodology of Evans et al., (J. Am. Chem. Soc., 104, 1737-9(1982)).

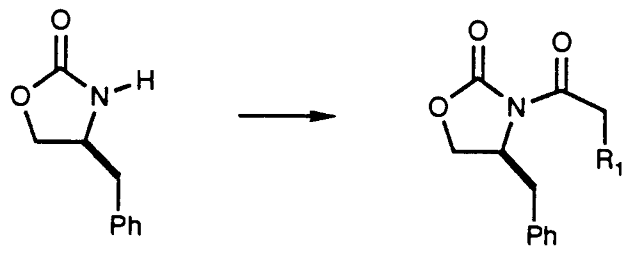

3

Other oxazolidinones of the general formula:

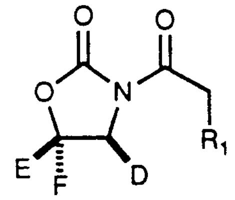

in which D = $C_1$ - $C_7$ alkyl; $-(CH_2)_n$-phenyl, n = 0 - 4; E and F are the same or different and selected from H, $C_1$ - $C_7$ alkyl and $-(CH_2)_n$-phenyl, n = 0 - 4, are equally effective as alternatives to 3. Prolinol derived amides of the general formula:

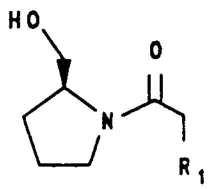

are also useful as alternatives to 3.

The anion of 3 is prepared by treatment of 3 with strong base, eq $NaN(TMS)_2$ , in which TMS = trimethylsilyl, at temperature < -70°C. When compound 2 is reacted with an excess of the anion of 3 at low temperatures, compounds of the formula 4 are produced. When the reaction is performed under these conditions $R_1 = R_2$.

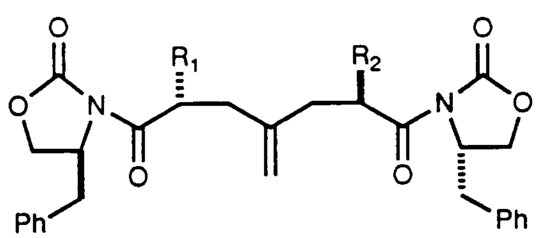

4

When an excess of compound 2 is reacted with the anion of 3 at low temperatures, compounds of formula 5 are produced. When compounds of formula 5 are reacted with an excess of the anion of 3 at low temperatures, compounds of formula 4 are produced. When the reaction is performed under these conditions, $R_1$ and $R_2$ can be either the same or different.

Treatment of compounds of formula 4 with salts of benzyl alcohol produce benzyl esters of formula 6, which are esters derived from acids of formula 1 where Q = a single bond. The chemistry used to prepare compounds of formula 6 is compatible with a wide variety of $R_1$ and $R_2$ groups. Specifically, compounds of formula 6 in which $R_1$ = $C_1$ - $C_7$ straight or branched alkyl; $-(CH_2)_n$ cyclic $C_3$ - $C_7$ alkyl, n = 0-4; $-(CH_2)_n$-

phenyl n = 0-4 or $(CH_2)_n$-substituted phenyl, where n = 0 to 4 and substituted with F,Cl,Br,I, $C_1$ - $C_4$ alkoxide, $C_1$ - $C_6$ straight, branched or cyclic alkyl, or phenyl;
$R_2$ = $C_1$ - $C_7$ straight or branched alkyl; $-(CH_2)_n$ cyclic $C_3$ - $C_7$ alkyl, n = 0-4; $-(CH_2)_n$-phenyl, n = 0-4 or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl, or phenyl; are prepared by this method.

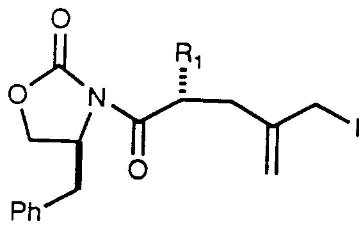

5

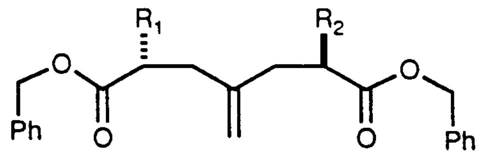

6

When compounds of formula 5 are reacted with secondary amines of formula 7 (A = O-$C_1$ - $C_7$ alkyl or O-$C_1$ - $C_7$ araalkyl), compounds of formula 8 are formed. Treatment of compounds of formula 8 with salts of benzyl alcohol product benzyl esters of formula 9, which are esters derived from acids of formula 1 where one Q = a single bond and the other Q = $NR_9$ . The chemistry used to prepare compounds of formula 9 is compatible with a wide variety of $R_1$, $R_2$ and $R_9$ groups. Specifically, compounds of formula 9 in which $R_1$ = $C_1$ -$C_7$ straight or branched alkyl; $-(CH_2)_n$ cyclic $C_3$ - $C_7$ alkyl, n = 0-4; $-(CH_2)_n$-phenyl, n = 0-4 or $-(CH_2)-_n$-substituted phenyl, where n = 0 - 4 and substituted with F,Cl,Br,I, $C_1$ - $C_4$ alkoxide, $C_1$ - $C_6$ straight, branched or cyclic alkyl, or phenyl;
$R_2$ = H, $C_1$ - $C_7$ straight or branched alkyl; $-(CH_2)_n$ cyclic $C_3$ - $C_7$ alkyl, n = 0-4; $-(CH_2)_n$-phenyl, n = 0-4 or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl, or phenyl;
$R_9$ = H, $C_1$ - $C_7$ straight or branched alkyl; $-(CH_2)_n$ cyclic $C_3$ - $C_7$ alkyl, n = 0-4; $-(CH_2)_n$-phenyl n = 0-4 or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; or $R_9$ and $R_2$ taken together are $-(CH_2)_m-$, m = 3 or 4; or $R_9$ and $R_2$ taken together are

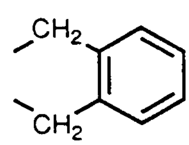

are prepared by this method.

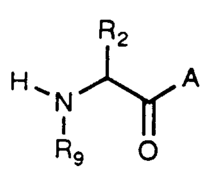

7

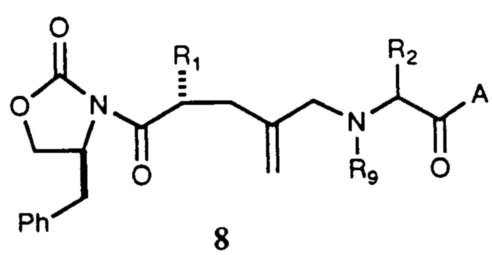

8

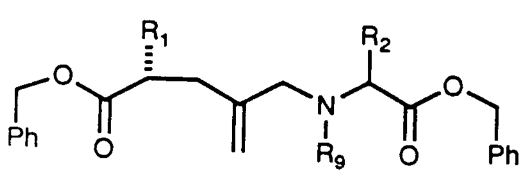

9

When compound 2 is reacted with an excess of secondary amines of formula 7, compounds of formula 10 are produced. When the reaction is performed under these conditions, $R_1 = R_2$ and $R_9 = R_{10}$. When an excess of compound 2 is reacted with a secondary amines of formula 7, followed by treatment with an excess of a different secondary amine of formula 7, compounds of formula 10 are produced in which $R_1$ and $R_2$ can be either the same or different and $R_9$ and $R_{10}$ can be either the same or different. Compounds of formula 10 are esters derived from acids of formula 1 where Q = same or different and selected from $NR_9$ or $NR_{10}$. The chemistry used to prepare compounds of formula 10 is compatible with a wide variety of $R_1$, $R_2$, $R_9$ and $R_{10}$ groups. Specifically, compounds of formula 10 in which

$R_1$ = H, $C_1$ - $C_7$ straight or branched alkyl; $-(CH_2)_n$ cyclic $C_3$ - $C_7$ alkyl, n = 0-4; $-(CH_2)_n$-phenyl n = 0-4 or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl, or phenyl;

$R_2$ = H, $C_1$ - $C_7$ straight or branched alkyl; $-(CH_2)_n$ cyclic $C_3$ - $C_7$ alkyl, n = 0-4; $-(CH_2)_n$-phenyl n = 0-4 or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl, or phenyl;

$R_9$ = H, $C_1$ - $C_7$ straight or branched alkyl; $-(CH_2)_n$ cyclic $C_3$ - $C_7$ alkyl, n = 0-4; $-(CH_2)_n$-phenyl, n = 0-4 or $-(CH_2)_n$-substituted phenyl, where n = 0 to 4 and substituted with F,Cl,Br,I, $C_1$ - $C_4$ alkoxide, $C_1$ - $C_6$ straight, branched or cyclic alkyl; or $R_9$ and $R_1$ taken together are $-(CH_2)_m-$, m = 3 or 4; or $R_9$ and $R_1$ taken together are

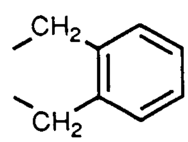

$R_{10}$ = H, $C_1$ - $C_7$ straight or branched alkyl; $-(CH_2)_n$ cyclic $C_3$ - $C_7$ alkyl, n = 0-4; $-(CH_2)_n$-phenyl, n = 0-4 or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; or $R_{10}$ and $R_2$ taken together are $-(CH_2)_m-$, m = 3 or 4; or $R_{10}$ and $R_2$ taken together are

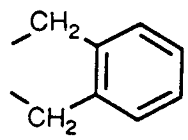

are prepared by this method.

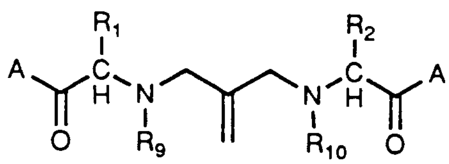

**10**

Compounds of formula 1 are then prepared from their corresponding esters (6, 9 and 10) by standard saponification methods.

When compounds of formula 1 are reacted with amines of formula $R_3NH_2$ in the presence of BOP, HOBT and triethylamine, bisamides of formula 11 are produced, where BOP = benzotriazol-1-yloxytris-(dimethylamino)phosphoniumhexafluorophosphate sequalog and HOBT = 1-hydroxybenzotriazole.

Other methods for formation of amide bonds, including DCC mediated coupling, are applicable, where DCC = 1,3-dicyclohexylcarbodiimide. A wide variety of $R_3$ groups are compatible with the above chemistry. Specifically, compounds of formula 11 in which $R_3$ = $C_1$ - $C_{10}$ straight or branched alkyl; $-(CH_2)_n$-cyclic $C_3$ - $C_7$ alkyl, n = 0 - 4 or $-(CH_2)_n$-substituted cyclic $C_3$ - $C_7$ alkyl, where n = 0 - 4 and substituted with $C_1$ - $C_7$ straight, branched or cyclic alkyl, $NH_2$ or $NHCOR_4$; wherein: $R_4$ = $UR_5$, U = a single bond, oxygen or NH; $R_5$ = $C_1$ - $C_5$ straight or branched alkyl; $-(CH_2)_n$ cyclic $C_3$ - $C_7$ alkyl, n = 0 - 4; $-(CH_2)_n$-phenyl, n = 0 - 4;

$-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; $-(CH_2)_n$ 1-napthyl, n = 0 - 4; $-(CH_2)_n$ 1-substituted napthyl, where n = 0 - 4 and substituted with F,Cl,Br,I, $C_1$ - $C_4$ alkoxide, $C_1$ -$C_6$ straight, branched or cyclic alkyl; $-(CH_2)_n$ 2-napthyl, n = 0 - 4; $-(CH_2)_n$ 2-substituted napthyl, where n = 0 - 4 and substituted with F,Cl,Br,I, $C_1$ - $C_4$ alkoxide, $C_1$ - $C_6$ straight, branched or cyclic alkyl; $-(CH_2)_n$ 2-quinoyl, n = 0 - 4; $-(CH_2)_n$ 2-substituted quinoyl, where n = 0 - 4 and substituted with F,Cl,Br,I, $C_1$ - $C_4$ alkoxide, $C_1$ - $C_6$ straight, branched or cyclic alkyl; $-(CH_2)_n$ heterocycle, where n = 0-4 and heterocycle is defined as a stable 5- to 7-membered mono- or bicyclic or a stable 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and include any bicyclic group in which any of the above defined heterocycles is fused to a benzene ring; the heterocyclic ring may be attached to any heteroatom or carbon atom which results in the creation of a stable structure; examples of such heterocycles include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazoyl, imidazolinyl, imidiazolidinyl, pyridyl, pyrazinyl, pyrimidinyl, pyridazinyl, oxazolyl, oxazolidinyl, indanyl, isoxazoyl, isoxazoyl, isoxazolidinyl, morpolinyl, thiazolyl, thiazolidinyl, isothiazolyl, quinuclidinyl, isothiazolidinyl, indolyl, quinolinyl, isoquinolinyl, benzimidazolyl, quinuclidinyl, isothiazolidinyl, indolyl, quinolinyl, isoquinolinyl, benzimidazolyl, thiadiazolyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone and oxadiazolyl; $-(CH_2)_n$ substituted heterocycle, where n = 0-4, heterocycle is as defined above and substituted with F,Cl,Br,I, $C_1$ -$C_6$ alkoxide, $C_1$ - $C_6$ straight, branched or cyclic alkyl; are prepared by this method.

Compounds of formula 11 in which $R_3$ = $-(CH_2)_n$ -substituted cyclic $C_3$ - $C_7$ alkyl, where n = 0 - 4 and substituted with $NHCOR_4$, where $R_4$ is as described previously, are prepared upon treatment of compounds of formula 11 in which $R_3$ = $-(CH_2)_n$ -substituted cyclic $C_3$ - $C_7$ alkyl, where n = 0 - 4 and substituted with $NH_2$, with acids of formula $R_5COOH$ in the presence of BOP, HOBT and triethylamine, or with acids of formula $R_5COOH$ in the presence of DCC or with acid chlorides of formula $R_4COCl$ in the presence of base.

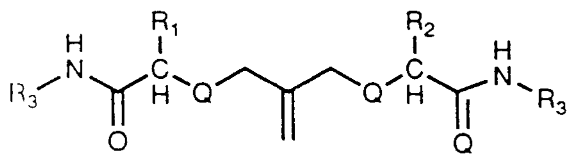

**11**

When compounds of formula 1 are reacted with amines of formula 12, in the presence of BOP, HOBT and triethylamine, bisamides of formula 13 are produced. Other methods for formation of amide bonds, including DCC mediated coupling, are applicable. A wide variety of $R_6$ and W groups are compatible with the above chemistry. Specifically, compounds of formula 13 in which $R_6$ = $C_1$ - $C_7$ straight or branched alkyl; $-(CH_2)_n$ cyclic $C_3$ - $C_7$ alkyl, n = 0 - 4; $(CH_2)_2CONH_2$,$CH_2CONH_2$, $CH_2OH$, or $CH(CH_3)OH$; W = $OR_7$ or $NHR_7$, wherein:

$R_7$ = $C_1$ - $C_8$ straight or branched alkyl; $-(CH_2)_n$ cyclic $C_3$ - $C_7$ alkyl, n = 0 - 4; $-(CH_2)_n$-phenyl, n = 0 - 4, $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; $-(CH_2)_n$ heterocycle, where n = 0-4 and heterocycle is as defined before; $-(CH_2)_n$ substituted heterocycle, where n = 0-4, heterocycle is as defined before and substituted with F,Cl,Br,I, $C_1$ - $C_4$ alkoxide, $C_1$ - $C_6$ straight, branched or cyclic alkyl; are prepared by this method.

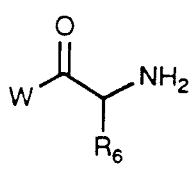

**12**

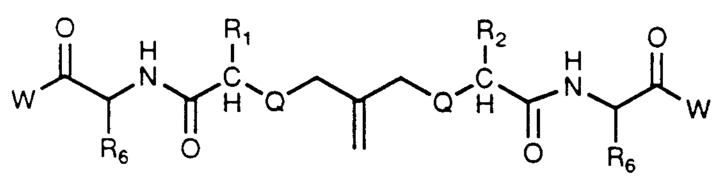

**13**

When compounds of formula 1 are reacted with amines of formula 14 in the presence of BOP, HOBT and triethylamine bisamides of formula 15 are produced. Other methods for formation of amide bonds, including DCC mediated coupling, are applicable. A wide variety of $R_6$, $R_8$ and $R_{11}$ groups are compatible with the above chemistry. Specifically, compounds of formula 15, in which $R_6$ = $C_1$ - $C_7$ straight, branched or cyclic alkyl; $(CH_2)_2CONH_2$, $CH_2CONH_2$, $CH_2OH$, or $CH(CH_3)OH$;

$R_8$ = $C_1$ - $C_7$ straight or branched alkyl; $-(CH_2)_n$ cyclic $C_3$ - $C_7$ alkyl, n= 0 - 4; $-(CH_2)_n$-phenyl, n= 0 - 4, $-(CH_2)_n$-substituted phenyl, where n= 0 - 4 and substituted with F,Cl,Br,I, $C_1$ - $C_4$ alkoxide, $C_1$ - $C_6$ straight, branched or cyclic alkyl; or $-(CH_2)_n-N=C(NH_2)_2$, n= 0 - 4;

$R_{11}$ = $C_1$ - $C_8$ straight, branched or cyclic alkyl; $-(CH_2)_n$-phenyl, n= 0 - 4 or $-(CH_2)_n$-substituted phenyl, where n= 0 - 4 and substituted with F,Cl,Br,I, $C_1$ - $C_4$ alkoxide, $C_1$ - $C_6$ straight, branched or cyclic alkyl are prepared by this method.

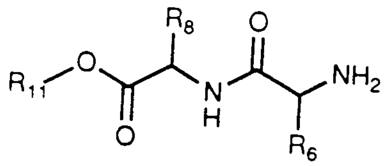

14

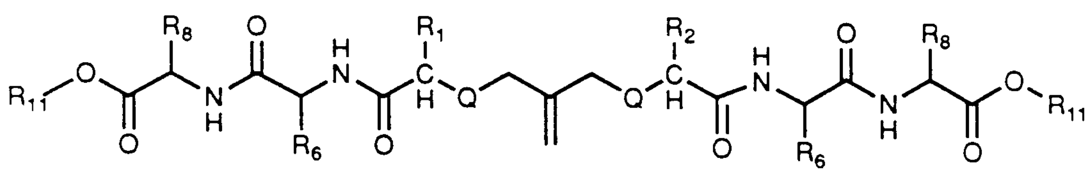

15

In the above methods of preparation, the $R_6$, $R_8$ and $R_{11}$ groups are present in an amino acid derived starting material. All the natural and many unnatural amino acids are commercially available. Other amino acids can be prepared synthetically by a wide variety of methods (reviewed in a recent monograph by R.M. Williams, "Synthesis of Optically Active $\alpha$-Amino Acids", Pergamon Press, Oxford 1989) and are thus considered readily available starting materials.

Compounds of formula 11, 13 and 15 are converted into their corresponding epoxides 16, 17 and 18, respectively, upon treatment with m-chloroperbenzoic acid in methylene chloride at 0°C.

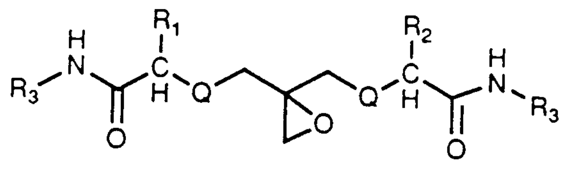

16

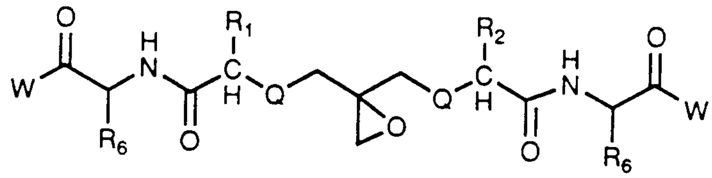

17

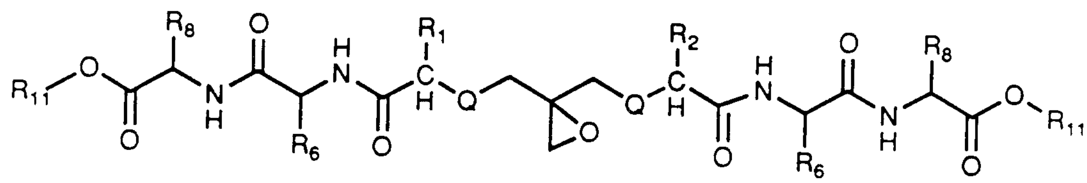

18

Compounds of formula 11, 13 and 15 are converted into their corresponding diols 19, 20 and 21, respectively, upon treatment with osmium tetroxide.

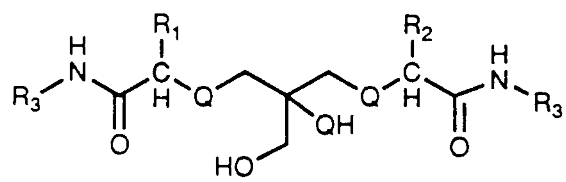

19

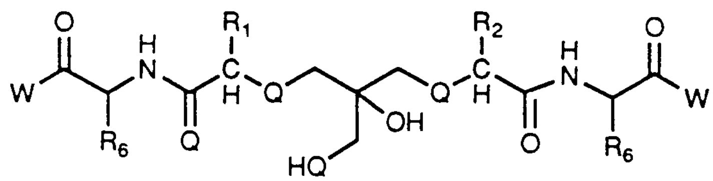

20

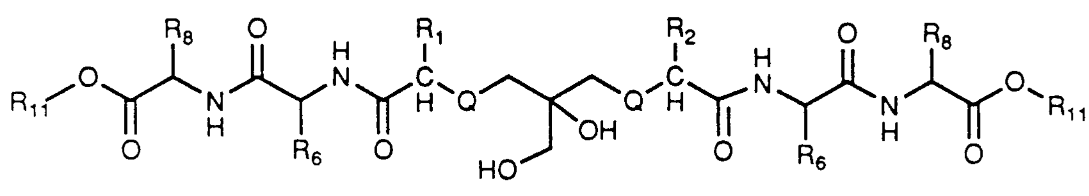

21

Diols of formula 19, 20 and 21, upon treatment with sodium periodate, are converted into intermediate ketones 22, 23 and 24, respectively, which are reduced upon treatment with sodium borohydride to give alcohols 25, 26 and 27, respectively. Alternatively, treatment of 11, 13 and 15 with osmium tetroxide in the presence of sodium periodate gives intermediate ketones 22, 23 and 24, respectively, which are reduced with sodium borohydride to give 25, 26 and 27, respectively.

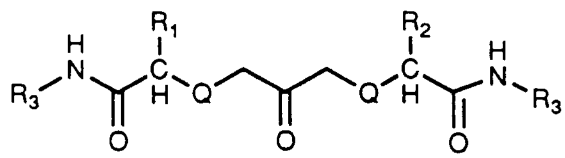

**22**

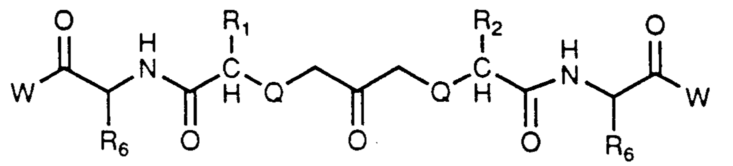

**23**

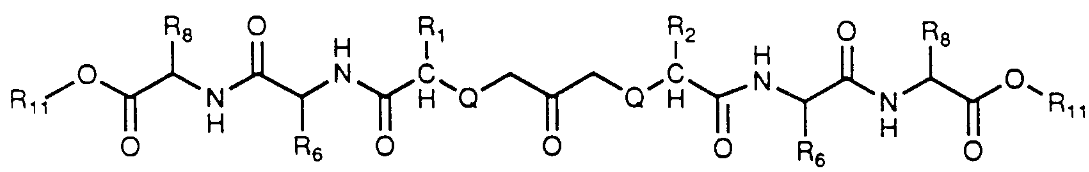

**24**

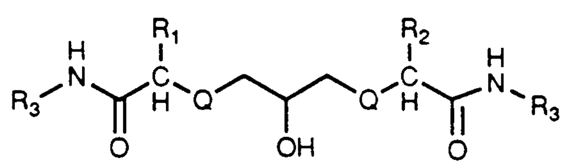

**25**

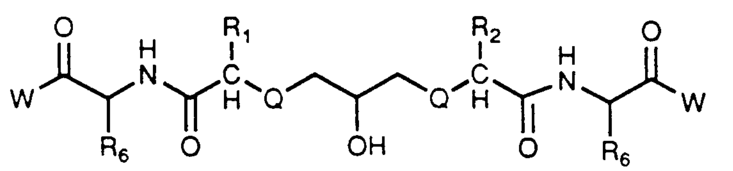

**26**

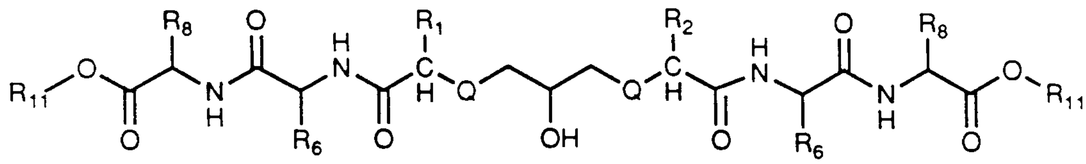

**27**

The in vitro inhibition of HIV-1 protease by the compounds provided by the present invention is demonstrated by means of the following test:

HIV-1 protease is available for purchase from Medigenics, 701 Mercy Rd., Suite 420, Omaha, Nebraska

68106. Protease activity is assayed using the octapeptide H-Val-Ser-Gln-Asn-Tyr-Pro-Ile-Val-OH as the substrate. Cleavage of the substrate is quantitated by measuring the production of H-Val-Ser-Gln-Asn-Tyr-OH by HPLC analysis by the method described by Tomaselli et al., (cited previously). The lower the $IC_{50}$ value, the more potent the compound.

Table 1

| Compound from Example # | IC50(μM) |
|---|---|
| 9 | 0.8 |
| 11 | 0.03 |
| 12 | 10.0 |
| 18 | 0.1 |
| 20 | 0.001 |
| 26 | 0.2 |
| 31 | 0.001 |
| 33 | 0.03 |
| 36 | 0.1 |
| 39 | 0.01 |
| 42 | 50.0 |
| 46 | 0.8 |
| 49 | 0.001 |
| 52 | 5.0 |
| 53 | 100.0 |
| 54 | 100.0 |
| 55 | 100.0 |
| 58 (Isomer A) | >1000.0 |
| 58 (Isomer B) | 100.0 |
| 60 | 50.0 |
| 323 | 0.1 |
| 324 | 0.1 |
| 331 | 0.5 |
| 110 | 0.01 |
| 328 | 0.005 |
| 327 | 0.1 |
| 329 | 0.005 |
| 330 | 0.1 |
| 326 | 0.1 |
| 325 | 0.1 |

Compounds are also tested for their ability to act as anti-HIV-1 compounds in tissue culture. The tests are performed in H9 based tissue culture challenged with $HTLV\text{-}III_B$ strain of HIV-1. The assay consists of evaluating the percent inhibition of supernatent p24 antigen concentrations relative to infected control cultures, as well as percent viable cells at 20 μg/ml in uninfected cells.

Table 2

| Compound from Example # | % Inhibition p24 (μg/ml) | | | % Viab. |
|---|---|---|---|---|
| | 20 | 2 | .2 | |
| 18 | 93 | 52 | 41 | >90 |
| 20 | 100 | 63 | 7 | >90 |
| 33 | 97 | 59 | 56 | 80 |
| 11 | 99 | 60 | 75 | ND |
| 52 | 90 | 0 | 0 | ND |
| 55 | 0 | 0 | 0 | ND |
| 330 | 0 | 0 | 0 | ND |
| 326 | 3 | 0 | 0 | ND |
| ND = not determined | | | | |

In Table 3 data is shown for the ability of these compounds to inhibit growth of HIV-1 strain IIIB in T lymphoid MT-2 cell lines. The endpoint of these experiments after 5 days is CPE (cell death) measured with the metabolic MTT dye.

Table 3

| Compound from Example # | % Inhibition at Concentration (μg/ml) | |
|---|---|---|
| | 2.5 | .625 |
| 329 | 97.9 | 92.5 |
| 49 | 104.1 | 11.3 |
| 31 | 93.5 | 31.7 |
| 33 | 54.1 | 23.7 |
| 325 | 3.8 | 0.0 |
| 323 | 3.1 | 0.2 |
| 328 | 72.8 | 17.1 |
| 110 | 58.7 | 16.6 |
| 327 | 3.2 | 3.2 |

As can be seen from the data presented hereinabove in Tables 1, 2 and 3, the compounds of the present invention are potent inhibitors of the HIV-I protease enzyme and thus are useful in treating mammals infected with retroviruses, especially HIV-1. The present invention also includes combinations of the HIV protease inhibitory compounds with one or more other agents useful in the treatment of AIDS.

When the compounds are employed for the above utility, they can be combined with one or more pharmaceutically acceptable carriers, for example, solvents, diluents and the like, and may be administered orally in such forms as tablets, capsules, dispersible powders, granules, or suspensions containing, for example, from about 0.05 to 5% of suspending agent, syrups containing, for example from about 10 to 50% of sugar, and elixirs containing, for example, from about 20 to 50% ethanol, and the like, or parenterally in the form of sterile injectable solutions or suspensions containing from about 0.05 to 5% suspending agent in an isotonic medium. Such pharmaceutical preparations may contain, for example, from about 0.05 up to about 90% of the active ingredient in combination with the carrier, more usually between about 5% and 60% by weight.

An effective amount of compound from 0.2 mg/kg of body weight to 100.0 mg/kg of body weight should be administered one to five times per day via any topical route of administration including but not limited to oral, parenteral (including subcutaneous, intravenous, intramuscular, intrasternal injection or infusion techniques), by inhalation spray, or rectally, in dosage unit formulations containing conventional non-toxic pharmaceutically acceptable carriers, adjuvants and vehicles. It will be understood, however, that the specific dose level and frequency of dosage for any particular patient may be varied and will depend upon a variety of factors including the activity of the specific compound employed, the metabolic stability and

length of action of that compound, the age, body weight, general health, sex, diet, mode and time of administration, rate of excretion, drug combination, the severity of the particular condition, and the host undergoing therapy.

These active compounds may be administered orally as well as by intravenous, intramuscular, or subcutaneous routes. Solid carriers include starch, lactose, dicalcium phosphate, microcrystalline cellulose, sucrose and kaolin, while liquid carriers include sterile water, polyethylene glycols, non-ionic surfactants and edible oils such as corn, peanut and sesame oils, as are appropriate to the nature of the active ingredient and the particular form of administration desired. Adjuvants customarily employed in the preparation of pharmaceutical compositions may be advantageously included, such as flavoring agents, coloring agents, preserving agents, and antioxidants, for example, vitamin E, ascorbic acid, BHT and BHA.

The preferred pharmaceutical compositions from the stand-point of ease of preparation and administration are solid compositions, particularly tablets and hard-filled or liquid-filled capsules. Oral administration of the compounds is preferred.

These active compounds may also be administered parenterally or intraperitoneally. Solutions or suspensions of these active compounds as a free base or pharmacologically acceptable salt can be prepared in water suitably mixed with a surfactant such as hydroxypropylcellulose. Dispersions can also be prepared in glycerol, liquid polyethylene glycols and mixtures thereof in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases, the form must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (e.g., glycerol, propylene glycol and liquid polyethylene glycol), suitable mixtures thereof, and vegetable oil.

The invention will be more fully described in conjunction with the following specific examples which are not to be construed as limiting the scope of the invention.

Example 1

## (S)-3-(1-Oxohexyl)-4-(phenylmethyl)-2-oxazolidinone

To a -78°C solution of 1.77 g of (S)-(-)-4-benzyl-2-oxazolidinone in 30 ml of dry tetrahydrofuran is added, dropwise, 4 ml of 2.5 M n-butyllithium. The mixture is stirred for 15 minutes at -78°C followed by the dropwise addition of 1.39 ml of hexanoyl chloride. The reaction is warmed to room temperature, quenched with water, extracted with ethyl acetate and concentrated *in vacuo*. The residue is purified by column chromatography to give 2.04 g of product as a colorless oil.

$^1$H NMR ($CDCl_3$): δ 0.93(t,3H); 1.38(m,4H); 1.71(m,2H); 2.77(dd,1H); 2.93(m,2H); 3.31(dd,1H); 4.19(m,2H); 4.68(m,1H); 7.21-7.38(m,5H).

D.A. Evans et al., JACS, 103, 2127 (1981).

Example 2

## [R-(R*,R*)]-2,6-Dibutyl-4-methyleneheptanedioic acid bis(phenylmethyl)ester

To a -78°C solution of 1.32 g of product from Example 1 in 40 ml of dry tetrahydrofuran is added, dropwise, 4 ml of 1 M sodium bis(trimethylsilyl)amide. The mixture is stirred for 10 minutes followed by the dropwise addition of 0.248 g of 3-iodo-2-iodomethyl-1-propene in 5 ml of dry tetrahydrofuran. The reaction is stirred at -78°C for 30 minutes, 0.8 ml of sodium bis(trimethylsilyl)amide is added and the reaction is maintained at -20°C for 96 hours. The reaction is quenched with a solution of saturated ammonium chloride, extracted with ethyl acetate and concentrated *in vacuo*. The residue is purified by column chromatography. The above product in 8 ml of dry tetrahydrofuran is added, dropwise, to a 0°C solution of 0.869 g of benzyl

alcohol and 2.4 ml of 2.5 M n-butyllithium. The reaction is stirred at 0°C for 1 hour, quenched with saturated ammonium chloride and extracted with ethyl acetate. The organic extract is dried and concentrated in vacuo to give 0.239 g of the desired product.
MS(FAB): m/z 465 (M+H).
$^1$H NMR ($CDCl_3$): δ 0.85(t,6H); 1.25(m,8H); 1.46(m,2H); 1.59(m,2H); 2.14(dd,2H); 2.33(dd,2H); 2.58(m,2H); 4.75(s,2H); 5.08(s,4H); 7.25-7.34(m,10 H).
The 3-iodo-2-iodomethyl-1-propene used in this example was prepared from 3-chloro-2-chloromethyl-1-propene as described by P.S. Skell; R.G. Doerr, JACS, 89, 4688 (1967).
D.A. Evans et al., JACS, 104, 1737 (1982).

Example 3

## N-L-alanyl-L-alanine methyl ester monohydrochloride

A mixture of 1.6 g of N-L-alanyl-L-alanine, 3 ml of chlorotrimethylsilane and 200 ml of methyl alcohol is stirred at room temperature for 18 hours. Concentration in vacuo gives 1.76 g of the desired product as a white solid.
$^1$H NMR ($CDCl_3$): δ 1.45(d,3H); 1.65(d,3H); 3.72(s,3H); 4.46(m,2H); 8.15(br s,2H); 8.54(br s,1H).

Example 4

## N-L-Valyl-L-valine methyl ester monohydrochloride

A mixture of 1.0 g of N-L-valyl-L-valine, 1.5 ml of chlorotrimethylsilane and 100 ml of methyl alcohol is stirred at room temperature for 20 hours. Concentration in vacuo gives 1.03 g of the desired product.
$^1$H NMR ($CDCl_3/CD_3OD$): δ 0.99-1.10(m,12H); 2.12(m,2H); 2.92(br s,3H); 3.74(s,3H); 3.92(d,1H); 4.40(d,1H).

Example 5

## N-L-Leucyl-3-phenyl-L-alanine methyl ester monohydrochloride

A mixture of 0.557 g of N-L-leucyl-3-phenyl-L-alanine, 0.6 ml of chlorotrimethylsilane and 40 ml of methyl alcohol is stirred at room temperature for 20 hours. Concentration in vacuo gives 0.560 g of the desired product.
$^1$H NMR ($CDCl_3$): δ 0.86(d,3H); 0.91(d,3H); 1.6-1.9 (m,3H); 3.14(m,2H); 3.57(s,3H); 4.22(m,1H); 4.81(dd,1H); 7.20-7.23(m,5H); 7.95(d,1H); 8.3-8.6(br s,2H).
Examples 3, 4 and 5 are made by the procedure of M.A. Brooks, T.H. Chan; Synthesis, 201 (1983).

Example 6

## [R-(R*,R*)]-$N^{\omega}$,$N^{\omega'}$-(2,6-Dibutyl-4-methylene-1,7-dioxo-1,7-heptanediyl)bis[N-L-leucyl-L-phenylalanine] dimethyl ester

A mixture of 0.046 g of product from Example 2 in 1 ml of methyl alcohol and 5 ml of 10% potassium hydroxide is heated at reflux temperature for 1/2 hour. The reaction is acidified, extracted and concentrated to give the crude diacid. To the diacid, in 5 ml of methylene chloride, is added 0.178 g of benzotriazol-1-yloxytris(dimethylamino)phosphonium hexafluorophosphate (BOP), 0.131 g of product from Example 5, 0.1 ml of triethylamine and 0.014 g of 1-hydroxybenzotriazole (HOBT). The reaction is stirred at room temperature for 3 hours, quenched with saturated sodium chloride and extracted with ethyl acetate. The combined extracts are washed with 2N hydrochloric acid, water, a solution containing both 5% potassium carbonate and 1% potassium hydroxide and then saturated sodium chloride. The organic layer is dried, concentrated in vacuo and chromatographed to give 0.044 g of the desired product.
$^1H$ NMR ($CDCl_3$): δ 0.89(m,18H); 1.2-1.7(m,18H); 2.05 (dd,2H); 2.32(dd,2H); 2.37(m,2H); 3.06(m,4H); 3.67 (s,6H); 4.47(m,2H); 4.71(s,2H); 4.77(m,2H); 6.61(d,2H); 6.84(d,2H); 7.12-7.27(m,10 H).

Example 7

**[R-(R*,R*)]-N$^{\omega}$,N$^{\omega'}$-(2,6-Dibutyl-4-methylene-1,7-dioxo-1,7-heptanediyl)bis[N-L-alanyl-L-alanine] dimethyl ester**

The title compound is prepared by the procedure of Example 6 using 0.042 g of the diacid liberated from diester of Example 2, 0.103 g of product from Example 3, 0.265 g BOP, 0.020 g of HOBT, 0.08 ml of triethylamine and 5 ml of methylene chloride. The residue is purified by chromatography to give 0.077 g of the desired product.
$^1H$ NMR ($CDCl_3$): δ 0.86(t,6H); 1.2-1.45(m,10 H); 1.33 (d,6H); 1.41(d,6H); 1.59(m,2H); 2.03(dd,2H); 2.35 (dd,2H); 2.42(m,2H); 3.74(s,6H); 4.49(q,2H); 4.62 (q,2H); 4.72(s,2H); 6.74(d,2H); 7.34(d,2H).

Example 8

**[R-(R*,R*)]-N$^{\omega}$,N$^{\omega'}$-(2,6-Dibutyl-4-methylene-1,7-dioxo-1,7-heptanediyl)bis[L-valyl-L-valine] dimethyl ester**

The title compound is prepared by the procedure of Example 6 using 0.046 g of product from Example 2, 0.177 g of BOP, 0.107 g of product from Example 4, 0.013 g of HOBT, 0.1 ml of triethylamine and 5 ml of methylene chloride. The residue is purified by chromatography to give 0.040 g of the desired product.
$^1H$ NMR ($CDCl_3$): δ 0.85(t,6H); 0.93(m,24H); 1.05-1.65 (m,12H); 1.95-2.42(m,10 H); 3.73(s,6H); 4.17(t,2H); 4.45(dd,2H); 4.78(s,2H); 7.08(d,2H); 7.40(d,2H).
Examples 6, 7 and 8 are made by the procedure of B. Castro, Synthesis, 751 (1976).

Example 9

**[R-(R*,R*)]-N$^{\omega}$,N$^{\omega'}$-[Oxiranylidenebis(2-butyl-1-oxo-3,1-propanediyl)]bis[N-L-leucyl-L-phenylalanine] dimethyl ester**

To a 0°C solution of 0.012 g of product from Example 6 in 2 ml of methylene chloride is added 0.009 g of 3-chloroperoxybenzoic acid and the reaction is stirred for 3 hours. Two ml of 10% sodium sulfite is

added and the mixture stirred for 10 minutes. The layers are separated; the organic phase washed with 1 M sodium carbonate and saturated sodium chloride, dried and concentrated in vacuo to give 0.009 g of the desired product as a white solid.
MP 78-83°C
MS(FAB): m/z 849 (M+H).
$^1$H NMR ($CDCl_3$): δ 1.10(m,18H); 1.4-1.9(m,22H); 2.22 (m,2H); 2.55(m,2H); 3.26(m,4H); 3.87(s,6H); 4.45-(m,2H); 4.81(m,2H); 6.41(d,1H); 6.78(d,1H); 6.88(d,1H); 6.99 (d,1H); 7.31-7.51(m,10 H).

Example 10

**[R-(R*,R*)]-$N^{\omega}$,$N^{\omega'}$-[Oxiranylidenebis(2-butyl-1-oxo-3,1-propanediyl)]bis[N-L-alanyl-L-alanine] dimethyl ester**

The title compound is prepared by the procedure of Example 9 using 0.030 g of product from Example 7, 0.016 g + 0.016 g of 3-chloroperoxybenzoic acid and 2 ml of methylene chloride. Concentration in vacuo gives 0.022 g of the desired product as a white waxy solid.
MS(FAB): m/z 613 (M+H).
$^1$H NMR ($CDCl_3$): δ 0.86(t,6H); 1.2-1.7(m,28H); 2.05 (t,2H); 2.33(m,1H); 2.41(m,1H); 3.74(s,6H); 4.52(m,4H); 6.78(d,1H); 6.96(d,1H); 7.28(m,1H); 7.37(m,1H).

Example 11

**[R-(R*,R*)]-$N^{\omega}$,$N^{\omega'}$-[Oxiranylidenebis(2-butyl-1-oxo-3,1-propanediyl)bis[N-L-valyl-L-valine] dimethyl ester**

The title compound is prepared by the procedure of Example 9 using 0.008 g of product from Example 8, 0.008 g of 3-chloroperoxybenzoic acid and 5 ml of methylene chloride. Concentration in vacuo gives 0.005 g of the desired product as a white waxy solid.
MP 132-134°C
MS(FAB): m/z 725 (M+H).
$^1$H NMR ($CDCl_3$): δ 0.93(m,30 H); 1.2-1.7(m,16H); 2.0-2.6(m,8H); 3.74(s,6H); 4.24(m,2H); 4.50(m,2H); 6.70-(t,2H); 6.83(d,1H); 6.90(d,1H).
Examples 9, 10 and 11 are made by the procedures of J.B. Lambert et al., JACS, 109, 7838 (1987) and E. Vedejs et al., JACS, 109, 5437 (1987).

Example 12

**[R-(R*,R*)]-$N^{\omega}$,$N^{\omega'}$-(2,6-Dibutyl-4-hydroxy-4-(hydroxymethyl)-1,7-dioxo-1,7-heptanediyl]bis[N-L-leucyl-L-phenylalanine] dimethyl ester**

To a room temperature solution of 0.017 g of product from Example 6 in 2 ml of dry tetrahydrofuran is added 0.1 ml of pyridine and 0.008 g of osmium tetroxide. The reaction is stirred for 1 hour followed by the

addition of 0.008 g of osmium tetroxide. After stirring for 1/2 hour, 5 ml of 5% sodium metabisulfite is added, the aqueous layer extracted with ethyl acetate and the combined organic layers are washed with 5% sodium metabisulfite and saturated sodium chloride. The organic layer is dried over magnesium sulfate and concentrated in vacuo. The residue is purified by chromatograph to give 0.011 g of the desired product as a solid.
MP 154-156°C
MS(FAB): m/z 867 (M+H).
$^1$H NMR ($CDCl_3$): δ 0.90(m,18H); 1.1-1.7(m,18H); 1.95(m,2H); 2.42(m,2H); 3.10(t,4H); 3.23(m,2H); 3.69(s,3H); 3.70(s,3H); 4.36(m,2H); 4.82(m,2H); 6.46(t,2H); 6.64(d,1H); 6.72(d,1H); 7.10-7.32(m,10 H).

Example 13

**[R-(R*,R*)]-$N^{\omega}$,$N^{\omega'}$-[2,6-Dibutyl-4-hydroxy-4-(hydroxymethyl)-1,7-dioxo-1,7-heptanediyl]bis[N-L-alanyl-L-alanine]dimethyl ester**

The title compound is prepared by the procedure of Example 12 using 0.032 g of product from Example 7, 0.20 ml of pyridine, 5 ml of dry tetrahydrofuran and 0.018 g of osmium tetroxide. Concentration in vacuo gives 0.030 g of the desired product as a yellow waxy solid.
MS(FAB): m/z 631 (M+H).
$^1$H NMR ($CDCl_3$): δ 0.86(t,6H); 1.23-1.41(m,24H); 1.54(m,2H); 2.05(m,2H); 2.42(m,1H); 2.55(m,1H); 3.29 (dd,2H); 3.74(s,6H); 4.53(m,4H); 6.92(d,1H); 7.02 (d,1H); 7.28(d,1H); 7.37(d,1H).

Example 14

**[R-(R*,R*)]-$N^{\omega}$,$N^{\omega'}$-[2,6-Dibutyl-4-hydroxy-4-(hydroxymethyl)-1,7-dioxo-1,7-heptanediyl]bis[N-L-valyl-L-valine] dimethyl ester**

The title compound is prepared by the procedure of Example 12 using 0.018 g of product from Example 8, 0.1 ml of pyridine, 2 ml of dry tetrahydrofuran and 0.013 g of osmium tetroxide. The residue is purified by chromatography to give 0.019 g of the desired product as a white solid.
MP 167-171°C
MS(FAB): m/z 743 (M+H).
$^1$H NMR ($CDCl_3$): δ 0.83-0.96(m,30 H); 1.1-1.7(m,12H); 1.85-2.25(m,8H); 2.45(m,1H); 2.55(m,1H); 3.28(m,2H); 3.74(s,6H); 4.28(m,2H); 4.54(dd,2H); 6.6-6.9(m,4H).
Examples 12, 13 and 14 are prepared by the procedure of H.G. Selnick, S.J. Danishefsky TL, 28, 4955 (1987) and G. Sodano et al., JOC, 52, 2301 (1987).

Example 15

**[R-(R*,R*)]-$N^{\omega}$,$N^{\omega'}$-[2,6-Dibutyl-1,4,7-trioxo-1,7-pentanediyl)bis[N-L-leucyl-L-phenylalanine] dimethyl ester**

To a room temperature solution of 0.012 g of product from Example 6 in 4 ml 3:1 dioxane/water is added 0.005 g of sodium periodate and 0.2 ml of a solution of 0.005 g osmium tetroxide in 1 ml of t-butyl alcohol. The reaction is stirred for 4 hours, filtered and the collected solid washed with 30 ml of chloroform. The chloroform solution is washed with 10% sodium sulfite, dried and concentrated in vacuo to give, after chromatography, 0.007 g of the desired product as a colorless solid.
$^1$H NMR ($CDCl_3$): δ 0.87(m,18H); 1.26(m,10 H); 1.60 (m,8H); 2.40(dd,2H); 2.53(m,2H); 2.83(dd,2H); 3.08-(d,4H); 3.69(s,6H); 4.30(m,2H); 4.82(m,2H); 6.19(d,2H); 6.64(d,2H); 7.08-7.24(m,10 H).

Example 16

**[R-(R*,R*)]-N$^\omega$,N$^{\omega'}$-[2,6-Dibutyl-1,4,7-trioxo-1,7-heptanediyl)bis[N-L-alanyl-L-alanine] dimethyl ester**

The title compound is prepared by the procedure of Example 15 using 0.041 g of product from Example 7, 0.016 g of sodium periodate, 0.2 ml of a solution of 0.005 mg osmium tetroxide in 1 ml of t-butyl alcohol and 3 ml of 1:1 dioxane/water. The residue is purified by chromatography to give 0.026 g of the desired product as a white solid.
$^1$H NMR ($CDCl_3$): δ 0.86(t,6H); 1.2-1.7(m,24H); 2.46 (dd,2H); 2.65(m,2H); 2.89(dd,2H); 3.74(s,6H); 4.36-4.60-(m,4H); 6.45(d,2H); 6.94(d,2H).

Example 17

**[R-(R*,R*)]-N$^\omega$,N$^{\omega'}$-[2,6-Dibutyl-1,4,7-trioxo-1,7-pentanediyl)bis[N-L-valyl-L-valine] dimethyl ester**

The title compound is prepared by the procedure of Example 15 using 0.018 g of product from Example 8, 0.008 g of sodium periodate, 0.2 ml of a solution of 0.005 g of osmium tetroxide in 1 ml of t-butyl alcohol and 15 ml of 2:1 dioxane/water. The reaction is stirred overnight at room temperature. The residue is purified by chromatography to give 0.020 g of the desired product.
$^1$H NMR ($CDCl_3$): δ 1.08(m,30 H); 1.41(m,6H); 1.78(m,4H); 2.37(m,6H); 2.64(dd,2H); 2.85(m,2H); 3.08(dd,2H); 3.89 (s,6H); 4.37(m,2H); 4.53(dd,2H); 6.38(d,2H); 6.58 (d,2H).
Example 15, 16 and 17 are prepared by the procedure of M. Demuth, et al., JACS, 108, 4149 (1986).

Example 18

**[R-(R*,R*)]-N$^\omega$,N$^{\omega'}$-(2,6-Dibutyl-4-hydroxy-1,7-heptanediyl)bis[N-L-leucyl-L-phenylalanine] dimethyl ester**

To a room temperature solution of 0.007 g of product from Example 15 in 2 ml of dry tetrahydrofuran is added 0.005 g of sodium borohydride. The reaction is stirred for 2 hours, quenched with 10 ml of water and extracted with ethyl acetate. The combined extracts are washed with water and saturated sodium chloride, dried and concentrated in vacuo to give 0.006 g of the desired product.
MP 178-181°C.
MS(FAB): m/z 859 (M + Na) and 837 (M + H).
$^1$H NMR ($CDCl_3$): δ 0.88(m,18H); 1.15-1.8(m,22H); 2.30 (m,1H); 2.43(m,1H); 2.83(m,2H); 3.09(m,2H); 3.55-

(m,1H); 3.92(s,6H); 4.05(m,1H); 4.3-4.5(m,2H); 4.74(t,1H); 7.23-7.38(m,10 H).

Example 19

**[R-(R*,R*)]-$N^{\omega}$,$N^{\omega'}$-(2,6-Dibutyl-4-hydroxy-1,7-heptanediyl)bis[N-L-alanyl-L-alanine] dimethyl ester**

The title compound is prepared by the procedure of Example 18 using 0.041 g of product from Example 16, 5 ml of dry tetrahydrofuran and 0.007 g of sodium borohydride. The reaction is stirred for 15 minutes. Concentration in vacuo, after standard work up, gives 0.028 g of the desired product as a white solid.
MP 183-186°C
MS(FAB): m/z 601 (M+H).
$^{1}H$ NMR ($CDCl_3$): δ 0.88(t,6H); 1.15-1.82(m,28H); 2.27(m,1H); 2.74(m,1H); 3.42(m,1H); 3.75(s,3H); 3.76 (s,3H); 4.51(m,4H); 7.35(d,1H); 7.42(d,1H); 7.63 (dd,2H).

Example 20

**[R-(R*,R*)]-$N^{\omega}$,$N^{\omega'}$-(2,6-Dibutyl-4-hydroxy-1,7-dioxo-1,7-heptanediyl)bis[N-L-valyl-L-valine] dimethyl ester**

The title compound is prepared by the procedure of Example 18 using 0.020 g of product from Example 17, 5 ml of dry tetrahydrofuran and 0.010 g of sodium borohydride. The reaction is stirred at room temperature for 15 minutes followed by standard work up giving 0.018 g of the desired product.
MP 219-223°C
MS(FAB): m/z 713 (M+H).
$^{1}H$ NMR ($CDCl_3$): δ 0.95(m,30 H); 1.2-1.8(m,16H); 2.04-2.55(m,6H); 3.55(m,1H); 3.74(s,3H); 3.75(s,3H); 4.16-(m,2H); 4.47(m,2H); 7.18(d,1H); 7.20(d,1H); 7.44 (t,2H).

Example 21

**(S)-2-Oxo-3-(1-oxo-3-phenylpropyl)-4-(phenylmethyl)-2-oxazolidinone**

To a -78°C solution of 1.77 g of (S)-(-)-4-benzyl-2-oxazolidinone in 30 ml of dry tetrahydrofuran is added, dropwise, 4 ml of 2.5 M n-butyllithium. The mixture is stirred at -78°C for 15 minutes, 1.51 ml of hydrocinnamoyl chloride is added and the solution warmed to room temperature. The reaction is quenched with water, extracted with ethyl acetate, dried and concentrated in vacuo. The residue is recrystallized from ethyl acetate/hexane to give 2.28 g of the desired product.
MP 92-93°C
$^{1}H$ NMR ($CDCl_3$): δ 2.75(dd,1H); 3.03(m,2H); 3.21-3.33 (m,3H); 4.16(m,2H); 4.65(m,1H); 7.15-7.35(m,10 H).

Example 22

[S-(R*,S*)]-3-[4-(Iodomethyl)-1-oxo-2-(phenylmethyl)-4-pentenyl]-4-(phenylmethyl)-2-oxazolidinone

To a -78°C solution of 0.309 g of product from Example 21 in 15 ml of dry tetrahydrofuran is added, dropwise, 1 ml of 1 M sodium bis(trimethylsilyl)amide. Five minutes later, 0.462 g of 3-iodo-2-iodomethyl-1-propene in 5 ml of dry tetrahydrofuran is added, dropwise. The reaction is stirred at -78°C for 2 hours followed by warming to room temperature and quenching with saturated ammonium chloride. The residue is purified by chromatography to give 0.357 g of the desired product as a yellow oil.
$^1$H NMR ($CDCl_3$): δ 2.53-2.80(m,3H); 2.89(d,2H); 3.14 (dd,1H); 3.65(m,1H); 3.93-4.13(m,3H); 4.34(m,1H); 4.51(m,1H); 5.05(s,1H); 5.31(s,1H); 7.12-7.33(m,10 H).

Example 23

[S-(R*,S*)]-1-[2-Methylene-5-oxo-5-[2-oxo-4-(phenylmethyl)-3-oxazolidinyl]-4-(phenylmethyl)-pentyl]-L-proline methyl ester

A mixture of 0.510 of L-proline methyl ester hydrogen chloride salt, 2 ml of triethylamine, 100 ml of toluene and 0.376 g of product from Example 22 is stirred for 48 hours at 80°C. The reaction is quenched with saturated ammonium chloride, extracted with ethyl acetate and chromatographed to give 0.232 g of the desired product as a colorless oil.
$^1$H NMR ($CDCl_3$): δ 1.43-3.25(m,14H); 3.41(d,1H); 3.59(t,1H); 3.69(s,3H); 3.91(d,1H); 4.33(m,1H); 4.55 (m,1H); 4.93(s,1H); 4.99(s,1H); 7.15-7.32(m,10 H).
The L-proline methyl ester hydrogen chloride salt is prepared from L-proline by the procedure of T.H. Chan, Synthesis, 201 (1983).

Example 24

[R-(R*,R*,R*)]-N-[N-[1-[5-[[1-[[[1-(Methoxycarbonyl)-2-methylpropyl]amino]carbonyl]-2-methylpropyl]amino]-2-methylene-5-oxo-4-(phenylmethyl)pentyl]-L-prolyl]-L-valyl]-L-valine methyl ester

To 0.108 g of benzyl alcohol in 10 ml of dry tetrahydrofuran is added 0.4 ml of 2.5 M n-butyllithium and 0.245 g of product from Example 23. After stirring for 1 hour, the reaction is quenched with saturated ammonium chloride, extracted with ethyl acetate, dried, concentrated *in vacuo* and chromatographed to give the diester as an oil. The diester is treated with 0.112 g of potassium hydroxide, 2 ml of methyl alcohol and 2 ml of water. The mixture is refluxed for 30 minutes and then concentrated to dryness. The residue is treated with 0.870 g of BOP, 0.464 g of product from Example 4, 0.5 ml of triethylamine, 0.076 g of HOBT, 15 ml of methylene chloride and 10 ml of acetonitrile. After 2 hours, the reaction is quenched with saturated sodium chloride, extracted with ethyl acetate and chromatographed to give 0.070 g of the desired product as a white solid.
$^1$H NMR ($CDCl_3$): δ 0.89(m,24H); 1.7-2.4(m,11H); 2.55 (dd,1H); 2.75(m,2H); 2.99(m,3H); 3.43(d,1H); 3.71 (s,3H); 3.72(s,3H); 4.13(m,1H); 4.28(dd,1H); 4.40 (dd,1H); 4.54(dd,1H); 4.93(s,1H); 4.98(s,1H); 6.60 (d,1H); 6.87(d,1H); 7.09(d,1H); 7.12-7.28(m,5H); 7.68 (d,1H).

Example 25

**[R-(R*,R*,R*)]-N-[N-[1-[5-[[1-[[[1-(Methoxycarbonyl)-2-methylpropyl]amino]carbonyl]-2-methylpropyl]-amino]-2,5-dioxo-4-(phenylmethyl)pentyl]-L-prolyl]-L-valyl]-L-valine methyl ester**

The title compound is prepared by the procedure of Example 15 using 0.048 g of product from Example 24, 0.028 g of sodium periodate, 0.5 ml of a solution of 0.005 g osmium tetroxide in 1 ml t-butyl alcohol and 1 ml of 3:1 dioxane/water. The desired product, 0.023 g, is obtained as a colorless oil.
$^1$H NMR ($CDCl_3$): δ 1.10(m,24H); 1.93-2.41(m,7H); 2.56 (m,2H); 2.72(m,1H); 2.88(dd,1H); 3.13(m,2H); 3.33 (m,3H); 3.63(d,1H); 3.80(d,1H); 3.92(s,3H); 3.93(s,3H); 4.13(dd,1H); 4.34(dd,1H); 4.47(dd,1H); 4.55(dd,1H); 6.40(d,1H); 6.70(d,1H); 7.08(d,1H); 7.13-7.28(m,6H).

Example 26

**[2R-[2R*,4R*,5[1S*(1S*)]]and 2S-[2R*,4S*,5[1R*(1R*)]]]-N-[N-[1-[2-Hydroxy-5-[[1-[[[1-(methoxycarbonyl)-2-methylpropyl]amino]carbonyl]-2-methylpropyl]amino]-5-oxo-4-(phenylmethyl)pentyl]-L-prolyl]-L-valyl]-L-valine methyl ester**

A mixture of 0.015 g of product from Example 25, 0.008 g of sodium borohydride and 4 ml of dry tetrahydrofuran is stirred at room temperature for 1/2 hour. The reaction is quenched with saturated ammonium chloride and extracted with ethyl acetate. The organic extracts are washed with saturated sodium chloride, dried and concentrated in vacuo to give 0.012 g of the product as a 2:1 diastereomeric mixture.
MP 73-76°C
MS(FAB): m/z 746 (M+H).
$^1$H NMR ($CDCl_3$): major isomer δ 0.84-0.98(m,24H); 1.63-3.33(m,18H); 3.74(s,6H); 3.97(dd,1H); 4.18(dd,1H); 4.26(dd,1H); 4.45(dd,1H); 4.55(dd,1H); 6.37(d,2H); 6.72(d,1H); 6.83(t,1H); 7.18-7.28(m,5H).

Example 27

**[R-(R*,R*)]-4-Methylene-2,6-bis(phenylmethyl)-heptanedioic acid bis(phenylmethyl)ester**

The title compound is prepared by the procedure of Example 2 using 0.743 g of product from Example 21 in 5 ml of dry tetrahydrofuran, 2 ml of 1 M sodium bis(trimethylsilyl)amide, 0.123 g of 3-iodo-2-iodomethyl-1-propene in 3 ml dry tetrahydrofuran, 0.4 ml of sodium bis(trimethylsilyl)amide and 0.432 g of benzyl alcohol and 1.2 ml of 2.5 M n-butyllithium. The residue is purified by chromatography to give 0.103 g of the desired product.
MS(FAB): m/z 425 (M- $C_6H_5$-$CH_2$-O).
$^1$H NMR ($CDCl_3$): δ 2.19(dd,2H); 2.38(dd,2H); 2.73(m,2H); 2.86(m,4H); 4.79(s,2H); 4.95(s,4H); 7.06-7.34(m,20 H).

Example 28

**[R-(R*,R*)]-$N^{\omega}$,$N^{\omega'}$-[4-Methylene-1,7-dioxo-2,6-bis(phenylmethyl)-1,7-heptanediyl]bis[N-L-valyl-L-valine]dimethyl ester**

The title compound is prepared by the procedure of Example 6 using 0.103 g of product from Example 27, 5 ml of 10% potassium hydroxide, 5 ml of methyl alcohol, 0.206 g of product from Example 4, 0.336 g of BOP, 0.2 ml triethylamine, 0.029 g of HOBT and 5 ml methylene chloride. The residue is purified by chromatography to give 0.137 g of the desired product as a solid.

$^1H$ NMR ($CDCl_3$): δ 0.86(m,24H); 1.95-2.20(m,8H); 2.36(m,2H); 2.62(m,2H); 2.94(m,2H); 3.72(s,6H); 4.13 (t,2H); 4.45(dd,2H); 4.78(s,2H); 6.41(d,2H); 6.63 (d,2H); 7.12-7.27(m,10 H).

Example 29

**[R-(R*,R*)]-$N^{\omega}$,$N^{\omega'}$-[4-Methylene-1,7-dioxo-2,6-bis(phenylmethyl)-1,7-heptanediyl]bis[N-L-leucyl-L-phenylalanine] dimethyl ester**

The title compound is prepared by the procedure of Example 6 using 0.167 g of product from Example 27, 5 ml of 10% potassium hydroxide, 5 ml of methyl alcohol, 0.411 g of product from Example 5, 0.556 g of BOP, 0.4 ml of triethylamine, 0.047 g of HOBT and 5 ml of methylene chloride. The residue is purified by chromatography to give 0.33 g of the desired product.

$^1H$ NMR ($CDCl_3$): δ 0.84(t,12H); 1.3-1.5(m,6H); 2.30-3.10(m,12H); 3.67(s,6H); 4.38(m,2H); 4.70(s,2H); 4.75-(m,2H); 6.25(d,2H); 6.73(d,2H); 7.12-7.38(m,20 H).

Example 30

**[R-(R*,R*)]-$N^{\omega}$,$N^{\omega'}$-[1,4,7-Trioxo-2,6-(phenylmethyl)-1,7-heptanediyl]bis[N-L-valyl-L-valine] dimethyl ester**

The title compound is prepared by the procedure of Example 15 using 0.032 g of product from Example 28, 0.035 g of sodium periodate, 0.005 mg of osmium tetroxide in 1 ml of t-butyl alcohol, 15 ml of acetonitrile and 2 ml of water. The residue is purified by column chromatography to give 0.017 g of the desired product as a white solid.

$^1H$ NMR ($CDCl_3$): δ 0.89(m,24H); 1.95-3.0(m,14H); 3.73 (s,6H); 4.15(m,2H); 4.45(m,2H); 6.30(d,2H); 6.45-(d,2H); 7.12-7.28(m,10 H).

Example 31

**[R-(R*,R*)]-N,N-[4-Hydroxy-1,7-dioxo-2,6-bis(phenylmethyl)-1,7-heptanediyl]bis[N-L-valyl-L-valine] dimethyl ester**

The title compound is prepared by the procedure of Example 26 using 0.017 g of product from Example 30, 5 ml of dry tetrahydrofuran and 0.005 g of sodium borohydride. The residue is purified by chromatography to give 0.009 g of the desired product as a white solid.
MP 203-206°C
MS(FAB): m/z 781 (M+H).
$^1$H NMR ($CDCl_3$): $\delta$ 0.90(m,24H); 1.4-2.2(m,8H); 2.6-3.0 (m,6H); 3.65(m,1H); 3.72(s,3H); 3.73(s,3H); 4.10-(m,2H); 4.45(m,2H); 6.2-6.8(m,4H); 7.13-7.26(m,10 H).

Example 32

**[R-(R*,R*)]-$N^{\omega}$,$N^{\omega'}$-[1,4,7-Trioxo-2,6-bis(phenylmethyl)-1,7-heptanediyl]bis[N-L-leucyl-L-phenylalanine] dimethyl ester**

The title compound is prepared by the procedure of Example 15 using 0.330 g of product from Example 29, 0.005 g of osmium tetroxide in 1 ml of t-butyl alcohol, 0.214 g of sodium periodate, 15 ml of dioxane and 5 ml of water. The residue is purified by chromatography to give 0.088 g of the desired product as white crystals.
$^1$H NMR ($CDCl_3$): $\delta$ 0.78(m,12H); 1.25-1.6(m,6H); 2.3-3.1 (m,14H); 3.68(s,6H); 4.20(m,2H); 4.79(m,2H); 6.21 (d,2H); 6.63(d,2H); 7.09-7.29(m,20 H).

Example 33

**[R-(R*,R*)]-$N^{\omega}$,$N^{\omega'}$-[4-Hydroxy-1,7-dioxo-2,6-bis-(phenylmethyl)-1,7-heptanediyl]bis[N-L-leucyl-L-phenylalanine]dimethyl ester**

The title compound is prepared by the procedure of Example 26 using 0.044 g of product from Example 32, 5 ml of dry tetrahydrofuran and 0.005 g of sodium borohydride. The residue is purified by chromatography to give 0.014 g of the desired product as a white solid.
MP 190-193°C
MS(FAB): m/z 905 (M+H).
$^1$H NMR ($CDCl_3$): $\delta$ 0.79(m,12H); 1.10-1.75(m,10 H). 2.4-3.0(m,6H); 3.06(d,4H); 3.50(m,1H); 3.68(s,6H); 4.02-(dd,1H); 4.12(dd,1H); 4.73(dd,1H); 4.82(dd,1H); 6.29(d,1H); 6.42(d,1H); 6.83(d,1H); 7.04(d,1H); 7.11-7.29-(m,20 H).

Example 34

**[R-(R*,R*)]-N,N'-Bis(1,1-dimethylmethyl)-4-methylene-2,6-bis(phenylmethyl)heptanediamide**

To a solution of 0.056 g of product from Example 27 in 10 ml of methyl alcohol is added, gradually as mixture is heated to reflux temperature, 0.5 g of sodium hydroxide in 10 ml of water. The mixture is heated at reflux temperature for 1/2 hour, cooled, acidified with concentrated hydrochloric acid and extracted with ethyl acetate. The organic layer is washed with saturated sodium chloride and concentrated in vacuo.

The diacid is dissolved in 10 ml of methylene chloride and treated with 0.185 g of BOP and 0.5 ml of t-butylamine. The reaction is stirred for 1 hour, quenched with sodium chloride, extracted with ethyl acetate,

washed with 2 N hydrochloric acid and saturated sodium chloride and concentrated in vacuo. The residue is purified by chromatography to give 0.035 g of the desired product as a colorless oil.
$^1$H NMR ($CDCl_3$); δ 1.13(s,18H); 2.45(dd,2H); 2.56-2.81(m,6H); 2.96(dd,2H); 5.02(s,2H); 7.17-7.28 (m,10 H).

Example 35

## [R-(R*,R*)]-N,N'-Bis(1,1-dimethylethyl)-4-oxo-2,6-bis(phenylmethyl)heptanediamide

The title compound is prepared by the procedure of Example 15 using 0.035 g of product from Example 34, 0.2 ml of a solution of 0.005 g osmium tetroxide in 1 ml of t-butyl alcohol, 0.032 g of sodium periodate, 3 ml of dioxane and 1 ml of water. The reaction is stirred for 2 hours followed by work up as in Example 15. The residue is purified by chromatography to give 0.035 g of the desired product as a white solid.
$^1$H NMR ($CDCl_3$): δ 1.12(s,18H); 2.07(dd,2H); 2.33-2.47 (m,4H); 2.68-2.89(m,4H); 4.83(s,2H); 5.10(s,2H); 7.16-7.37(m,10 H).

Example 36

## [R-(R*,R*)]-N,N'-Bis(1,1-dimethylethyl)-4-hydroxy-2,6-bis(phenylmethyl)heptanediamide

The title compound is prepared by the procedure of Example 12 using 0.035 g of product from Example 35, 0.028 g of sodium borohydride and 5 ml of dry tetrahydrofuran. The residue is purified by column chromatography to give 0.023 g of the desired product as a white solid.
MP 156-160°C
MS(FAB): m/z 467 (M+H).
$^1$H NMR ($CDCl_3$): δ 1.13(s,9H); 1.15(s,9H); 1.8-3.0 (m,10 H); 3.62(m,1H); 5.09(s,1H); 5.24(s,1H); 7.15-7.28-(m,10 H).

Example 37

## [R-(R*,R*)]-N,N'-Dicyclopentyl-4-methylene-2,6-bis(phenylmethyl)heptanediamide

The title compound is prepared by the procedure of Example 34 using 0.094 g of product from Example 27, 1.0 g of sodium hydroxide, 10 ml of methyl alcohol, 10 ml of water followed by 0.311 g of BOP, 5 ml of methylene chloride, 1 ml of cyclopentyl amine. The reaction is stirred for 2 hours. The residue is purified by chromatography to give 0.061 g of the desired product as a white solid.
$^1$H NMR ($CDCl_3$): δ 1.04(m,2H); 1.12(m,2H); 1.46(m,8H); 1.76(m,4H); 2.05(m,2H); 2.39-2.53(m,4H); 2.69-(dd,2H); 2.86(dd,2H); 4.02(m,2H); 4.77(s,2H); 5.50(d,2H); 7.15-7.29(m,10 H).

Example 38

## [R-(R*,R*)]-N,N'-Dicyclopentyl-4-oxo-2,6-bis(phenylmethyl)heptanediamide

The title compound is prepared by the procedure of Example 35 using 0.061 g of product from Example 37, 0.5 ml of a solution of 0.005 g osmium tetroxide in 1 ml of t-butyl alcohol, 0.054 g of sodium periodate, 6 ml of dioxane and 2 ml of water. The residue is purified by chromatography to give 0.025 g of the desired product as a white solid.
$^1$H NMR ($CDCl_3$): δ 1.01-1.85(m,16H); 2.45(dd,2H); 2.66-2.99(m,8H); 4.00(m,2H); 5.24(d,2H); 7.13-7.30 (m,10 H).

Example 39

**[R-(R*,R*)]-N,N'-Dicyclopentyl-4-hydroxy-2,6-bis-(phenylmethyl)heptanediamide**

The title compound is prepared by the procedure of Example 36 using 0.025 g of product from Example 38, 0.019 g of sodium borohydride and 5 ml of dry tetrahydrofuran. The residue is purified by column chromatography to give 0.012 g of the desired product as a white solid.
MP 191-194°C
MS(FAB): m/z 491 (M + H).
$^1$H NMR ($CDCl_3$): δ 0.95-1.89(m,20 H); 2.41(m,1H); 2.58-2.93(m,5H); 3.57(m,1H); 4.02(m,2H); 5.40(m,2H); 7.14-7.29(m,10 H).

Example 40

**[R-(R*,R*)]-N,N'-Bis(cyclohexylmethyl)-4-methylene-2,6-bis(phenylmethyl)heptanediamide**

The title compound is prepared by the procedure of Example 34 using 0.081 g of product from Example 27, 1.0 g of sodium hydroxide, 10 ml of methyl alcohol and 10 ml of water followed by 0.269 g of BOP, 5 ml of methylene chloride and 1 ml of cyclohexanemethylamine. The residue is purified by chromatography to give 0.061 g of the desired product as a white solid.
$^1$H NMR ($CDCl_3$): δ 0.67-1.6(m,22H); 2.06(dd,2H); 2.45 (dd,2H); 2.58(m,4H); 2.70(dd,2H); 2.89(dd,2H); 3.10 (m,2H); 4.77(s,2H); 5.68(t,2H); 7.13-7.29(m,10 H).

Example 41

**[R-(R*,R*)]-N,N'-Bis(cyclohexylmethyl)-4-oxo-2,6-bis(phenylmethyl)heptanediamide**

The title compound is prepared by the procedure of Example 35 using 0.061 g of product from Example 40, 0.0025 g of osmium tetroxide in 0.5 ml of t-butyl alcohol, 0.049 g of sodium periodate, 16 ml of dioxane and 2 ml of water. The residue is purified by chromatography to give 0.029 g of the desired product as a white solid.
$^1$H NMR ($CDCl_3$): δ 0.66-1.70(m,22H); 2.46(dd,2H); 2.66-3.08(m,8H); 3.62-4.02(m,4H); 5.46(t,2H); 7.13-7.30-(m,10 H).

Example 42

[R-(R*,R*)]-N,N'-Bis(cyclohexylmethyl)-4-hydroxy-2,6-bis(phenylmethyl)heptanediamide

The title compound is prepared by the procedure of Example 36 using 0.029 g of product from Example 41, 0.020 g of sodium borohydride and 5 ml of dry tetrahydrofuran. The residue is purified by chromatography to give 0.0062 g of the desired product as a white solid.
MP 185-188°C
MS(FAB): m/z 547 (M+H).
$^{1}H$ NMR ($CDCl_3$): δ 0.66-1.68(m,24H); 1.92(m,2H); 2.42-3.15(m,10 H); 3.53(m,1H); 5.50(t,1H); 5.57(t,1H); 7.14-7.29(m,10 H).

Example 43

[1S-[1α[2R*,6R*,7(1R*,2R*)],2β]]-N,N'-Bis(2-aminocyclohexyl)-4-methylene-2,6-bis(phenylmethyl)-heptanediamide

To a mixture of 0.206 g of product from Example 27 in 10 ml of methyl alcohol is added, slowly at reflux temperature, 1.0 g of sodium hydroxide in 10 ml of water. The reaction is heated at reflux temperature for 1/2 hour followed by acidification, extraction with ethyl acetate and concentration in vacuo. The residue is treated with 0.442 g of (1S,2S)-(+)-1,2-diamino-cyclohexane, 0.684 g of BOP, 0.5 ml of triethylamine and 25 ml of methylene chloride. The reaction is stirred at room temperature for 18 hours, quenched with saturated sodium chloride and extracted with ethyl acetate. The organic layer is washed with water, saturated sodium chloride and concentrated in vacuo. The residue is purified by chromatography to give 0.137 g of the desired product as a yellow solid.
$^{1}H$ NMR ($CDCl_3/CD_3OD$): δ 0.97-1.27(m,8H); 1.68(m,6H); 1.87(m,2H); 2.17(m,4H); 2.49(dd,2H); 2.78(m,6H); 3.29(m,2H); 4.85(s,2H); 7.19-7.32(m,10 H).

Example 44

[1S-[1α[2R*,6*,7(1R*,2R*)],2β]]-[[4-Methylene-1,7-dioxo-2,6-(phenylmethyl)-1,7-heptanediyl]bis(imino-2,1-cyclohexanediyl)]bis carbamic acid bis-(1,1-dimethylethyl)ester

To a solution of 0.034 g of product from Example 43 in 10 ml of dry tetrahydrofuran is added 0.055 g of di-tert-butyl dicarbonate and 0.1 ml of triethylamine. The reaction is stirred at room temperature for 1 hour, quenched with saturated ammonium chloride and extracted with ethyl acetate. The organic layer is washed with saturated sodium chloride, dried and concentrated in vacuo. The residue is purified by chromatography to give 0.015 g of the desired product as a white solid.
$^{1}H$ NMR ($CDCl_3$): δ 1.25(m,8H); 1.42(s,18H); 1.69(m,4H); 1.95(m,6H); 2.31(dd,2H); 2.63(d,4H); 2.97(dd,2H); 3.33(m,2H); 3.61(m,2H); 4.69(s,2H); 5.24(d,2H); 6.51 (d,2H); 7.14-7.28(m,10 H).

Example 45

**[1S-[1α[2R*,6R*,7(1R*,2R*)],2β]]-[1,4,7-Trioxo-2,6-bis(phenylmethyl)-1,7-heptanediyl]bis(imino-2,1-cyclohexanediyl)bis carbamic acid bis(1,1-dimethylethyl)ester**

The title compound is prepared by the procedure of Example 15 using 0.015 g of a product from Example 44, 0.2 ml of a solution of 0.005 g osmium tetroxide in 1 ml of t-butyl alcohol, 0.008 g of sodium periodate, 10 ml of dioxane and 3 ml of water. The reaction is stirred at room temperature for 48 hours. The residue is purified by chromatography to give 0.015 g of the desired product as a white solid.

$^1$H NMR ($CDCl_3$): δ 1.0-1.3(m,8H); 1.38(s,18H); 1.70 (m,4H); 1.98(m,6H); 2.27(d,2H); 2.60(dd,2H); 2.72-(m,4H); 3.24(m,2H); 3.47(m,2H); 4.84(d,2H); 5.99(d,2H); 7.09-7.27(m,10 H).

Example 46

**[1S-[1α[2R*,6R*,7(1R*,2R*)],2β]-[[4-Hydroxy-1,7-dioxo-2,6-bis(phenylmethyl)-1,7-heptanediyl]bis(imino-2,1-cyclohexanediyl)]bis carbamic acid bis(1,1-dimethylethyl)ester**

The title compound is prepared by the procedure of Example 18 using 0.015 g of product from Example 45, 0.007 g of sodium borohydride and 5 ml of dry tetrahydrofuran. The residue is purified by chromatography to give 0.007 g of the desired product as a white solid.

MP 218-223°C.

MS(FAB): m/z 749 (M + H).

$^1$H NMR ($CDCl_3$): δ 1.0-1.3(m,8H); 1.39(s,18H); 1.70 (m,8H); 2.02(m,4H); 2.48(m,1H); 2.66(m,3H); 2.89-(m,2H); 3.27(m,2H); 3.52(m,3H); 4.79(m,2H); 6.24(m,1H); 6.40(m,1H); 7.09-7.28(m,10 H).

Example 47

**[1S-[1α[2R*,6R*,7(1R*,2R*)],2β]]-4-Methylene-2,6-bis-(phenylmethyl)-N,N'-bis[2-[(2-quinolinylcarbonyl)-amino]cyclohexyl]heptanediamide**

To a solution of 0.034 g of product from Example 43 in 5 ml of methylene chloride is added 0.043 g of quinaldic acid, 0.110 g of BOP and 0.2 ml triethylamine. The reaction is stirred at room temperature for 2 hours, quenched with saturated sodium chloride and extracted with ethyl acetate. The organic layer is washed with 10% sodium carbonate, 1% sodium hydroxide and saturated sodium chloride. The ethyl acetate solution is dried and concentrated in vacuo. The residue is purified by chromatography to give 0.042 g of the desired product as a yellow solid.

$^1$H NMR ($CDCl_3$): δ 1.0-2.82(m,26H); 3.78-3.98(m,4H); 4.70(s,2H); 6.43(d,2H); 6.61(t,2H); 6.82(m,4H); 6.96-(m,4H); 7.61(m,2H); 7.77(m,2H); 7.84(d,2H); 8.08(m,6H); 8.39(d,2H).

Example 48

## [1S-[1α[2R*,6R*,7(1R*,2R*)],2β]]-4-Oxo-2,6-bis-(phenylmethyl)-N,N'-bis[2-[(2-quinolinylcarbonyl)amino]cyclohexyl]-heptanediamide

The title compound is prepared by the procedure of Example 15 using 0.042 g of product from Example 47, 0.5 ml of a solution of 0.005 g osmium tetroxide in 1 ml t-butyl alcohol, 0.021 g of sodium periodate, 10 ml of dioxane and 3 ml of water. The residue is purified by chromatography to give 0.034 g of the desired product as a white solid.

$^1$H NMR ($CDCl_3$): δ 1.17-1.60(m,8H); 1.68(m,4H); 1.95-2.38(m,10 H); 2.60-2.70(m,4H); 3.62-3.98(m,4H); 6.32-(d,2H); 6.74(m,2H); 6.88(m,8H); 7.60(t,2H); 7.76(t,2H); 7.83(d,2H); 8.13(d,2H); 8.21(m,4H); 8.34(d,2H).

### Example 49

## [1S-[1α[2R*,6R*,7(1R*,2R*)],2β]]-4-Hydroxy-2,6-bis-(phenylmethyl)-N,N'-bis[2-[(2-quinolinylcarbonyl)-amino]cyclohexyl]-heptanediamide

The title compound is prepared by the procedure of Example 18 using 0.034 g of product from Example 48, 0.015 g of sodium borohydride and 5 ml dry tetrahydrofuran. The residue is purified by chromatography to give 0.016 g of the desired product as a white solid.

MP 221-225°C.

MS(FAB): m/z 859 (M+1).

$^1$H NMR ($CDCl_3$): δ 1.19-1.75(m,12H); 1.78(m,4H); 2.13(m,4H); 2.31-2.40(m,3H); 2.57-2.70(m,3H); 3.50-(m,1H); 3.72-3.87(m,4H); 6.50(dd,2H); 6.62(dd,2H); 6.74(m,2H); 6.88(m,6H); 7.60(m,2H); 7.76(m,2H); 7.85-(d,2H); 8.10-8.27(d,6H); 8.33(d,2H).

### Example 50

## N,N'-(2-Methylene-1,3-propanediyl)bis[N-(phenylmethyl)glycine] diethyl ester

A mixture of 0.56 g of N-benzylglycine ethyl ester, 0.74 g of 3-iodo-2-iodomethyl-1-propene and 5 ml of benzene is heated at reflux temperature for 1 hour. The reaction is cooled, diluted with diethyl ether, washed with a saturated potassium carbonate solution, dried and concentrated in vacuo. The residue is purified by chromatography to give 0.68 g of the desired product. The 3-iodo-2-iodomethyl-1-propene used in this example was prepared from 3-chloro-2-chloromethyl-1-propene as described by P.S. Skell; R.G. Doerr, JACS, 89, 4688(1967).

MS(FAB): m/z 439 ($M+H^+$).

IR(neat): 1737 $cm^{-1}$.

$^1$H NMR ($CDCl_3$): δ 1.25(t,6H); 3.24(s,4H); 3.30(s,4H); 3.75(s,4H); 4.12(q,4H); 5.17(s,2H); 7.25(m,10 H).

### Example 51

## 2,2'-[(2-Methylene-1,3-propanediyl)bis[(phenylmethyl)-imino]]bis[N-(1,1-dimethylethyl)acetamide]

To a -10°C solution of 1.4 ml of trimethyl-aluminum in 20 ml of methylene chloride is added 1.6 ml of tert-butylamine. The reaction is stirred for 20 minutes followed by warning to room temperature. One and one tenth grams of product from Example 50 is added, followed by heating at reflux temperature for 48 hours. The reaction is diluted with ammonium chloride/water. The resulting precipitate is washed with methylene chloride and the organic layer purified by chromatography to give 1.1 g of the desired product.
MS(FAB): m/z 493 $(M+H^+)$.
IR(neat): 3351 $cm^{-1}$, 1681 $cm^{-1}$ and 1513 $cm^{-1}$.
$^1H$ NMR ($CDCl_3$): δ 1.27(s,18H); 2.97(s,4H); 3.06(s,4H); 3.56(s,4H); 5.32(s,2H); 6.89(s,6.89); 7.31(m,10 H).
A minor by product, N-[2-[[[2-[(1,1-dimethylethyl)amino]-2-oxoethyl](phenylmethyl)amino]methyl]-2-propenyl]-N-(phenylmethyl)glycine ethyl ester is formed.
$^1H$ NMR ($CDCl_3$): δ 1.37(s,9H); 4.13(q,2H); 5.24(s,1H); 5.29(s,1H); 7.38(s,5H).

Example 52

## 2,2'-[[2-Hydroxy-2-(hydroxymethyl)-1,3-propanediyl]-bis[(phenylmethyl)imino]]bis[N-(1,1-dimethylethyl)-acetamide]

To a solution of 0.25 g of product from Example 51 in 15 ml of acetone is added 0.12 g of 4-methylmorpholine N-oxide in 0.5 ml of a 4% solution of osmium tetroxide in water. Sufficient water and/or acetone is added to reaction to maintain a homogeneous solution. The reaction is stirred at room temperature for 48 hours, diluted with diethyl ether and washed with sodium bisulfite solution. The layers are separated and the aqueous layer is re-extracted with methylene chloride. The organic layers are combined, washed with saturated sodium chloride, dried and concentrated in vacuo. The residue is purified by chromatography to give 0.150 g of the desired product.
MS(FAB): m/z 526 $(M^+)$.
IR(neat): 3295 $cm^{-1}$, 1649 $cm^{-1}$ and 1527 $cm^{-1}$.
$^1H$ NMR ($CDCl_3$): δ 1.30(s,18H); 2.51(d,2H); 2.59(d,2H); 3.06(d,2H); 3.26(d,2H); 3.47(s,2H); 3.59(d,2H); 3.80-(d,2H); 6.19(s,2H); 7.23(m,10 H).

Example 53

## 2,2'-[(2-Hydroxy-1,3-propanediyl)bis[(phenylmethyl)-imino]]bis[N-(1,1-dimethylethyl)acetamide]

To a solution of 0.25 g of product from Example 52 in 20 ml of tetrahydrofuran is added 0.20 g of sodium periodate dissolved in a minimum volume of water. Sufficient water and/or tetrahydrofuran is added to reaction to maintain a homogeneous solution. The reaction is stirred at room temperature for 30 minutes, concentrated in vacuo, diluted with saturated sodium chloride, extracted with methylene chloride and concentrated in vacuo. The residue dissolved in 20 ml of ethyl alcohol is treated with 0.5 g of sodium borohydride (excess). The reaction is stirred at room temperature for 30 minutes, concentrated in vacuo, diluted with saturated sodium chloride, extracted with methylene chloride and concentrated in vacuo. The residue is purified by chromatography to give 0.17 g of the desired product.
MS(FAB): m/z 497 $(M+H^+)$.
IR(neat): 3300 $cm^{-1}$, 1650 $cm^{-1}$ and 1531 $cm^{-1}$.
$^1H$ NMR ($CDCl_3$): δ 1.35(s,18H); 6.87(s,2H); 7.26(m,10 H).

Example 54

**N-[3-[[2-[(1,1-Dimethylethyl)amino]-2-oxoethyl](phenylmethyl)amino]-2-hydroxy-2-(hydroxymethyl)-propyl]-N-(phenylmethyl)glycine ethyl ester**

The by product, 0.35 g, from Example 51 in 20 ml of acetone is treated with 0.2 g of 4-methylmorpholine N-oxide in 0.5 ml of a 4% solution of osmium tetroxide in water. The reaction is stirred at room temperature for 48 hours, quenched with aqueous sodium bisulfite, extracted with diethyl ether and concentrated in vacuo. The residue is purified by chromatography to give 0.18 g of the desired product.
MS(FAB): m/z 500 ($M+H^+$) and 454 ($MH^+$-$C_2H_5OH$).
IR(neat): 3320 $cm^{-1}$, 1735 $cm^{-1}$, 1655 $cm^{-1}$ and 1527 $cm^{-1}$.
$^1$H NMR ($CDCl_3$): $\delta$ 1.24(t,3H); 1.32(s,9H); 4.14(q,2H); 6.54(bs,1H); 7.30(m,10 H).

Example 55

**N,N'-[2-Hydroxy-2-(hydroxymethyl)-1,3-propanediyl]bis-[N-(phenylmethyl)glycine] diethyl ester**

To a solution of 0.2 g of product from Example 50 in 5 ml of acetone is added 0.10 g of 4-methylmorpholine N-oxide in 0.25 ml of a 4% solution of osmium tetroxide in water. The reaction is stirred at room temperature for 18 hours. Thin layer chromatography indicates the presence of starting material. An additional 0.3 g of 4-methylmorpholine N-oxide in 0.5 ml of a 4% solution of osmium tetroxide in water is added and the reaction stirred for 7 hours. The mixture is diluted with diethyl ether, washed with aqueous sodium sulfite and concentrated in vacuo. The residue is purified by chromatography to give 0.092 g of the desired product.
MS(FAB): m/z 473 ($M+H^+$) and 427 ($M+H^+$-$C_2H_5OH$).
IR(neat): 3446 $cm^{-1}$ and 1735 $cm^{-1}$.
$^1$H NMR ($CDCl_3$): $\delta$ 1.23(t,6H); 2.53(d,2H); 2.73(d,2H); 3.24(d,2H); 3.45(d,2H); 3.57(s,2H); 3.70(d,2H); 4.03-(d,2H); 4.13(q,4H).

Example 56

**1,1'-(2-Methylene-1,3-propanediyl)bis-2-piperidinecarboxylic acid dimethyl ester**

A mixture of 1.75 g of pipecolic acid methyl ester, 1.88 g of 3-iodo-2-iodomethyl-1-propene in 25 ml of benzene is heated at reflux temperature for 3 hours followed by stirring at room temperature for 18 hours. The reaction is diluted with diethyl ether, water and sodium sulfite. The organic layer is concentrated in vacuo. The residue is purified by chromatography to give 0.53 g of the desired product.

| | | | |
|---|---|---|---|
| Calculated for $C_{18}H_{30}N_2O$: | C = 63.88; | H = 8.93; | N = 8.28 |
| Found: | C = 63.72; | H = 8.74; | N = 8.89 |

MS(FAB): m/z 339 ($M+H^+$).
IR(neat): 1737 $cm^{-1}$.
$^1$H NMR ($CDCl_3$): $\delta$ 3.69(s,6H); 5.04(s,2H).

Example 57

**1,1'-(2-Methylene-1,3-propanediyl)bis[N-(1,1-dimethylethyl)-2-piperidinecarboxamide]**

To a 0°C solution of 6.4 ml of trimethyl aluminum in 20 ml of methylene chloride is added 7.2 ml of tert-butylamine. The reaction is stirred for 30 minutes followed by warming to room temperature. One and four tenth grams of product from Example 56 is added. The reaction is heated at reflux temperature for 120 hours, quenched with saturated ammonium chloride, filtered and the resulting precipitate washed with methylene chloride. The organic layer is purified by chromatography to give 0.94 g of the desired product as a mixture of diastereoisomers.
MS(FAB): m/z 421 $(M+H^+)$.
IR(neat): 3371 $cm^{-1}$, 1681 $cm^{-1}$ and 1511 $cm^{-1}$.
$^1H$ NMR ($CDCl_3$): $\delta$ 1.30(s,9H); 1.33(s,9H); 5.20(bs,2H); 6.51(s,1H); 6.53(s,1H).

Example 58

**1,1'-[2-Hydroxy-2-(hydroxymethyl)-1,3-propanediyl]-bis[N-(1,1-dimethylethyl)-2-piperidinecarboxamide] (Isomer A) and 1,1'-[2-hydroxy-2-(hydroxymethyl)-1,3-propanediyl]bis[N-(1,1-dimethylethyl)-2-piperidine-carboxamide] (Isomer B)**

The title compounds are prepared by the procedure of Example 52 using 0.86 g of product from Example 57 in 20 ml of acetone, 0.5 g of 4-methylmorpholine N-oxide in 0.5 ml of a 4% solution of osmium tetroxide in water. The residue is purified by chromatography to give 0.11 g of isomer A and 0.45 g of isomer B.

Isomer A:

MS(FAB): m/z 455 $(M+H^+)$.
IR(Kbr): 3322 $cm^{-1}$, 1654 $cm^{-1}$ and 1538 $cm^{-1}$.
$^1H$ NMR ($CDCl_3$): $\delta$ 1.35(s,9H); 1.36(s,9H).

Isomer B:

MS(FAB): m/z 455 $(M+H^+)$.
IR(Kbr): 3361 $cm^{-1}$, 1657 $cm^{-1}$ and 1522 $cm^{-1}$.
$^1H$ NMR ($CDCl_3$): $\delta$ 1.35(s,18H).

Example 59

**N,N'-(2-Methylene-1,3-propadiyl)bis[N-cyclohexyl-methyl)glycine] diethyl ester**

A mixture of 0.7 g of N-(cyclohexylmethyl)glycine ethyl ester, 0.54 g of 3-iodo-2-iodomethyl-1-propane and 10 ml of benzene is heated at reflux temperature for 8 hours. The reaction is concentrated *in vacuo* and

the residue purified by chromatography to give 0.05 g of the desired product.
MS(FAB): m/z 450 ($M^+$).
IR(neat): 1741 $cm^{-1}$.
$^1H$ NMR ($CDCl_3$): $\delta$ 1.26(t,4H); 2.36(d,4H); 3.18(s,4H); 3.27(s,4H); 4.13(q,4H); 5.05(s,2H).

Example 60

**N,N'-[2-Hydroxy-2-(hydroxymethyl)-1,3-propanediyl]-bis[N-(cyclohexylmethyl)glycine] diethyl ester**

The title compound is prepared by the procedure of Example 52 using 0.050 g of product from Example 59, 0.25 g of 4-methylmorpholine N-oxide in 0.25 ml of a 4% solution of osmium tetroxide in water. The reaction is stirred at room temperature for 72 hours. The residue is purified by chromatography to give 0.06 g of the desired product.
MS(FAB): m/z 485 ($M+H^+$) and 439 ($MH^+$-$C_2H_5OH$).
IR(neat): 3453 $cm^{-1}$ and 1739 $cm^{-1}$.
$^1H$ NMR ($CDCl_3$): $\delta$ 1.27(t,4H); 4.17(q,4H).

Example 61

**2,2'-(2-Methylene-1,3-propanediyl)bis[1,2,3,4-tetrahydro-3-isoquinolinecarboxylic acid] dimethyl ester**

A mixture of 1.3 g of 1,2,3,4-tetrahydro-3-isoquinolinecarboxylic acid methyl ester, 1.05 g of 3-iodo-2-iodomethyl-1-propane and 10 ml of benzene is heated at reflux temperature for 4 hours. The reaction is cooled, diluted with diethyl ether, washed with saturated potassium carbonate and sodium sulfite and concentrated in vacuo. The residue is purified by chromatography to give 1.1 g of the desired product.
MS(FAB): m/z 435 ($M+H^+$).
IR(neat): 1734 $cm^{-1}$.
$^1H$ NMR ($CDCl_3$): 3.59(s,3H); 3.60(s,3H); 5.16(s,2H); 7.01(m,2H); 7.11(m,6H).

Example 62

**2,2'-(2-Methylene-1,3-propanediyl)bis[N-(1,1-dimethylethyl)-1,2,3,4-tetrahydro-3-isoquinolinecarboxamide]**

The title compound is prepared by the procedure of Example 51 using 0.77 g of product from Example 61, 1.36 ml of trimethyl aluminum in 20 ml of methylene chloride, 1.5 ml of tert-butylamine. The reaction is heated at reflux temperature for 96 hours. The residue is purified by chromatography to give 0.58 g of the desired product as diastereoisomers.
MS(FAB): m/z 516 ($M^+$).
IR(neat): 3330 $cm^{-1}$, 1675 $cm^{-1}$ and 1514 $cm^{-1}$.
$^1H$ NMR ($CDCl_3$): $\delta$ 1.21(s,9H); 1.22(s,9H).

Examples 63-155

Substantially following the methods described in detail hereinabove in Examples 6-8, 15-20 and 28-33, the compounds of this invention listed below in examples 63-155 are prepared using the dipeptide ester

indicated.

Example 63

[R-(R*,R*)]-$N^{\omega}$,$N^{\omega'}$-(2,6-Dibutyl-4-hydroxy-1,7-dioxo-1,7-heptanediyl)bis[N-L-valyl-L-leucine] dimethyl ester is prepared from L-valyl-L-leucine methyl ester.

Example 64

[R-(R*,R*)]-$N^{\omega}$,$N^{\omega'}$-(2,6-Dibutyl-4-hydroxy-1,7-dioxo-1,7-heptanediyl)bis[N-L-valyl-L-phenylalanine] dimethyl ester is prepared from L-valyl-L-phenylalanine methyl ester.

Example 65

[R-(R*,R*)]-$N^{\omega}$,$N^{\omega'}$-(2,6-Dibutyl-4-hydroxy-1,7-dioxo-1,7-heptanediyl)bis[N-L-valyl-L-tyrosine] dimethyl ester is prepared from L-valyl-L-tyrosine methyl ester.

Example 66

[R-(R*,R*)]-$N^{\omega}$,$N^{\omega'}$-(2,6-Dibutyl-4-hydroxy-1,7-dioxo-1,7-heptanediyl)bis[N-L-valyl-L-arganine] dimethyl ester is prepared from L-valyl-L-arganine methyl ester.

Example 67

[R-(R*,R*)]-$N^{\omega}$,$N^{\omega'}$-(2,6-Dibutyl-4-hydroxy-1,7-dioxo-1,7-heptanediyl)bis[N-L-valyl-L-isoleucine] dimethyl ester is prepared from L-valyl-L-isoleucine methyl ester.

Example 68

[R-(R*,R*)]-$N^{\omega}$,$N^{\omega'}$-(2,6-Dibutyl-4-hydroxy-1,7-dioxo-1,7-heptanediyl)bis[N-L-valyl-L-alanine] dimethyl ester is prepared from L-valyl-L-alanine methyl ester.

Example 69

[R-(R*,R*)]-$N^{\omega}$,$N^{\omega'}$-(2,6-Dibutyl-4-hydroxy-1,7-dioxo-1,7-heptanediyl)bis[N-L-leucyl-L-valine] dimethyl ester is prepared from L-leucyl-L-valine methyl ester.

Example 70

[R-(R*,R*)]-$N^{\omega}$,$N^{\omega'}$-(2,6-Dibutyl-4-hydroxy-1,7-dioxo-1,7-heptanediyl)bis[N-L-leucyl-L-leucine] dimethyl ester is prepared from L-leucyl-L-leucine methyl ester.

Example 71

[R-(R*,R*)]-$N^{\omega}$,$N^{\omega'}$-(2,6-Dibutyl-4-hydroxy-1,7-dioxo-1,7-heptanediyl)bis[N-L-leucyl-L-tyrosine] dimethyl ester is prepared from L-leucyl-L-tyrosine methyl ester.

Example 72

[R-(R*,R*)]-$N^{\omega}$,$N^{\omega'}$-(2,6-Dibutyl-4-hydroxy-1,7-dioxo-1,7-heptanediyl)bis[N-L-leucyl-L-arganine] dimethyl ester is prepared from L-leucyl-L-arganine methyl ester.

Example 73

[R-(R*,R*)]-$N^{\omega}$,$N^{\omega'}$-(2,6-Dibutyl-4-hydroxy-1,7-dioxo-1,7-heptanediyl)bis[N-L-leucyl-L-isoleucine] dimethyl ester is prepared from L-leucyl-L-isoleucine methyl ester.

Example 74

[R-(R*,R*)]-N$^{\omega}$,N$^{\omega'}$-(2,6-Dibutyl-4-hydroxy-1,7-dioxo-1,7-heptanediyl)bis[N-L-leucyl-L-alanine] dimethyl ester is prepared from L-leucyl-L-alanine methyl ester.

Example 75

[R-(R*,R*)]-N$^{\omega}$,N$^{\omega'}$-(2,6-Dibutyl-4-hydroxy-1,7-dioxo-1,7-heptanediyl)bis[N-L-isoleucyl-L-valine] dimethyl ester is prepared from L-isoleucyl-L-valine methyl ester.

Example 76

[R-(R*,R*)]-N$^{\omega}$,N$^{\omega'}$-(2,6-Dibutyl-4-hydroxy-1,7-dioxo-1,7-heptanediyl)bis[N-L-isoleucyl-L-leucine] dimethyl ester is prepared from L-isoleucyl-L-leucine methyl ester.

Example 77

[R-(R*,R*)]-N$^{\omega}$,N$^{\omega'}$-(2,6-Dibutyl-4-hydroxy-1,7-dioxo-1,7-heptanediyl)bis[N-L-isoleucyl-L-phenylalanine] dimethyl ester is prepared from L-isoleucyl-L-phenylalanine methyl ester.

Example 78

[R-(R*,R*)]-N$^{\omega}$,N$^{\omega'}$-(2,6-Dibutyl-4-hydroxy-1,7-dioxo-1,7-heptanediyl)bis[N-L-isoleucyl-L-tyrosine] dimethyl ester is prepared from L-isoleucyl-L-tyrosine methyl ester.

Example 79

[R-(R*,R*)]-N$^{\omega}$,N$^{\omega'}$-(2,6-Dibutyl-4-hydroxy-1,7-dioxo-1,7-heptanediyl)bis[N-L-isoleucyl-L-arganine] dimethyl ester is prepared from L-isoleucyl-L-arganine methyl ester.

Example 80

[R-(R*,R*)]-N$^{\omega}$,N$^{\omega'}$-(2,6-Dibutyl-4-hydroxy-1,7-dioxo-1,7-heptanediyl)bis[N-L-isoleucyl-L-isoleucine] dimethyl ester is prepared from L-isoleucyl-L-isoleucine methyl ester.

Example 81

[R-(R*,R*)]-N$^{\omega}$,N$^{\omega'}$-(2,6-Dibutyl-4-hydroxy-1,7-dioxo-1,7-heptanediyl)bis[N-L-isoleucyl-L-alanine] dimethyl ester is prepared from L-isoleucyl-L-alanine methyl ester.

Example 82

[R-(R*,R*)]-N$^{\omega}$,N$^{\omega'}$-(2,6-Dibutyl-4-hydroxy-1,7-dioxo-1,7-heptanediyl)bis[N-L-asparaginyl-L-valine] dimethyl ester is prepared from L-asparaginyl-L-valine methyl ester.

Example 83

[R-(R*,R*)]-N$^{\omega}$,N$^{\omega'}$-(2,6-Dibutyl-4-hydroxy-1,7-dioxo-1,7-heptanediyl)bis[N-L-asparaginyl-L-leucine] dimethyl ester is prepared from L-asparaginyl-L-leucine methyl ester.

Example 84

[R-(R*,R*)]-N$^{\omega}$,N$^{\omega'}$-(2,6-Dibutyl-4-hydroxy-1,7-dioxo-1,7-heptanediyl)bis[N-L-asparaginyl-L-phenylalanine] dimethyl ester is prepared from L-asparaginyl-L-phenylalanine methyl ester.

Example 85

[R-(R*,R*)]-N$^{\omega}$,N$^{\omega'}$-(2,6-Dibutyl-4-hydroxy-1,7-dioxo-1,7-heptanediyl)bis[N-L-asparaginyl-L-tyrosine] dimethyl ester is prepared from L-asparaginyl-L-tyrosine methyl ester.

Example 86

[R-(R*,R*)]-$N^{\omega}$,$N^{\omega'}$-(2,6-Dibutyl-4-hydroxy-1,7-dioxo-1,7-heptanediyl)bis[N-L-asparaginyl-L-arganine] dimethyl ester is prepared from L-asparaginyl-L-arganine methyl ester.

Example 87

[R-(R*,R*)]-$N^{\omega}$,$N^{\omega'}$-(2,6-Dibutyl-4-hydroxy-1,7-dioxo-1,7-heptanediyl)bis[N-L-asparaginyl-L-isoleucine] dimethyl ester is prepared from L-asparaginyl-L-isoleucine methyl ester.

Example 88

[R-(R*,R*)]-$N^{\omega}$,$N^{\omega'}$-(2,6-Dibutyl-4-hydroxy-1,7-dioxo-1,7-heptanediyl)bis[N-L-asparaginyl-L-alanine] dimethyl ester is prepared from L-asparaginyl-L-alanine methyl ester.

Example 89

[R-(R*,R*)]-$N^{\omega}$,$N^{\omega'}$-(2,6-Dibutyl-4-hydroxy-1,7-dioxo-1,7-heptanediyl)bis[N-L-glutaminyl-L-valine] dimethyl ester is prepared from L-glutaminyl-L-valine methyl ester.

Example 90

[R-(R*,R*)]-$N^{\omega}$,$N^{\omega'}$-(2,6-Dibutyl-4-hydroxy-1,7-dioxo-1,7-heptanediyl)bis[N-L-glutaminyl-L-leucine] dimethyl ester is prepared from L-glutaminyl-L-leucine methyl ester.

Example 91

[R-(R*,R*)]-$N^{\omega}$,$N^{\omega'}$-(2,6-Dibutyl-4-hydroxy-1,7-dioxo-1,7-heptanediyl)bis[N-L-glutaminyl-L-phenylalanine] dimethyl ester is prepared from L-glutaminyl-L-phenylalanine methyl ester.

Example 92

[R-(R*,R*)]-$N^{\omega}$,$N^{\omega'}$-(2,6-Dibutyl-4-hydroxy-1,7-dioxo-1,7-heptanediyl)bis[N-L-glutaminyl-L-tyrosine] dimethyl ester is prepared from L-glutaminyl-L-tyrosine methyl ester.

Example 93

[R-(R*,R*)]-$N^{\omega}$,$N^{\omega'}$-(2,6-Dibutyl-4-hydroxy-1,7-dioxo-1,7-heptanediyl)bis[N-L-glutaminyl-L-arganine] dimethyl ester is prepared from L-glutaminyl-L-arganine methyl ester.

Example 94

[R-(R*,R*)]-$N^{\omega}$,$N^{\omega'}$-(2,6-Dibutyl-4-hydroxy-1,7-dioxo-1,7-heptanediyl)bis[N-L-glutaminyl-L-isoleucine] dimethyl ester is prepared from L-glutaminyl-L-isoleucine methyl ester.

Example 95

[R-(R*,R*)]-$N^{\omega}$,$N^{\omega'}$-(2,6-Dibutyl-4-hydroxy-1,7-dioxo-1,7-heptanediyl)bis[N-L-glutaminyl-L-alanine] dimethyl ester is prepared from L-glutaminyl-L-alanine methyl ester.

Example 96

[R-(R*,R*)]-$N^{\omega}$,$N^{\omega'}$-(2,6-Dibutyl-4-hydroxy-1,7-dioxo-1,7-heptanediyl)bis[N-L-alanyl-L-valine] dimethyl ester is prepared from L-alanyl-L-valine methyl ester.

Example 97

[R-(R*,R*)]-$N^{\omega}$,$N^{\omega'}$-(2,6-Dibutyl-4-hydroxy-1,7-dioxo-1,7-heptanediyl)bis[N-L-alanyl-L-leucine] dimethyl ester is

prepared from L-alanyl-L-leucine methyl ester.

Example 98

[R-(R*,R*)]-N$^{\omega}$,N$^{\omega'}$-(2,6-Dibutyl-4-hydroxy-1,7-dioxo-1,7-heptanediyl)bis[N-L-alanyl-L-phenylalanine] dimethyl ester is prepared from L-alanyl-L-phenylalanine methyl ester.

Example 99

[R-(R*,R*)]-N$^{\omega}$,N$^{\omega'}$-(2,6-Dibutyl-4-hydroxy-1,7-dioxo-1,7-heptanediyl)bis[N-L-alanyl-L-tyrosine] dimethyl ester is prepared from L-alanyl-L-tyrosine methyl ester.

Example 100

[R-(R*,R*)]-N$^{\omega}$,N$^{\omega'}$-(2,6-Dibutyl-4-hydroxy-1,7-dioxo-1,7-heptanediyl)bis[N-L-alanyl-L-arganine] dimethyl ester is prepared from L-alanyl-L-arganine methyl ester.

Example 101

[R-(R*,R*)]-N$^{\omega}$,N$^{\omega'}$-(2,6-Dibutyl-4-hydroxy-1,7-dioxo-1,7-heptanediyl)bis[N-L-alanyl-L-isoleucine] dimethyl ester is prepared from L-alanyl-L-isoleucine methyl ester.

Example 102

[R-(R*,R*)]-N$^{\omega}$,N$^{\omega'}$-(2,6-Dibutyl-4-hydroxy-1,7-dioxo-1,7-heptanediyl)bis[N-L-threonyl-L-valine] dimethyl ester is prepared from L-threonyl-L-valine methyl ester.

Example 103

[R-(R*,R*)]-N$^{\omega}$,N$^{\omega'}$-(2,6-Dibutyl-4-hydroxy-1,7-dioxo-1,7-heptanediyl)bis[N-L-threonyl-L-leucine] dimethyl ester is prepared from L-threonyl-L-leucine methyl ester.

Example 104

[R-(R*,R*)]-N$^{\omega}$,N$^{\omega'}$-(2,6-Dibutyl-4-hydroxy-1,7-dioxo-1,7-heptanediyl)bis[N-L-threonyl-L-phenylalanine] dimethyl ester is prepared from L-threonyl-L-phenylalanine methyl ester.

Example 105

[R-(R*,R*)]-N$^{\omega}$,N$^{\omega'}$-(2,6-Dibutyl-4-hydroxy-1,7-dioxo-1,7-heptanediyl)bis[N-L-threonyl-L-tyrosine] dimethyl ester is prepared from L-threonyl-L-tyrosine methyl ester.

Example 106

[R-(R*,R*)]-N$^{\omega}$,N$^{\omega'}$-(2,6-Dibutyl-4-hydroxy-1,7-dioxo-1,7-heptanediyl)bis[N-L-threonyl-L-arganine] dimethyl ester is prepared from L-threonyl-L-arganine methyl ester.

Example 107

[R-(R*,R*)]-N$^{\omega}$,N$^{\omega'}$-(2,6-Dibutyl-4-hydroxy-1,7-dioxo-1,7-heptanediyl)bis[N-L-threonyl-L-isoleucine] dimethyl ester is prepared from L-threonyl-L-isoleucine methyl ester.

Example 108

[R-(R*,R*)]-N$^{\omega}$,N$^{\omega'}$-(2,6-Dibutyl-4-hydroxy-1,7-dioxo-1,7-heptanediyl)bis[N-L-threonyl-L-alanine] dimethyl ester is prepared from L-threonyl-L-alanine methyl ester.

Example 109

[R-(R*,R*)]-$N^{\omega}$,$N^{\omega'}$-(2,6-Bis(phenylmethyl)-4-hydroxy-1,7-dioxo-1,7-heptanediyl)bis[N-L-valyl-L-leucine] dimethyl ester is prepared from L-valyl-L-leucine methyl ester.

Example 110

[R-(R*,R*)]-$N^{\omega}$,$N^{\omega'}$-(2,6-Bis(phenylmethyl)-4-hydroxy-1,7-dioxo-1,7-heptanediyl)bis[N-L-valyl-L-phenylalanine] dimethyl ester is prepared from L-valyl-L-phenylalanine methyl ester.
MS (FAB): 877
$^1$H NMR (CDCl3): 7.02 - 7.35 (20H, m), 7.01 (1H, d), 6.83 (1H, m), 6.70 (1H, m), 6.68 (1H, m), 4.72 (2H, m), 4.05 (2H, m), 3.71 (3H, s), 3.67 (3H, s), 3.60 (1H, m), 3.05 (4H, m), 2.60 - 3.00 (6H, m), 1.80 - 2.25 (6H, m). 0.93 (12H, m).

Example 111

[R-(R*,R*)]-$N^{\omega}$,$N^{\omega'}$-(2,6-Bis(phenylmethyl)-4-hydroxy-1,7-dioxo-1,7-heptanediyl)bis[N-L-valyl-L-tyrosine] dimethyl ester is prepared from L-valyl-L-tyrosine methyl ester.

Example 112

[R-(R*,R*)]-$N^{\omega}$,$N^{\omega'}$-(2,6-Bis(phenylmethyl)-4-hydroxy-1,7-dioxo-1,7-heptanediyl)bis[N-L-valyl-L-arganine] dimethyl ester is prepared from L-valyl-L-arganine methyl ester.

Example 113

[R-(R*,R*)]-$N^{\omega}$,$N^{\omega'}$-(2,6-Bis(phenylmethyl)-4-hydroxy-1,7-dioxo-1,7-heptanediyl)bis[N-L-valyl-L-isoleucine] dimethyl ester is prepared from L-valyl-L-isoleucine methyl ester.

Example 114

[R-(R*,R*)]-$N^{\omega}$,$N^{\omega'}$-(2,6-Bis(phenylmethyl)-4-hydroxy-1,7-dioxo-1,7-heptanediyl)bis[N-L-valyl-L-alanine] dimethyl ester is prepared from L-valyl-L-alanine methyl ester.

Example 115

[R-(R*,R*)]-$N^{\omega}$,$N^{\omega'}$-(2,6-Bis(phenylmethyl)-4-hydroxy-1,7-dioxo-1,7-heptanediyl)bis[N-L-leucyl-L-valine] dimethyl ester is prepared from L-leucyl-L-valine methyl ester.

Example 116

[R-(R*,R*)]-$N^{\omega}$,$N^{\omega'}$-(2,6-Bis(phenylmethyl)-4-hydroxy-1,7-dioxo-1,7-heptanediyl)bis[N-L-leucyl-L-leucine] dimethyl ester is prepared from L-leucyl-L-leucine methyl ester.

Example 117

[R-(R*,R*)]-$N^{\omega}$,$N^{\omega'}$-(2,6-Bis(phenylmethyl)-4-hydroxy-1,7-dioxo-1,7-heptanediyl)bis[N-L-leucyl-L-tyrosine] dimethyl ester is prepared from L-leucyl-L-tyrosine methyl ester.

Example 118

[R-(R*,R*)]-$N^{\omega}$,$N^{\omega'}$-(2,6-Bis(phenylmethyl)-4-hydroxy-1,7-dioxo-1,7-heptanediyl)bis-[N-L-leucyl-L-arganine] dimethyl ester is prepared from L-leucyl-L-arganine methyl ester.

Example 119

[R-(R*,R*)]-$N^{\omega}$,$N^{\omega'}$-(2,6-Bis(phenylmethyl)-4-hydroxy-1,7-dioxo-1,7-heptanediyl)bis[N-L-leucyl-L-isoleucine] dimethyl ester is prepared from L-leucyl-L-isoleucine methyl ester.

Example 120

[R-(R*,R*)]-$N^{\omega}$,$N^{\omega'}$-(2,6-Bis(phenylmethyl)-4-hydroxy-1,7-dioxo-1,7-heptanediyl)bis[N-L-leucyl-L-alanine] dimethyl ester is prepared from L-leucyl-L-alanine methyl ester.

Example 121

[R-(R*,R*)]-$N^{\omega}$,$N^{\omega'}$-(2,6-Bis(phenylmethyl)-4-hydroxy-1,7-dioxo-1,7-heptanediyl)bis[N-L-isoleucyl-L-valine] dimethyl ester is prepared from L-isoleucyl-L-valine methyl ester.

Example 122

[R-(R*,R*)]-$N^{\omega}$,$N^{\omega'}$-(2,6-Bis(phenylmethyl)-4-hydroxy-1,7-dioxo-1,7-heptanediyl)bis[N-L-isoleucyl-L-leucine] dimethyl ester is prepared from L-isoleucyl-L-leucine methyl ester.

Example 123

[R-(R*,R*)]-$N^{\omega}$,$N^{\omega'}$-(2,6-Bis(phenylmethyl)-4-hydroxy-1,7-dioxo-1,7-heptanediyl)bis[N-L-isoleucyl-L-phenylalanine] dimethyl ester is prepared from L-isoleucyl-L-phenylalanine methyl ester.

Example 124

[R-(R*,R*)]-$N^{\omega}$,$N^{\omega'}$-(2,6-Bis(phenylmethyl)-4-hydroxy-1,7-dioxo-1,7-heptanediyl)bis[N-L-isoleucyl-L-tyrosine] dimethyl ester is prepared from L-isoleucyl-L-tyrosine methyl ester.

Example 125

[R-(R*,R*)]-$N^{\omega}$,$N^{\omega'}$-(2,6-Bis(phenylmethyl)-4-hydroxy-1,7-dioxo-1,7-heptanediyl)bis[N-L-isoleucyl-L-arganine] dimethyl ester is prepared from L-isoleucyl-L-arganine methyl ester.

Example 126

[R-(R*,R*)]-$N^{\omega}$,$N^{\omega'}$-(2,6-Bis(phenylmethyl)-4-hydroxy-1,7-dioxo-1,7-heptanediyl)bis[N-L-isoleucyl-L-isoleucine] dimethyl ester is prepared from L-isoleucyl-L-isoleucine methyl ester.

Example 127

[R-(R*,R*)]-$N^{\omega}$,$N^{\omega'}$-(2,6-Bis(phenylmethyl)-4-hydroxy-1,7-dioxo-1,7-heptanediyl)bis[N-L-isoleucyl-L-alanine] dimethyl ester is prepared from L-isoleucyl-L-alanine methyl ester.

Example 128

[R-(R*,R*)]-$N^{\omega}$,$N^{\omega'}$-(2,6-Bis(phenylmethyl)-4-hydroxy-1,7-dioxo-1,7-heptanediyl)bis[N-L-asparaginyl-L-valine] dimethyl ester is prepared from L-asparaginyl-L-valine methyl ester.

Example 129

[R-(R*,R*)]-$N^{\omega}$,$N^{\omega'}$-(2,6-Bis(phenylmethyl)-4-hydroxy-1,7-dioxo-1,7-heptanediyl)bis[N-L-asparaginyl-L-leucine] dimethyl ester is prepared from L-asparaginyl-L-leucine methyl ester.

Example 130

[R-(R*,R*)]-$N^{\omega}$,$N^{\omega'}$-(2,6-Bis(phenylmethyl)-4-hydroxy-1,7-dioxo-1,7-heptanediyl)bis[N-L-asparaginyl-L-phenylalanine] dimethyl ester is prepared from L-asparaginyl-L-phenylalanine methyl ester.

Example 131

[R-(R*,R*)]-$N^{\omega}$,$N^{\omega'}$-(2,6-Bis(phenylmethyl)-4-hydroxy-1,7-dioxo-1,7-heptanediyl)bis[N-L-asparaginyl-L-tyrosine] dimethyl ester is prepared from L-asparaginyl-L-tyrosine methyl ester.

Example 132

[R-(R*,R*)]-$N^{\omega}$,$N^{\omega'}$-(2,6-Bis(phenylmethyl)-4-hydroxy-1,7-dioxo-1,7-heptanediyl)bis[N-L-asparaginyl-L-arganine] dimethyl ester is prepared from L-asparaginyl-L-arganine methyl ester.

Example 133

[R-(R*,R*)]-$N^{\omega}$,$N^{\omega'}$-(2,6-Bis(phenylmethyl)-4-hydroxy-1,7-dioxo-1,7-heptanediyl)bis[N-L-asparaginyl-L-isoleucine] dimethyl ester is prepared from L-asparaginyl-L-isoleucine methyl ester.

Example 134

[R-(R*,R*)]-$N^{\omega}$,$N^{\omega'}$-(2,6-Bis(phenylmethyl)-4-hydroxy-1,7-dioxo-1,7-heptanediyl)bis[N-L-asparaginyl-L-alanine] dimethyl ester is prepared from L-asparaginyl-L-alanine methyl ester.

Example 135

[R-(R*,R*)]-$N^{\omega}$,$N^{\omega'}$-(2,6-Bis(phenylmethyl)-4-hydroxy-1,7-dioxo-1,7-heptanediyl)bis[N-L-glutaminyl-L-valine] dimethyl ester is prepared from L-glutaminyl-L-valine methyl ester.

Example 136

[R-(R*,R*)]-$N^{\omega}$,$N^{\omega'}$-(2,6-Bis(phenylmethyl)-4-hydroxy-1,7-dioxo-1,7-heptanediyl)bis[N-L-glutaminyl-L-leucine] dimethyl ester is prepared from L-glutaminyl-L-leucine methyl ester.

Example 137

[R-(R*,R*)]-$N^{\omega}$,$N^{\omega'}$-(2,6-Bis(phenylmethyl)-4-hydroxy-1,7-dioxo-1,7-heptanediyl)bis[N-L-glutaminyl-L-phenylalanine] dimethyl ester is prepared from L-glutaminyl-L-phenylalanine methyl ester.

Example 138

[R-(R*,R*)]-$N^{\omega}$,$N^{\omega'}$-(2,6-Bis(phenylmethyl)-4-hydroxy-1,7-dioxo-1,7-heptanediyl)bis[N-L-glutaminyl-L-tyrosine] dimethyl ester is prepared from L-glutaminyl-L-tyrosine methyl ester.

Example 139

[R-(R*,R*)]-$N^{\omega}$,$N^{\omega'}$-(2,6-Bis(phenylmethyl)-4-hydroxy-1,7-dioxo-1,7-heptanediyl)bis[N-L-glutaminyl-L-arganine] dimethyl ester is prepared from L-glutaminyl-L-arganine methyl ester.

Example 140

[R-(R*,R*)]-$N^{\omega}$,$N^{\omega'}$-(2,6-Bis(phenylmethyl)-4-hydroxy-1,7-dioxo-1,7-heptanediyl)bis[N-L-glutaminyl-L-isoleucine] dimethyl ester is prepared from L-glutaminyl-L-isoleucine methyl ester.

Example 141

[R-(R*,R*)]-$N^{\omega}$,$N^{\omega'}$-(2,6-Bis(phenylmethyl)-4-hydroxy-1,7-dioxo-1,7-heptanediyl)bis[N-L-glutaminyl-L-alanine] dimethyl ester is prepared from L-glutaminyl-L-alanine methyl ester.

Example 142

[R-(R*,R*)]-$N^{\omega}$,$N^{\omega'}$-(2,6-Bis(phenylmethyl)-4-hydroxy-1,7-dioxo-1,7-heptanediyl)bis[N-L-alanyl-L-valine] dimethyl ester is prepared from L-alanyl-L-valine methyl ester.

Example 143

[R-(R*,R*)]-$N^{\omega}$,$N^{\omega'}$-(2,6-Bis(phenylmethyl)-4-hydroxy-1,7-dioxo-1,7-heptanediyl)bis[N-L-alanyl-L-leucine]

dimethyl ester is prepared from L-alanyl-L-leucine methyl ester.

Example 144

[R-(R*,R*)]-$N^{\omega}$,$N^{\omega'}$-(2,6-Bis(phenylmethyl)-4-hydroxy-1,7-dioxo-1,7-heptanediyl)bis[N-L-alanyl-L-phenylalanine] dimethyl ester is prepared from L-alanyl-L-phenylalanine methyl ester.

Example 145

[R-(R*,R*)]-$N^{\omega}$,$N^{\omega'}$-(2,6-Bis(phenylmethyl)-4-hydroxy-1,7-dioxo-1,7-heptanediyl)bis[N-L-alanyl-L-tyrosine] dimethyl ester is prepared from L-alanyl-L-tyrosine methyl ester.

Example 146

[R-(R*,R*)]-$N^{\omega}$,$N^{\omega'}$-(2,6-Bis(phenylmethyl)-4-hydroxy-1,7-dioxo-1,7-heptanediyl)bis[N-L-alanyl-L-arganine] dimethyl ester is prepared from L-alanyl-L-arganine methyl ester.

Example 147

[R-(R*,R*)]-$N^{\omega}$,$N^{\omega'}$-(2,6-Bis(phenylmethyl)-4-hydroxy-1,7-dioxo-1,7-heptanediyl)bis[N-L-alanyl-L-isoleucine] dimethyl ester is prepared from L-alanyl-L-isoleucine methyl ester.

Example 148

[R-(R*,R*)]-$N^{\omega}$,$N^{\omega'}$-(2,6-Bis(phenylmethyl)-4-hydroxy-1,7-dioxo-1,7-heptanediyl)bis[N-L-threonyl-L-valine] dimethyl ester is prepared from L-threonyl-L-valine methyl ester.

Example 149

[R-(R*,R*)]-$N^{\omega}$,$N^{\omega'}$-(2,6-Bis(phenylmethyl)-4-hydroxy-1,7-dioxo-1,7-heptanediyl)bis[N-L-threonyl-L-leucine] dimethyl ester is prepared from L-threonyl-L-leucine methyl ester.

Example 150

[R-(R*,R*)]-$N^{\omega}$,$N^{\omega'}$-(2,6-Bis(phenylmethyl)-4-hydroxy-1,7-dioxo-1,7-heptanediyl)bis[N-L-threonyl-L-phenylalanine] dimethyl ester is prepared from L-threonyl-L-phenylalanine methyl ester.

Example 151

[R-(R*,R*)]-$N^{\omega}$,$N^{\omega'}$-(2,6-Bis(phenylmethyl)-4-hydroxy-1,7-dioxo-1,7-heptanediyl)bis[N-L-threonyl-L-tyrosine] dimethyl ester is prepared from L-threonyl-L-tyrosine methyl ester.

Example 152

[R-(R*,R*)]-$N^{\omega}$,$N^{\omega'}$-(2,6-Bis(phenylmethyl)-4-hydroxy-1,7-dioxo-1,7-heptanediyl)bis[N-L-threonyl-L-arganine] dimethyl ester is prepared from L-threonyl-L-arganine methyl ester.

Example 153

[R-(R*,R*)]-$N^{\omega}$,$N^{\omega'}$-(2,6-Bis(phenylmethyl)-4-hydroxy-1,7-dioxo-1,7-heptanediyl)bis[N-L-threonyl-L-isoleucine] dimethyl ester is prepared from L-threonyl-L-isoleucine methyl ester.

Example 154

[R-(R*,R*)]-$N^{\omega}$,$N^{\omega'}$-(2,6-Bis(phenylmethyl)-4-hydroxy-1,7-dioxo-1,7-heptanediyl)bis[N-L-threonyl-L-alanine] dimethyl ester is prepared from L-threonyl-L-alanine methyl ester.

Example 155

[R-(R*,R*)]-$N^{\omega}$,$N^{\omega'}$-(2,6-Bis(phenylmethyl)-4-hydroxy-1,7-dioxo-1,7-heptanediyl)bis[N-L-alanyl-L-alanine] dimethyl ester is prepared from L-alanyl-L-alanine methyl ester.

Examples 156-169

Substantially following the methods described in detail hereinabove in Examples 6-8, 15-20 and 28-33, the compounds of this invention listed below in examples 156-169 are prepared using the aminoacid ester indicated.

Example 156

[R-(R*,R*)]-$N^{\omega}$,$N^{\omega'}$-(2,6-Dibutyl-4-hydroxy-1,7-dioxo-1,7-heptanediyl)bis[N-L-valine] bis(phenylmethyl) ester is prepared from L-valine phenylmethyl ester.

Example 157

[R-(R*,R*)]-$N^{\omega}$,$N^{\omega'}$-(2,6-Dibutyl-4-hydroxy-1,7-dioxo-1,7-heptanediyl)bis[N-L-leucine] bis(phenylmethyl) ester is prepared from L-leucine phenylmethyl ester.

Example 158

[R-(R*,R*)]-$N^{\omega}$,$N^{\omega'}$-(2,6-Dibutyl-4-hydroxy-1,7-dioxo-1,7-heptanediyl)bis[N-L-isoleucine] bis(phenylmethyl) ester is prepared from L-isoleucine phenylmethyl ester.

Example 159

[R-(R*,R*)]-$N^{\omega}$,$N^{\omega'}$-(2,6-Dibutyl-4-hydroxy-1,7-dioxo-1,7-heptanediyl)bis[N-L-asparagine] bis(phenylmethyl) ester is prepared from L-asparagine phenylmethyl ester.

Example 160

[R-(R*,R*)]-$N^{\omega}$,$N^{\omega'}$-(2,6-Dibutyl-4-hydroxy-1,7-dioxo-1,7-heptanediyl)bis[N-L-glutamine] bis(phenylmethyl) ester is prepared from L-glutamine phenylmethyl ester.

Example 161

[R-(R*,R*)]-$N^{\omega}$,$N^{\omega'}$-(2,6-Dibutyl-4-hydroxy-1,7-dioxo-1,7-heptanediyl)bis[N-L-alanine] bis(phenylmethyl) ester is prepared from L-alanine phenylmethyl ester.

Example 162

[R-(R*,R*)]-$N^{\omega}$,$N^{\omega'}$-(2,6-Dibutyl-4-hydroxy-1,7-dioxo-1,7-heptanediyl)bis[N-L-threonine] bis(phenylmethyl) ester is prepared from L-threonine phenylmethyl ester.

Example 163

[R-(R*,R*)]-$N^{\omega}$,$N^{\omega'}$-(2,6-Bis(phenylmethyl)-4-hydroxy-1,7-dioxo-1,7-heptanediyl)bis[N-L-valine] bis-(phenylmethyl) ester is prepared from L-valine phenylmethyl ester.

Example 164

[R-(R*,R*)]-$N^{\omega}$,$N^{\omega'}$-(2,6-Bis(phenylmethyl)-4-hydroxy-1,7-dioxo-1,7-heptanediyl)bis[N-L-leucine] bis-(phenylmethyl) ester is prepared from L-leucine phenylmethyl ester.

Example 165

[R-(R*,R*)]-$N^{\omega}$,$N^{\omega'}$-(2,6-Bis(phenylmethyl)-4-hydroxy-1,7-dioxo-1,7-heptanediyl)bis[N-L-isoleucine] bis-(phenylmethyl) ester is prepared from L-isoleucine phenylmethyl ester.

Example 166

[R-(R*,R*)]-N$^{\omega}$,N$^{\omega'}$-(2,6-Bis(phenylmethyl)-4-hydroxy-1,7-dioxo-1,7-heptanediyl)bis[N-L-asparagine] bis-(phenylmethyl) ester is prepared from L-asparagine phenylmethyl ester.

Example 167

[R-(R*,R*)]-N$^{\omega}$,N$^{\omega'}$-(2,6-Bis(phenylmethyl)-4-hydroxy-1,7-dioxo-1,7-heptanediyl)bis[N-L-glutamine] bis-(phenylmethyl) ester is prepared from L-glutamine phenylmethyl ester.

Example 168

[R-(R*,R*)]-N$^{\omega}$,N$^{\omega'}$-(2,6-Bis(phenylmethyl)-4-hydroxy-1,7-dioxo-1,7-heptanediyl)bis[N-L-alanine] bis-(phenylmethyl) ester is prepared from L-alanine phenylmethyl ester.

Example 169

[R-(R*,R*)]-N$^{\omega}$,N$^{\omega'}$-(2,6-Bis(phenylmethyl)-4-hydroxy-1,7-dioxo-1,7-heptanediyl)bis[N-L-threonine] bis-(phenylmethyl) ester is prepared from L-threonine phenylmethyl ester.

Examples 170-249

Substantially following the methods described in detail hereinabove in Examples 6-11 and 27-29 the compounds of this invention listed below in examples 170-249 are prepared using the dipeptide ester indicated.

Example 170

[R-(R*,R*)]-N$^{\omega}$,N$^{\omega'}$-[Oxiranylidenebis(2-butyl-1-oxo-3,1-propanediyl)]bis[N-L-valyl-L-leucine] dimethyl ester is prepared from L-valyl-L-leucine methyl ester.

Example 171

[R-(R*,R*)]-N$^{\omega}$,N$^{\omega'}$-[Oxiranylidenebis(2-butyl-1-oxo-3,1-propanediyl)]bis[N-L-valyl-L-phenylalanine] dimethyl ester is prepared from L-valyl-L-phenylalanine methyl ester.

Example 172

[R-(R*,R*)]-N$^{\omega}$,N$^{\omega'}$-[Oxiranylidenebis(2-butyl-1-oxo-3,1-propanediyl)]bis[N-L-valyl-L-tyrosine] dimethyl ester is prepared from L-valyl-L-tyrosine methyl ester.

Example 173

[R-(R*,R*)]-N$^{\omega}$,N$^{\omega'}$-[Oxiranylidenebis(2-butyl-1-oxo-3,1-propanediyl)]bis[N-L-valyl-L-isoleucine] dimethyl ester is prepared from L-valyl-L-isoleucine methyl ester.

Example 174

[R-(R*,R*)]-N$^{\omega}$,N$^{\omega'}$-[Oxiranylidenebis(2-butyl-1-oxo-3,1-propanediyl)]bis[N-L-valyl-L-alanine] dimethyl ester is prepared from L-valyl-L-alanine methyl ester.

Example 175

[R-(R*,R*)]-N$^{\omega}$,N$^{\omega'}$-[Oxiranylidenebis(2-butyl-1-oxo-3,1-propanediyl)]bis[N-L-leucyl-L-valine] dimethyl ester is prepared from L-leucyl-L-valine methyl ester.

Example 176

[R-(R*,R*)]-$N^{\omega}$,$N^{\omega'}$-[Oxiranylidenebis(2-butyl-1-oxo-3,1-propanediyl)]bis[N-L-leucyl-L-leucine] dimethyl ester is prepared from L-leucyl-L-leucine methyl ester.

Example 177

[R-(R*,R*)]-$N^{\omega}$,$N^{\omega'}$-[Oxiranylidenebis(2-butyl-1-oxo-3,1-propanediyl)]bis[N-L-leucyl-L-tyrosine] dimethyl ester is prepared from L-leucyl-L-tyrosine methyl ester.

Example 178

[R-(R*,R*)]-$N^{\omega}$,$N^{\omega'}$-[Oxiranylidenebis(2-butyl-1-oxo-3,1-propanediyl)]bis[N-L-leucyl-L-isoleucine] dimethyl ester is prepared from L-leucyl-L-isoleucine methyl ester

Example 179

[R-(R*,R*)]-$N^{\omega}$,$N^{\omega'}$-[Oxiranylidenebis(2-butyl-1-oxo-3,1-propanediyl)]bis[N-L-leucyl-L-alanine] dimethyl ester is prepared from L-leucyl-L-alanine methyl ester.

Example 180

[R-(R*,R*)]-$N^{\omega}$,$N^{\omega'}$-[Oxiranylidenebis(2-butyl-1-oxo-3,1-propanediyl)]bis[N-L-isoleucyl-L-valine] dimethyl ester is prepared from L-isoleucyl-L-valine methyl ester.

Example 181

[R-(R*,R*)]-$N^{\omega}$,$N^{\omega'}$-[Oxiranylidenebis(2-butyl-1-oxo-3,1-propanediyl)]bis[N-L-isoleucyl-L-leucine] dimethyl ester is prepared from L-isoleucyl-L-leucine methyl ester.

Example 182

[R-(R*,R*)]-$N^{\omega}$,$N^{\omega'}$-[Oxiranylidenebis(2-butyl-1-oxo-3,1-propanediyl)]bis[N-L-isoleucyl-L-phenylalanine] dimethyl ester is prepared from L-isoleucyl-L-phenylalanine methyl ester.

Example 183

[R-(R*,R*)]-$N^{\omega}$,$N^{\omega'}$-[Oxiranylidenebis(2-butyl-1-oxo-3,1-propanediyl)]bis[N-L-isoleucyl-L-tyrosine] dimethyl ester is prepared from L-isoleucyl-L-tyrosine methyl ester.

Example 184

[R-(R*,R*)]-$N^{\omega}$,$N^{\omega'}$-[Oxiranylidenebis(2-butyl-1-oxo-3,1-propanediyl)]bis[N-L-isoleucyl-L-isoleucine] dimethyl ester is prepared from L-isoleucyl-L-isoleucine methyl ester.

Example 185

[R-(R*,R*)]-$N^{\omega}$,$N^{\omega'}$-[Oxiranylidenebis(2-butyl-1-oxo-3,1-propanediyl)]bis[N-L-isoleucyl-L-alanine] dimethyl ester is prepared from L-isoleucyl-L-alanine methyl ester.

Example 186

[R-(R*,R*)]-$N^{\omega}$,$N^{\omega'}$-[Oxiranylidenebis(2-butyl-1-oxo-3,1-propanediyl)]bis[N-L-alanyl-L-valine] dimethyl ester is prepared from L-alanyl-L-valine methyl ester.

Example 187

[R-(R*,R*)]-$N^{\omega}$,$N^{\omega'}$-[Oxiranylidenebis(2-butyl-1-oxo-3,1-propanediyl)]bis[N-L-alanyl-L-leucine] dimethyl ester is prepared from L-alanyl-L-leucine methyl ester.

Example 188

[R-(R*,R*)]-$N^{\omega}$,$N^{\omega'}$-[Oxiranylidenebis(2-butyl-1-oxo-3,1-propanediyl)]bis[N-L-alanyl-L-phenylalanine] dimethyl ester is prepared from L-alanyl-L-phenylalanine methyl ester.

Example 189

[R-(R*,R*)]-$N^{\omega}$,$N^{\omega'}$-[Oxiranylidenebis(2-butyl-1-oxo-3,1-propanediyl)]bis[N-L-alanyl-L-tyrosine] dimethyl ester is prepared from L-alanyl-L-tyrosine methyl ester.

Example 190

[R-(R*,R*)]-$N^{\omega}$,$N^{\omega'}$-[Oxiranylidenebis(2-butyl-1-oxo-3,1-propanediyl)]bis[N-L-alanyl-L-isoleucine] dimethyl ester is prepared from L-alanyl-L-isoleucine methyl ester.

Example 191

[R-(R*,R*)]-$N^{\omega}$,$N^{\omega'}$-[Oxiranylidenebis(2-butyl-1-oxo-3,1-propanediyl)]bis[N-L-asparaginyl-L-valine] dimethyl ester is prepared from L-asparaginyl-L-valine methyl ester.

Example 192

[R-(R*,R*)]-$N^{\omega}$,$N^{\omega'}$-[Oxiranylidenebis(2-butyl-1-oxo-3,1-propanediyl)]bis[N-L-asparaginyl-L-leucine] dimethyl ester is prepared from L-asparaginyl-L-leucine methyl ester.

Example 193

[R-(R*,R*)]-$N^{\omega}$,$N^{\omega'}$-[Oxiranylidenebis(2-butyl-1-oxo-3,1-propanediyl)]bis[N-L-asparaginyl-L-phenylalanine] dimethyl ester is prepared from L-asparaginyl-L-phenylalanine methyl ester.

Example 194

[R-(R*,R*)]-$N^{\omega}$,$N^{\omega'}$-[Oxiranylidenebis(2-butyl-1-oxo-3,1-propanediyl)]bis[N-L-asparaginyl-L-tyrosine] dimethyl ester is prepared from L-asparaginyl-L-tyrosine methyl ester.

Example 195

[R-(R*,R*)]-$N^{\omega}$,$N^{\omega'}$-[Oxiranylidenebis(2-butyl-1-oxo-3,1-propanediyl)]bis[N-L-asparaginyl-L-isoleucine] dimethyl ester is prepared from L-asparaginyl-L-isoleucine methyl ester.

Example 196

[R-(R*,R*)]-$N^{\omega}$,$N^{\omega'}$-[Oxiranylidenebis(2-butyl-1-oxo-3,1-propanediyl)]bis[N-L-asparaginyl-L-alanine] dimethyl ester is prepared from L-asparaginyl-L-alanine methyl ester.

Example 197

[R-(R*,R*)]-$N^{\omega}$,$N^{\omega'}$-[Oxiranylidenebis(2-butyl-1-oxo-3,1-propanediyl)]bis[N-L-glutaminyl-L-valine] dimethyl ester is prepared from L-glutaminyl-L-valine methyl ester.

Example 198

[R-(R*,R*)]-$N^{\omega}$,$N^{\omega'}$-[Oxiranylidenebis(2-butyl-1-oxo-3,1-propanediyl)]bis[N-L-glutaminyl-L-leucine] dimethyl ester is prepared from L-glutaminyl-L-leucine methyl ester.

Example 199

[R-(R*,R*)]-$N^{\omega}$,$N^{\omega'}$-[Oxiranylidenebis(2-butyl-1-oxo-3,1-propanediyl)]bis[N-L-glutaminyl-L-phenylalanine]

dimethyl ester is prepared from L-glutaminyl-L-phenylalanine methyl ester.

Example 200

[R-(R*,R*)]-$N^{\omega}$,$N^{\omega'}$-[Oxiranylidenebis(2-butyl-1-oxo-3,1-propanediyl)]bis[N-L-glutaminyl-L-tyrosine] dimethyl ester is prepared from L-glutaminyl-L-tyrosine methyl ester.

Example 201

[R-(R*,R*)]-$N^{\omega}$,$N^{\omega'}$-[Oxiranylidenebis(2-butyl-1-oxo-3,1-propanediyl)]bis[N-L-glutaminyl-L-isoleucine] dimethyl ester is prepared from L-glutaminyl-L-isoleucine methyl ester.

Example 202

[R-(R*,R*)]-$N^{\omega}$,$N^{\omega'}$-[Oxiranylidenebis(2-butyl-1-oxo-3,1-propanediyl)]bis[N-L-glutaminyl-L-alanine] dimethyl ester is prepared from L-glutaminyl-L-alanine methyl ester.

Example 203

[R-(R*,R*)]-$N^{\omega}$,$N^{\omega'}$-[Oxiranylidenebis(2-butyl-1-oxo-3,1-propanediyl)]bis[N-L-threonyl-L-valine] dimethyl ester is prepared from L-threonyl-L-valine methyl ester.

Example 204

[R-(R*,R*)]-$N^{\omega}$,$N^{\omega'}$-[Oxiranylidenebis(2-butyl-1-oxo-3,1-propanediyl)]bis[N-L-threonyl-L-leucine] dimethyl ester is prepared from L-threonyl-L-leucine methyl ester.

Example 205

[R-(R*,R*)]-$N^{\omega}$,$N^{\omega'}$-[Oxiranylidenebis(2-butyl-1-oxo-3,1-propanediyl)]bis[N-L-threonyl-L-phenylalanine] dimethyl ester is prepared from L-threonyl-L-phenylalanine methyl ester.

Example 206

[R-(R*,R*)]-$N^{\omega}$,$N^{\omega'}$-[Oxiranylidenebis(2-butyl-1-oxo-3,1-propanediyl)]bis[N-L-threonyl-L-tyrosine] dimethyl ester is prepared from L-threonyl-L-tyrosine methyl ester.

Example 207

[R-(R*,R*)]-$N^{\omega}$,$N^{\omega'}$-[Oxiranylidenebis(2-butyl-1-oxo-3,1-propanediyl)]bis[N-L-threonyl-L-isoleucine] dimethyl ester is prepared from L-threonyl-L-isoleucine methyl ester.

Example 208

[R-(R*,R*)]-$N^{\omega}$,$N^{\omega'}$-[Oxiranylidenebis(2-butyl-1-oxo-3,1-propanediyl)]bis[N-L-threonyl-L-alanine] dimethyl ester is prepared from L-threonyl-L-alanine methyl ester.

Example 209

[R-(R*,R*)]-$N^{\omega}$,$N^{\omega'}$-[Oxiranylidenebis(2-(phenylmethyl)-1-oxo-3,1-propanediyl)]bis[N-L-valyl-L-valine] dimethyl ester is prepared from L-valyl-L-valine methyl ester.

Example 210

[R-(R*,R*)]-$N^{\omega}$,$N^{\omega'}$-[Oxiranylidenebis(2-(phenylmethyl)-1-oxo-3,1-propanediyl)]bis[N-L-valyl-L-leucine] dimethyl ester is prepared from L-valyl-L-leucine methyl ester.

Example 211

[R-(R*,R*)]-$N^{\omega}$,$N^{\omega'}$-[Oxiranylidenebis(2-(phenylmethyl)-1-oxo-3,1-propanediyl)]bis[N-L-valyl-L-phenylalanine] dimethyl ester is prepared from L-valyl-L-phenylalanine methyl ester.

Example 212

[R-(R*,R*)]-$N^{\omega}$,$N^{\omega'}$-[Oxiranylidenebis(2-(phenylmethyl)-1-oxo-3,1-propanediyl)]bis[N-L-valyl-L-tyrosine] dimethyl ester is prepared from L-valyl-L-tyrosine methyl ester.

Example 213

[R-(R*,R*)]-$N^{\omega}$,$N^{\omega'}$-[Oxiranylidenebis(2-(phenylmethyl)-1-oxo-3,1-propanediyl)]bis[N-L-valyl-L-isoleucine] dimethyl ester is prepared from L-valyl-L-isoleucine methyl ester.

Example 214

[R-(R*,R*)]-$N^{\omega}$,$N^{\omega'}$-[Oxiranylidenebis(2-(phenylmethyl)-1-oxo-3,1-propanediyl)]bis[N-L-valyl-L-alanine] dimethyl ester is prepared from L-valyl-L-alanine methyl ester.

Example 215

[R-(R*,R*)]-$N^{\omega}$,$N^{\omega'}$-[Oxiranylidenebis(2-(phenylmethyl)-1-oxo-3,1-propanediyl)]bis[N-L-leucyl-L-valine] dimethyl ester is prepared from L-leucyl-L-valine methyl ester.

Example 216

[R-(R*,R*)]-$N^{\omega}$,$N^{\omega'}$-[Oxiranylidenebis(2-(phenylmethyl)-1-oxo-3,1-propanediyl)]bis[N-L-leucyl-L-leucine] dimethyl ester is prepared from L-leucyl-L-leucine methyl ester.

Example 217

[R-(R*,R*)]-$N^{\omega}$,$N^{\omega'}$-[Oxiranylidenebis(2-(phenylmethyl)-1-oxo-3,1-propanediyl)]bis[N-L-leucyl-L-phenylalanine] dimethyl ester is prepared from L-leucyl-L-phenylalanine methyl ester.

Example 218

[R-(R*,R*)]-$N^{\omega}$,$N^{\omega'}$-[Oxiranylidenebis(2-(phenylmethyl)-1-oxo-3,1-propanediyl)]bis[N-L-leucyl-L-tyrosine] dimethyl ester is prepared from L-leucyl-L-tyrosine methyl ester.

Example 219

[R-(R*,R*)]-$N^{\omega}$,$N^{\omega'}$-[Oxiranylidenebis(2-(phenylmethyl)-1-oxo-3,1-propanediyl)]bis[N-L-leucyl-L-isoleucine] dimethyl ester is prepared from L-leucyl-L-isoleucine methyl ester.

Example 220

[R-(R*,R*)]-$N^{\omega}$,$N^{\omega'}$-[Oxiranylidenebis(2-(phenylmethyl)-1-oxo-3,1-propanediyl)]bis[N-L-leucyl-L-alanine] dimethyl ester is prepared from L-leucyl-L-alanine methyl ester.

Example 221

[R-(R*,R*)]-$N^{\omega}$,$N^{\omega'}$-[Oxiranylidenebis(2-(phenylmethyl)-1-oxo-3,1-propanediyl)]bis[N-L-isoleucyl-L-valine] dimethyl ester is prepared from L-isoleucyl-L-valine methyl ester.

Example 221

[R-(R*,R*)]-$N^{\omega}$,$N^{\omega'}$-[Oxiranylidenebis(2-(phenylmethyl)-1-oxo-3,1-propanediyl)]bis[N-L-isoleucyl-L-leucine] dimethyl ester is prepared from L-isoleucyl-L-leucine methyl ester.

Example 222

[R-(R*,R*)]-$N^{\omega}$,$N^{\omega'}$-[Oxiranylidenebis(2-(phenylmethyl)-1-oxo-3,1-propanediyl)]bis[N-L-isoleucyl-L-phenylalanine] dimethyl ester is prepared from L-isoleucyl-L-phenylalanine methyl ester.

Example 223

[R-(R*,R*)]-$N^{\omega}$,$N^{\omega'}$-[Oxiranylidenebis(2-(phenylmethyl)-1-oxo-3,1-propanediyl)]bis[N-L-isoleucyl-L-tyrosine] dimethyl ester is prepared from L-isoleucyl-L-tyrosine methyl ester.

Example 224

[R-(R*,R*)]-$N^{\omega}$,$N^{\omega'}$-[Oxiranylidenebis(2-(phenylmethyl)-1-oxo-3,1-propanediyl)]bis[N-L-isoleucyl-L-isoleucine] dimethyl ester is prepared from L-isoleucyl-L-isoleucine methyl ester.

Example 225

[R-(R*,R*)]-$N^{\omega}$,$N^{\omega'}$-[Oxiranylidenebis(2-(phenylmethyl)-1-oxo-3,1-propanediyl)]bis[N-L-isoleucyl-L-alanine] dimethyl ester is prepared from L-isoleucyl-L-alanine methyl ester.

Example 226

[R-(R*,R*)]-$N^{\omega}$,$N^{\omega'}$-[Oxiranylidenebis(2-(phenylmethyl)-1-oxo-3,1-propanediyl)]bis[N-L-alanyl-L-valine] dimethyl ester is prepared from L-alanyl-L-valine methyl ester.

Example 227

[R-(R*,R*)]-$N^{\omega}$,$N^{\omega'}$-[Oxiranylidenebis(2-(phenylmethyl)-1-oxo-3,1-propanediyl)]bis[N-L-alanyl-L-leucine] dimethyl ester is prepared from L-alanyl-L-leucine methyl ester.

Example 228

[R-(R*,R*)]-$N^{\omega}$,$N^{\omega'}$-[Oxiranylidenebis(2-(phenylmethyl)-1-oxo-3,1-propanediyl)]bis[N-L-alanyl-L-phenylalanine] dimethyl ester is prepared from L-alanyl-L-phenylalanine methyl ester.

Example 229

[R-(R*,R*)]-$N^{\omega}$,$N^{\omega'}$-[Oxiranylidenebis(2-(phenylmethyl)-1-oxo-3,1-propanediyl)]bis[N-L-alanyl-L-tyrosine] dimethyl ester is prepared from L-alanyl-L-tyrosine methyl ester.

Example 230

[R-(R*,R*)]-$N^{\omega}$,$N^{\omega'}$-[Oxiranylidenebis(2-(phenylmethyl)-1-oxo-3,1-propanediyl)]bis[N-L-alanyl-L-isoleucine] dimethyl ester is prepared from L-alanyl-L-isoleucine methyl ester.

Example 231

[R-(R*,R*)]-$N^{\omega}$,$N^{\omega'}$-[Oxiranylidenebis(2-(phenylmethyl)-1-oxo-3,1-propanediyl)]bis[N-L-alanyl-L-alanine] dimethyl ester is prepared from L-alanyl-L-alanine methyl ester.

Example 232

[R-(R*,R*)]-$N^{\omega}$,$N^{\omega'}$-[Oxiranylidenebis(2-(phenylmethyl)-1-oxo-3,1-propanediyl)]bis[N-L-asparaginyl-L-valine] dimethyl ester is prepared from L-asparaginyl-L-valine methyl ester.

Example 233

[R-(R*,R*)]-$N^{\omega}$,$N^{\omega'}$-[Oxiranylidenebis(2-(phenylmethyl)-1-oxo-3,1-propanediyl)]bis[N-L-asparaginyl-L-leucine]

dimethyl ester is prepared from L-asparaginyl-L-leucine methyl ester.

Example 234

[R-(R*,R*)]-N$^{\omega}$,N$^{\omega'}$-[Oxiranylidenebis(2-(phenylmethyl)-1-oxo-3,1-propanediyl)]bis[N-L-asparaginyl-L-phenylalanine] dimethyl ester is prepared from L-asparaginyl-L-phenylalanine methyl ester.

Example 235

[R-(R*,R*)]-N$^{\omega}$,N$^{\omega'}$-[Oxiranylidenebis(2-(phenylmethyl)-1-oxo-3,1-propanediyl)]bis[N-L-asparaginyl-L-tyrosine] dimethyl ester is prepared from L-asparaginyl-L-tyrosine methyl ester.

Example 236

[R-(R*,R*)]-N$^{\omega}$,N$^{\omega'}$-[Oxiranylidenebis(2-(phenylmethyl)-1-oxo-3,1-propanediyl)]bis[N-L-asparaginyl-L-isoleucine] dimethyl ester is prepared from L-asparaginyl-L-isoleucine methyl ester.

Example 237

[R-(R*,R*)]-N$^{\omega}$,N$^{\omega'}$-[Oxiranylidenebis(2-(phenylmethyl)-1-oxo-3,1-propanediyl)]bis[N-L-asparaginyl-L-alanine] dimethyl ester is prepared from L-asparaginyl-L-alanine methyl ester.

Example 238

[R-(R*,R*)]-N$^{\omega}$,N$^{\omega'}$-[Oxiranylidenebis(2-(phenylmethyl)-1-oxo-3,1-propanediyl)]bis[N-L-glutaminyl-L-valine] dimethyl ester is prepared from L-glutaminyl-L-valine methyl ester.

Example 239

[R-(R*,R*)]-N$^{\omega}$,N$^{\omega'}$-[Oxiranylidenebis(2-(phenylmethyl)-1-oxo-3,1-propanediyl)]bis[N-L-glutaminyl-L-leucine] dimethyl ester is prepared from L-glutaminyl-L-leucine methyl ester.

Example 240

[R-(R*,R*)]-N$^{\omega}$,N$^{\omega'}$-[Oxiranylidenebis(2-(phenylmethyl)-1-oxo-3,1-propanediyl)]bis[N-L-glutaminyl-L-phenylalanine] dimethyl ester is prepared from L-glutaminyl-L-phenylalanine methyl ester.

Example 241

[R-(R*,R*)]-N$^{\omega}$,N$^{\omega'}$-[Oxiranylidenebis(2-(phenylmethyl)-1-oxo-3,1-propanediyl)]bis[N-L-glutaminyl-L-tyrosine] dimethyl ester is prepared from L-glutaminyl-L-tyrosine methyl ester.

Example 242

[R-(R*,R*)]-N$^{\omega}$,N$^{\omega'}$-[Oxiranylidenebis(2-(phenylmethyl)-1-oxo-3,1-propanediyl)]bis[N-L-glutaminyl-L-isoleucine] dimethyl ester is prepared from L-glutaminyl-L-isoleucine methyl ester.

Example 243

[R-(R*,R*)]-N$^{\omega}$,N$^{\omega'}$-[Oxiranylidenebis(2-(phenylmethyl)-1-oxo-3,1-propanediyl)]bis[N-L-glutaminyl-L-alanine] dimethyl ester is prepared from L-glutaminyl-L-alanine methyl ester.

Example 244

[R-(R*,R*)]-N$^{\omega}$,N$^{\omega'}$-[Oxiranylidenebis(2-(phenylmethyl)-1-oxo-3,1-propanediyl)]bis[N-L-threonyl-L-valine] dimethyl ester is prepared from L-threonyl-L-valine methyl ester.

Example 245

[R-(R*,R*)]-$N^{\omega}$,$N^{\omega'}$-[Oxiranylidenebis(2-(phenylmethyl)-1-oxo-3,1-propanediyl)]bis[N-L-threonyl-L-leucine] dimethyl ester is prepared from L-threonyl-L-leucine methyl ester.

Example 246

[R-(R*,R*)]-$N^{\omega}$,$N^{\omega'}$-[Oxiranylidenebis(2-(phenylmethyl)-1-oxo-3,1-propanediyl)]bis[N-L-threonyl-L-phenylalanine] dimethyl ester is prepared from L-threonyl-L-phenylalanine methyl ester.

Example 247

[R-(R*,R*)]-$N^{\omega}$,$N^{\omega'}$-[Oxiranylidenebis(2-(phenylmethyl)-1-oxo-3,1-propanediyl)]bis[N-L-threonyl-L-tyrosine] dimethyl ester is prepared from L-threonyl-L-tyrosine methyl ester.

Example 248

[R-(R*,R*)]-$N^{\omega}$,$N^{\omega'}$-[Oxiranylidenebis(2-(phenylmethyl)-1-oxo-3,1-propanediyl)]bis[N-L-threonyl-L-isoleucine] dimethyl ester is prepared from L-threonyl-L-isoleucine methyl ester.

Example 249

[R-(R*,R*)]-$N^{\omega}$,$N^{\omega'}$-[Oxiranylidenebis(2-(phenylmethyl)-1-oxo-3,1-propanediyl)]bis[N-L-threonyl-L-alanine] dimethyl ester is prepared from L-threonyl-L-alanine methyl ester.

Examples 250-263

Substantially following the methods described in detail hereinabove in Examples 6-11 and 27-29 the compounds of this invention listed below in examples 250-263 are prepared using the aminoacid ester indicated.

Example 250

[R-(R*,R*)]-$N^{\omega}$,$N^{\omega'}$-Oxiranylidenbis(2-butyl-1-oxo-3,1-propanediyl)]bis[N-L-valine] bis(phenylmethyl) ester is prepared from L-valine (phenylmethyl)ester.

Example 251

[R-(R*,R*)]-$N^{\omega}$,$N^{\omega'}$-Oxiranylidenbis(2-butyl-1-oxo-3,1-propanediyl)]bis[N-L-leucine] bis(phenylmethyl) ester is prepared from L-leucine (phenylmethyl)ester.

Example 252

[R-(R*,R*)]-$N^{\omega}$,$N^{\omega'}$-Oxiranylidenbis(2-butyl-1-oxo-3,1-propanediyl)]bis[N-L-isoleucine] bis(phenylmethyl) ester is prepared from L-isoleucine (phenylmethyl)ester.

Example 253

[R-(R*,R*)]-$N^{\omega}$,$N^{\omega'}$-Oxiranylidenbis(2-butyl-1-oxo-3,1-propanediyl)]bis[N-L-alanine] bis(phenylmethyl) ester is prepared from L-alanine (phenylmethyl)ester.

Example 254

[R-(R*,R*)]-$N^{\omega}$,$N^{\omega'}$-Oxiranylidenbis(2-butyl-1-oxo-3,1-propanediyl)]bis[N-L-glutamine] bis(phenylmethyl) ester is prepared from L-glutamine (phenylmethyl)ester.

Example 255

[R-(R*,R*)]-$N^{\omega}$,$N^{\omega'}$-Oxiranylidenbis(2-butyl-1-oxo-3,1-propanediyl)]bis[N-L-asparagine] bis(phenylmethyl) ester is prepared from L-asparagine (phenylmethyl)ester.

Example 256

[R-(R*,R*)]-$N^{\omega}$,$N^{\omega'}$-Oxiranylidenbis(2-butyl-1-oxo-3,1-propanediyl)]bis[N-L-threonine] bis(phenylmethyl) ester is prepared from L-threonine (phenylmethyl)ester.

Example 257

[R-(R*,R*)]-$N^{\omega}$,$N^{\omega'}$-Oxiranylidenbis(2-(phenylmethyl)-1-oxo-3,1-propanediyl)]bis[N-L-valine] bis(phenylmethyl) ester is prepared from L-valine (phenylmethyl)ester.

Example 258

[R-(R*,R*)]-$N^{\omega}$,$N^{\omega'}$-Oxiranylidenbis(2-(phenylmethyl)-1-oxo-3 1-propanediyl)]bis[N-L-leucine] bis-(phenylmethyl) ester is prepared from L-leucine (phenylmethyl)ester.

Example 259

[R-(R*,R*)]-$N^{\omega}$,$N^{\omega'}$-Oxiranylidenbis(2-(phenylmethyl)-1-oxo-3 1-propanediyl)]bis[N-L-isoleucine] bis-(phenylmethyl) ester is prepared from L-isoleucine (phenylmethyl)ester.

Example 260

[R-(R*,R*)]-$N^{\omega}$,$N^{\omega'}$-Oxiranylidenbis(2-(phenylmethyl)-1-oxo-3 1-propanediyl)]bis-[N-L-alanine] bis-(phenylmethyl) ester is prepared from L-alanine (phenylmethyl)ester.

Example 261

[R-(R*,R*)]-$N^{\omega}$,$N^{\omega'}$-Oxiranylidenbis(2-(phenylmethyl)-1-oxo-3 1-propanediyl)]bis[N-L-glutamine] bis-(phenylmethyl) ester is prepared from L-glutamine (phenylmethyl)ester.

Example 262

[R-(R*,R*)]-$N^{\omega}$,$N^{\omega'}$-Oxiranylidenbis(2-(phenylmethyl)-1-oxo-3 1-propanediyl)]bis[N-L-asparagine] bis-(phenylmethyl) ester is prepared from L-asparagine (phenylmethyl)ester.

Example 263

[R-(R*,R*)]-$N^{\omega}$,$N^{\omega'}$-Oxiranylidenbis(2-(phenylmethyl)-1-oxo-3 1-propanediyl)]bis[N-L-threonine] bis-(phenylmethyl) ester is prepared from L-threonine (phenylmethyl)ester.

Examples 264-271

Substantially following the methods described in detail hereinabove in Examples 34-42, the compounds of this invention listed below in examples 264-271 are prepared using the amine indicated.

Example 264

[R-(R*,R*)-N,N'-Bis(4-methylcyclohexyl )-4-hydroxy-2,6-bis(phenylmethyl)heptanediamine is prepared from 4-methylcyclohexyl amine.

Example 265

[R-(R*,R*)-N,N'-Bis(cyclopentylmethyl )-4-hydroxy-2,6-bis(phenylmethyl)heptanediamine is prepared from cyclopentylmethyl amine.

Example 266

[R-(R*,R*)-N,N'-Bis(cyclohexyl )-4-hydroxy-2,6-bis(phenylmethyl)heptanediamine is prepared from

cyclohexyl amine.

Example 267

[R-(R*,R*)-N,N'-Bis(cycloheptyl )-4-hydroxy-2,6-bis(phenylmethyl)heptanediamine is prepared from cycloheptyl amine.

Example 268

[R-(R*,R*)-N,N'-Bis(3,3-dimethylbutyl)-4-hydroxy-2,6-bis(phenylmethyl)heptanediamine is prepared from 3,3-dimethylbutyl amine.

Example 269

[R-(R*,R*)-N,N'-Bis(2,2-dimethylpropyl)-4-hydroxy-2,6-bis(phenylmethyl)heptanediamine is prepared from 2,2-dimethylpropyl amine.

Example 270

[R-(R*,R*)-N,N'-Bis(2-ethylbutyl)-4-hydroxy-2,6-bis(phenylmethyl)heptanediamine is prepared from 2-ethylbutyl amine.

Example 271

[R-(R*,R*)-N,N'-Bis(1-methylethyl)-4-hydroxy-2,6-bis(phenylmethyl)heptanediamine is prepared from 1-methylethyl amine.

Examples 272-282

Substantially following the methods described in detail hereinabove in Examples 34-42, the compounds of this invention listed below in examples 272-282 are prepared using the amine indicated. The product from example 2 is used in place of the product from example 27 in example 34.

Example 272

[R-(R*,R*)-N,N'-Bis(cyclohexylmethyl)-4-hydroxy-2,6-dibutylheptanediamine is prepared from cyclohexylmethyl amine.

Example 273

[R-(R*,R*)-N,N'-Bis(cyclopentyl)-4-hydroxy-2,6-dibutylheptanediamine is prepared from cyclopentyl amine.

Example 274

[R-(R*,R*)-N,N'-Bis(1,1-dimethylethyl)-4-hydroxy-2,6-dibutylheptanediamine is prepared from 1,1-dimethylethyl amine.

Example 275

[R-(R*,R*)-N,N'-Bis(4-methylcyclohexyl)-4-hydroxy-2,6-dibutylheptanediamine is prepared from 4-methylcyclohexyl amine.

Example 276

[R-(R*,R*)-N,N'-Bis(cyclopentylmethyl )-4-hydroxy-2,6-dibutylheptanediamine is prepared from cyclopentylmethyl amine.

Example 277

[R-(R*,R*)-N,N'-Bis(cyclohexyl )-4-hydroxy-2,6-dibutylheptanediamine is prepared from cyclohexyl amine.

Example 278

[R-(R*,R*)-N,N'-Bis(cycloheptyl )-4-hydroxy-2,6-dibutylheptanediamine is prepared from cycloheptyl amine.

Example 279

[R-(R*,R*)-N,N'-Bis(3,3-dimethylbutyl)-4-hydroxy-2,6-dibutylheptanediamine is prepared from 3,3-dimethylbutyl amine.

Example 280

[R-(R*,R*)-N,N'-Bis(2,2-dimethylpropyl)-4-hydroxy-2,6-dibutylheptanediamine is prepared from 2,2-dimethylpropyl amine.

Example 281

[R-(R*,R*)-N,N'-Bis(2-ethylbutyl)-4-hydroxy-2,6-dibutylheptanediamine is prepared from 2-ethylbutyl amine.

Example 282

[R-(R*,R*)-N,N'-Bis(1-methylethyl)-4-hydroxy-2,6-dibutylheptanediamine is prepared from 1-methylethyl amine.

Examples 283-292

Substantially following the methods described in detail hereinabove in Examples 51-53, the compounds of this invention listed below in examples 283-292 are prepared using the amine indicated.

Example 283

2,2-[(2-Hydroxy-1,3-propanediyl)bis[ (phenylmethyl)-imino]]bis[N-(cyclohexylmethyl ) acetamide is prepared from cyclohexylmethyl amine.

Example 284

2,2-[(2-Hydroxy-1,3-propanediyl)bis[ (phenylmethyl)-imino]]bis[N-(cyclopentyl ) acetamide is prepared from cyclopentyl amine.

Example 285

2,2-[(2-Hydroxy-1,3-propanediyl)bis[ (phenylmethyl)-imino]]bis[N-(4-methylcyclohexyl ) acetamide is prepared from 4-methylcyclohexyl amine.

Example 286

2,2-[(2-Hydroxy-1,3-propanediyl)bis[ (phenylmethyl)-imino]]bis[N-(4-cyclopentylmethyl) acetamide is prepared from cyclopentylmethyl amine.

Example 287

2,2-[(2-Hydroxy-1,3-propanediyl)bis[ (phenylmethyl)-imino]]bis[N-(cyclohexyl ) acetamide is prepared from cyclohexyl amine.

Example 288

2,2-[(2-Hydroxy-1,3-propanediyl)bis[ (phenylmethyl)-imino]]bis[N-(cycloheptyl) acetamide is prepared from

cycloheptyl amine.

Example 289

2,2-[(2-Hydroxy-1,3-propanediyl)bis[ (phenylmethyl)-imino]]bis[N-(3,3-dimethylbutyl) acetamide is prepared from 3,3-dimethylbutyl amine.

Example 290

2,2-[(2-Hydroxy-1,3-propanediyl)bis[ (phenylmethyl)-imino]]bis[N-(2,2-dimethylpropyl) acetamide is prepared from 2,2-dimethylpropyl amine.

Example 291

2,2-[(2-Hydroxy-1,3-propanediyl)bis[ (phenylmethyl)-imino]]bis[N-(2 ethylbutyl) acetamide is prepared from 2-ethylbutyl amine.

Example 292

2,2-[(2-Hydroxy-1,3-propanediyl)bis[ (phenylmethyl)-imino]]bis[N-(1-methylethyl) acetamide is prepared from 1-methylethyl amine.

Examples 293-303

Substantially following the methods described in detail hereinabove in Examples 51-53 and 60 the compounds of this invention listed below in examples 293-303 are prepared using the amine indicated.

Example 293

2,2-[(2-Hydroxy-1,3-propanediyl)bis[ (cyclohexylmethyl)-imino]]bis[N-(cyclohexylmethyl ) acetamide is prepared from cyclohexylmethyl amine.

Example 294

2,2-[(2-Hydroxy-1,3-propanediyl)bis[ (cyclohexylmethyl)-imino]]bis[N-(cyclopentyl ) acetamide is prepared from cyclopentyl amine.

Example 295

2,2-[(2-Hydroxy-1,3-propanediyl)bis[ (cyclohexylmethyl)-imino]]bis[N-(4-methylcyclohexyl ) acetamide is prepared from 4-methylcyclohexyl amine.

Example 296

2,2-[(2-Hydroxy-1,3-propanediyl)bis[ (cyclohexylmethyl)-imino]]bis[N-(4-cyclopentylmethyl) acetamide is prepared from cyclopentylmethyl amine.

Example 297

2,2-[(2-Hydroxy-1,3-propanediyl)bis[ (cyclohexylmethyl)imino]]bis[N-(cyclohexyl ) acetamide is prepared from cyclohexyl amine.

Example 298

2,2-[(2-Hydroxy-1,3-propanediyl)bis[ (cyclohexylmethyl)imino]]bis[N-(cycloheptyl) acetamide is prepared from cycloheptyl amine.

Example 299

2,2-[(2-Hydroxy-1,3-propanediyl)bis[ (cyclohexylmethyl)imino]]bis[N-(3,3-dimethylbutyl) acetamide is prepared from 3,3-dimethylbutyl amine.

Example 300

2,2-[(2-Hydroxy-1,3-propanediyl)bis[ (cyclohexylmethyl)-imino]]bis[N-(2,2-dimethylpropyl) acetamide is prepared from 2,2-dimethylpropyl amine.

Example 301

2,2-[(2-Hydroxy-1,3-propanediyl)bis[ (cyclohexylmethyl)-imino]]bis[N-(2-ethylbutyl) acetamide is prepared from 2-ethylbutyl amine.

Example 302

2,2-[(2-Hydroxy-1,3-propanediyl)bis[ (cyclohexylmethyl)-imino]]bis[N-(1-methylethyl) acetamide is prepared from 1-methylethyl amine.

Example 303

2,2-[(2-Hydroxy-1,3-propanediyl)bis[ (cyclohexylmethyl)-imino]]bis[N-(1,1-dimethylethyl) acetamide is prepared from 1,1-dimethylethyl amine.

Examples 304 - 312

Substantially following the methods described in detail hereinabove in Examples 43,47-49, the compounds of this invention listed below in examples 304-312 are prepared using the carboxylic acid indicated.

Example 304

[1S-[1α[2R*,6R*,7(1R*,2R*)],2β]]-4-Hydroxy-2,6-bis(phenylmethyl)-N,N'-bis[2-[(furoylcarbonyl) amino] cyclohexyl]-heptanediamde is prepared from 2-furoic acid.

Example 305

[1S-[1α[2R*,6R*,7(1R*,2R*)],2β]]-4-Hydroxy-2,6-bis(phenylmethyl)-N,N'-bis[3-[(furoylcarbonyl) amino] cyclohexyl]-heptanediamde is prepared from 3-furoic acid.

Example 306

[1S-[1α[2R*,6R*,7(1R*,2R*)],2β]]-4-Hydroxy-2,6-bis(phenylmethyl)-N,N'-bis[3-[(pyridinylcarbonyl) amino] cyclohexyl]-heptanediamde is prepared from nicotinic acid.

Example 307

[1S-[1α[2R*,6R*,7(1R*,2R*)],2β]]-4-Hydroxy-2,6-bis(phenylmethyl)-N,N'-bis[4-[(pyridinylcarbonyl) amino] cyclohexyl]-heptanediamde is prepared from isonicotinic acid.

Example 308

[1S-[1α[2R*,6R*,7(1R*,2R*)],2β]]-4-Hydroxy-2,6-bis(phenylmethyl)-N,N'-bis[[((phenylmethyl)carbonyl) amino] cyclohexyl]-heptanediamde is prepared from (phenylmethyl) carboxylic acid.

Example 309

[1S-[1α[2R*,6R*,7(1R*,2R*)],2β]]-4-Hydroxy-2,6-bis(phenylmethyl)-N,N'-bis[[(phenylcarbonyl) amino] cyclohexyl]-heptanediamde is prepared from benzoic acid.

Example 310

[1S-[1α[2R*,6R*,7(1R*,2R*)],2β]]-4-Hydroxy-2,6-bis(phenylmethyl)-N,N'-bis[[(4-methoxyphenylcarbonyl) amino] cyclohexyl]-heptanediamde is prepared from 4-methoxybenzoic acid.

Example 311

[1S-[1α[2R*,6R*,7(1R*,2R*)],2β]]-4-Hydroxy-2,6-bis(phenylmethyl)-N,N'-bis[[(3,4,5-trimethoxyphenylcarbonyl) amino] cyclohexyl]-heptanediamde is prepared from 3,4,5-trimethoxybenzoic acid.

Example 312

[1S-[1α[2R*,6R*,7(1R*,2R*)],2β]]-4-Hydroxy-2,6-bis(phenylmethyl)-N,N'-bis[[(2-benzofuranylcarbonyl) amino] cyclohexyl]-heptanediamde is prepared from 2-benzofuran carboxylic acid.

Examples 313 - 322

Substantially following the methods described in detail hereinabove in Examples 43,47-49, the compounds of this invention listed below in examples 313-322 are prepared using the carboxylic acid indicated. The product from example 2 is used in place of the product from example 27 in example 43.

Example 313

[1S-[1α[2R*,6R*,7(1R*,2R*)],2β]]-4-Hydroxy-2,6-dibutyl-N,N'-bis[2-[(furoylcarbonyl) amino] cyclohexyl]-heptanediamde is prepared from 2-furoic acid.

Example 314

[1S-[1α[2R*,6R*,7(1R*,2R*)],2β]]-4-Hydroxy-2,6-dibutyl-N,N'-bis[3-[(furoylcarbonyl) amino] cyclohexyl]-heptanediamde is prepared from 3-furoic acid.

Example 315

[1S-[1α[2R*,6R*,7(1R*,2R*)],2β]]-4-Hydroxy-2,6-dibutyl-N,N'-bis[3-[(pyridinylcarbonyl) amino] cyclohexyl]-heptanediamde is prepared from nicotinic acid.

Example 316

[1S-[1α[2R*,6R*,7(1R*,2R*)],2β]]-4-Hydroxy-2,6-dibutyl-N,N'-bis[4-[(pyridinylcarbonyl) amino] cyclohexyl]-heptanediamde is prepared from isonicotinic acid.

Example 317

[1S-[1α[2R*,6R*,7(1R*,2R*)],2β]]-4-Hydroxy-2,6-dibutyl-N,N'-bis[[((phenylmethyl)carbonyl) amino] cyclohexyl]-heptanediamde is prepared from (phenylmethyl) carboxylic acid.

Example 318

[1S-[1α[2R*,6R*,7(1R*,2R*)],2β]]-4-Hydroxy-2,6-bis(dibutyl)-N,N'-bis[[(phenylcarbonyl) amino] cyclohexyl]-heptanediamde is prepared from benzoic acid.

Example 319

[1S-[1α[2R*,6R*,7(1R*,2R*)],2β]]-4-Hydroxy-2,6-dibutyl-N,N'-bis[[(4-methoxyphenylcarbonyl) amino] cyclohexyl]-heptanediamde is prepared from 4-methoxybenzoic acid.

Example 320

[1S-[1α[2R*,6R*,7(1R*,2R*)],2β]]-4-Hydroxy-2,6-dibutyl-N,N'-bis[[(3,4,5-trimethoxyphenylcarbonyl) amino] cyclohexyl]-heptanediamde is prepared from 3,4,5-trimethoxybenzoic acid.

Example 321

[1S-[1α[2R*,6R*,7(1R*,2R*)],2β]]-4-Hydroxy-2,6-dibutyl-N,N'-bis[[(2-benzofuranylcarbonyl) amino] cyclohexyl]-heptanediamde is prepared from 2-benzofuran carboxylic acid.

Example 322

[1S-[1α[2R*,6R*,7(1R*,2R*)],2β]]-4-Hydroxy-2,6-dibutyl-N,N'-bis[[(2-quinolylcarbonyl) amino] cyclohexyl]-heptanediamde is prepared from quinaldic acid.

Example 323

[1S-[1α[2R*,6R*,7(1R*,2R*)],2β]]-4-Hydroxy-2,6-bis(phenylmethyl)-N,N'-bis[[(4-pyridyl) amino] cyclohexyl]-heptanediamde is prepared from the product from Example 43 and 4-pyridine carboxylic acid substantially following the methods described hereinabove in Examples 43-47.
MS (FAB ): 759
$^1$H NMR (CDCl3/CD3OD): 8.65 (4H, d), 8.07 (2H, m), 7.64 (2H, d), 7.63 (2H, d), 7.36 (2H, m), 6.92 - 7.06 (10H, m), 3.69 (3H, m), 3.39 (2H, m), 2.46 - 3.73 (6H, m), 2.19 (2H, m), 1.69 - 1.95 (8H, m), 1.25 - 1.40 (10H, m).

Example 324

[1S-[1α[2R*,6R*,7(1R*,2R*)],2β]]-4-Hydroxy-2,6-bis(phenylmethyl)-N,N'-bis[[(3-pyridyl) amino] cyclohexyl]-heptanediamde is prepared from the product from Example 43 and 3-pyridine carboxylic acid substantially following the methods described hereinabove in Examples 43-47.
MS (FAB): 759
$^1$H NMR (CDCl3/CD3OD): 8.95 (2H, m), 8.66 (2H, m), 8.03 (4H,m), 7.18 - 7.40 (4H, m), 6.92 - 7.05 (10H, m), 3.69 (3H, m), 3.38 (2H, m), 2.47 - 2.74 (6H, m), 2.18 (2H, m), 1.68 - 1.95 (8H, m), 1.24 - 1.42 (10H, m).

Example 325

[1S-[1α[2R*,6R*,7(1R*,2R*)],2β]]-4-Hydroxy-2,6-bis(phenylmethyl)-N,N'-bis[[(2-pyridyl) amino] cyclohexyl]-heptanediamde is prepared from the product from Example 43 and 2-pyridine carboxylic acid substantially following the methods described hereinabove in Examples 43-47.
MS (FAB) : 759
$^1$H NMR (CDCl3): 8.54 (2H, m), 8.12 (4H, m), 7.78 (2H, m), 7.40 (2H, m), 6.82 - 7.11 (10H, m), 6.64 (1H, d), 6.56 (1H,. d), 3.41 - 3.82 (5H, m), 3.00 (2H, m), 2.66 (2H, m), 2.40 (2H, m), 2.04 (4H, m), 1.76 (4H, m), 1.17 - 1.57 (12H, m).

Example 326

[1S-[1α[2R*,6R*,7(1R*,2R*)],2β]]-4-Hydroxy-2,6-bis(phenylmethyl)-N,N'-bis[[(2-acetyl) amino] cyclohexyl]-heptanediamde is prepared from the product from Example 43 and acetic acid substantially following the methods described hereinabove in Examples 43-47.
MS (FAB) : 633
$^1$H NMR (CDCl3): 7.10 - 7.35 (10H, m), 6.70 - 7.00 (4H, m), 3.39 - 3.61 (5H, m), 2.38 - 2.92 (6H, m), 1.58 - 2.06 (8H, m), 1.84 (3H, s), 1.85 (3H, s), 1.10 - 1.39 (12H, m).

Example 327

[1S-[1α[2R*,6R*,7(1R*,2R*)],2β]]-4-Hydroxy-2,6-bis(phenylmethyl)-N,N'-bis[[((2-pyridyl)acetyl) amino] cyclohexyl]-heptanediamde is prepared from the product from Example 43 and 2-pyridylacetic acid substantially following the methods described hereinabove in Examples 43-47.
MS (FAB) : 759

1H NMR (CDCl3): 8.54 (2H, m), 8.12 (4H, m), 7.78 (2H, m), 7.40 (2H, m), 6.82 - 7.11 (10H, m), 6.64 (1H, d), 6.56 (1H,. d), 3.41 - 3.82 (5H, m), 3.00 (2H, m), 2.66 (2H, m), 2.40 (2H, m), 2.04 (4H, m), 1.76 (4H, m), 1.17 - 1.57 (12H, m).

Example 328

[1S-[ 1R*[2S*,6S*,7R*],2R*]]-4-Hydroxy-2,6-bis(phenylmethyl)-N,N'-bis[1-[[(1-H-benzimidazoyl-2ylmethyl)-amino]carbonyl]-2-methylpropyl]-heptanediamide is prepared substantially following the methods described hereinabove in Examples 29,30 and 31. In example 29, N-(2-benzimidazoylmethyl)-valinyl amide (preaped according to EP 0 337 714 A2) is used in place of the product from example 5.
MS (FAB) : 813
$^1$H NMR ($CDCl_3/CD_3OD$): 7.55 (4H, m), 7.26 (4H, m), 7.09 (10H, m), 4.46 (4H, m), 4.11 (2H, m), 3.60 (1H, m), 2.62 - 3.04 (10H, m), 2.15 (2H, m), 0.87 (12H, m).

Example 329

[1S-[ 1R*[2S*,6S*,7(1R*,2R*)],2R*]]-4-Hydroxy-2,6-bis(phenylmethyl)-N,N'-bis[1-[[(1-H-benzimidazoyl-2ylmethyl)amino]carbonyl]-2-methylbutyl]heptanediamide is prepared substantially following the methods described hereinabove in Examples 29,30 and 31. In example 29, N-(2-benzimidazoylmethyl)-isoleucinyl amide (preaped according to EP 0 337 714 A2) is used in place of the product from example 5.
MS (FAB) : 841
$^1$H NMR ($CDCl_3/CD_3OD$): 7.55 (4H, m), 7.25 (6H, m), 7.09 (8H, m), 4.46 (4H, m), 4.15 (2H, m), 3.60 (1H, m), 2.60 - 3.00 (10H, m), 1.89 (2H, m), 0.95 - 1.70 (4H, m), 0.83 (12H, m).

Example 330

[R-(R*,R*)]-N,N'dicyclohexyl-4-hydroxy-2,6-bis(phenylmethyl) heptanediamide

Substantially following the methods described in detail hereinabove in Examples 34-42, the named compound of this example is prepared using cyclohexylamine.
MS (FAB) : 519
$^1$H NMR (CDCl3): 7.12 - 7.30 (10H m), 5.31 (2H, t), 3.57 (3H, m), 2.35 - 2.89 (6H, m), 1.18 - 1.88 (24 H, m).

Example 331

[R,(R*,R*)-N,N'-4-hydroxy-N,N'-bis(2-methylpropyl)-2,6-bis(phenylmethyl) heptanediamide.

Substantially following the methods described in detail hereinabove in Examples 34-42, the named compound of this example is prepared using 2-methylpropylamine.
MS (FAB) : 467
$^1$H NMR (CDCl3): 7.10 - 7.30 (10H. m), 7.02 (1H, t), 6.85 (1H, t), 3.33 (1H, m), 2.43 - 3.03 (10H, m), 2.11 (2H, m), 1.50 (4H, m), 0.68 (12H, m).

**Claims**

**1.** Compounds of the formula:

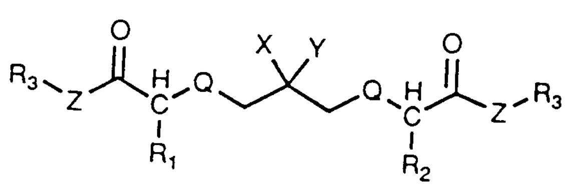

wherein:
Q is the same or different and is selected from a single bond or $NR_9$ or $NR_{10}$;
wherein:
$R_9$ = H, $C_1$ - $C_7$ straight or branched alkyl; $-(CH_2)_n$-cyclic $C_3$ - $C_7$ alkyl, n = 0 - 4; $(CH_2)_n$-phenyl, n = 0-4; or $-(CH_2)_n$-substituted phenyl, where n = 0 - 4 and substituted with F,Cl,Br,I, $C_1$ - $C_4$ alkoxide, $C_1$ - $C_6$

straight, branched or cyclic alkyl; or $R_9$ and $R_2$ taken together are $-(CH_2)_m-$, m = 3 or 4; or $R_9$ and $R_1$ taken together are $-(CH_2)_m-$, m = 3 or 4; or $R_9$ and $R_2$ taken together are

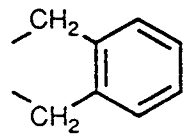

or $R_9$ and $R_1$ taken together are

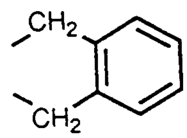

$R_{10}$ = H, $C_1$ - $C_7$ straight or branched alkyl; $-(CH_2)_n$-cyclic $C_3$ - $C_7$ alkyl, n = 0 - 4; $-(CH_2)_n$-phenyl, n = 0-4; or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; or $R_{10}$ and $R_2$ taken together are $-(CH_2)_m-$, m = 3 or 4; or $R_{10}$ and $R_1$ taken together are $-(CH_2)_m-$, m = 3 or 4; or $R_{10}$ and $R_2$ taken together are

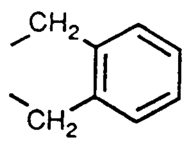

or $R_{10}$ and $R_1$ taken together are

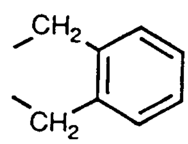

wherein:
Z = O or NH; X = OH and Y = H or $CH_2OH$ or X and Y taken together may be an epoxide $-(CH_2O)-$;
$R_1$ = H, $C_1$ - $C_7$ straight or branched alkyl; $-(CH_2)_n$-cyclic $C_3$ - $C_7$ alkyl, n = 0 - 4; $-(CH_2)_n$-phenyl, n = 0-4; or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl, or phenyl;
$R_2$ = H, $C_1$ - $C_7$ straight, branched or cyclic alkyl; $-(CH_2)_n$-cyclic $C_3$ - $C_7$ alkyl, n = 0 - 4; $-(CH_2)_n$-phenyl, n = 0-4; or $-(CH_2)_n$-substituted phenyl, where n = 0 - 4 and substituted with F,Cl,Br,I, $C_1$ - $C_4$ alkoxide, $C_1$ -$C_6$ straight, branched or cyclic alkyl, or phenyl;
$R_3$ =

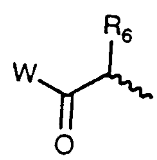

or $C_1$ - $C_{10}$ straight or branched alkyl; $-(CH_2)_n$-cyclic $C_3$ - $C_7$ alkyl, n = 0 - 4; or $-(CH_2)_n$ -substituted cyclic $C_3$ - $C_7$ alkyl, where n = 0 - 4 and substituted with $C_1$ - $C_7$ straight, branched or cyclic alkyl, or $NHCOR_4$;
wherein:
$R_4$ = $UR_5$; U = a single bond, oxygen or NH; $R_5$ = $C_1$ -$C_8$ straight or branched alkyl; $-(CH_2)_n$ cyclic $C_3$ - $C_7$ alkyl, n = 0 - 4; $-(CH_2)_n$-phenyl, n = 0 - 4; $-(CH_2)_n$ substituted phenyl, where n = 0 - 4 and

substituted with F,Cl,Br,I, $C_1$ - $C_4$ alkoxide, $C_1$ - $C_6$ straight, branched or cyclic alkyl; $-(CH_2)_n$ 1-napthyl, n = 0 - 4; $-(CH_2)_n$ 1-substituted napthyl, where n = 0 - 4 and substituted with F,Cl,Br,I, $C_1$ - $C_4$ alkoxide, $C_1$ -$C_6$ straight, branched or cyclic alkyl; $-(CH_2)_n$ 2-napthyl, n = 0 - 4; $-(CH_2)_n$ 2-substituted napthyl, where n = 0 - 4 and substituted with F,Cl,Br,I, $C_1$ - $C_4$ alkoxide, $C_1$ - $C_6$ straight, branched or cyclic alkyl; $-(CH_2)_n$ 2-quinoyl, n = 0 - 4; $-(CH_2)_n$ 2-substituted quinoyl, where n = 0 - 4 and substituted with F,Cl,Br,I, $C_1$ - $C_4$ alkoxide, $C_1$ - $C_6$ straight, branched or cyclic alkyl; $-(CH_2)_n$ heterocycle, where n = 0-4 and heterocycle is defined as a stable 5- to 7-membered mono- or bicyclic or a stable 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and may include any bicyclic group in which any of the above defined heterocycles is fused to a benzene ring; $-(CH_2)_n$ substituted heterocycle, where n = 0-4, substituted with F,Cl,Br,I, $C_1$ - $C_6$ alkoxide, $C_1$ - $C_6$ straight, branched or cyclic alkyl;
$R_6$ = $C_1$ - $C_7$ straight, branched or cyclic alkyl; $(CH_2)_2CONH_2$,$CH_2CONH_2$, $CH_2OH$, or $CH(CH_3)OH$;
W =

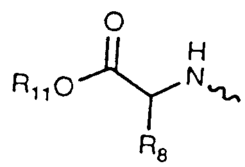

or $OR_7$ or $NHR_7$, wherein: $R_7$ = $C_1$ - $C_8$ straight or branched alkyl; $-(CH_2)_n$ cyclic $C_3$ - $C_7$ alkyl, n = 0 - 4; $-(CH_2)_n$-phenyl, n = 0 - 4; $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; $-(CH_2)_n$ heterocycle, where n = 0-4 and heterocycle is as defined before; $-(CH_2)_n$ substituted heterocycle, where n = 0-4, heterocycle is as defined before and substituted with F,Cl,Br,I, $C_1$ - $C_4$ alkoxide, $C_1$ - $C_6$ straight, branched or cyclic alkyl;
$R_8$ = $C_1$ - $C_7$ straight or branched alkyl; $-(CH_2)_n$ cyclic $C_3$ - $C_7$ alkyl, n = 0 - 4; $-(CH_2)_n$ -phenyl, n = 0 - 4; $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; or $-(CH_2)_n-N=C(NH_2)_2$, n = 0 - 4;
$R_{11}$ = $C_1$ - $C_8$ straight, branched or cyclic alkyl; $-(CH_2)_n$-phenyl, n = 0 - 4, or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl.

**2.** Compounds according to Claim 1 of the formula:

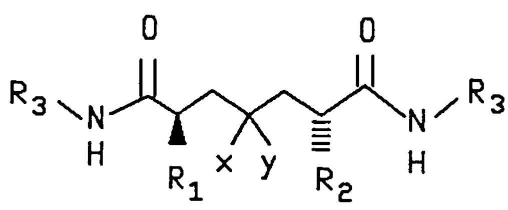

wherein: X = OH and Y = H or $CH_2OH$ or X and Y taken together are an epoxide $-CH_2O-$;
$R_1 = R_2 = -nC_4H_9$, $-CH_2$ phenyl, or $-CH_2$ cyclohexyl;
and
$R_3$ = $-CH(CH_3)_2$, $-CH(CH_3)CH_2CH_3$, $-CH_2CH(CH_3)_2$,$-CH(CH_2CH_3)_2$, $-C(CH_3)_3$, $-CH_2C(CH_3)_3$, $-CH_2CH_2C(CH_3)_3$, cyclopentyl, cyclohexyl, cycloheptyl, $-CH_2$(cyclopentyl), $-CH_2$(cyclohexyl), $-CH_2$(cycloheptyl), 2-methylcyclohexyl, 3-methylcyclohexyl, or 4-methylcyclohexyl.

**3.** Compounds according to Claim 1 of the formula:

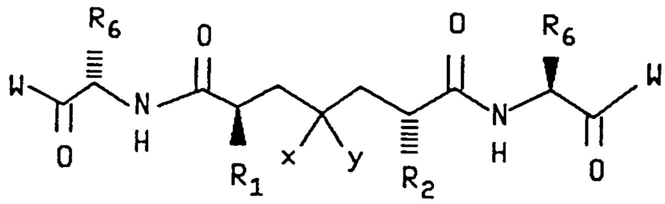

wherein:
X = OH and Y = H or $CH_2OH$, or X and Y taken together are an epoxide
$R_1 = R_2 =$ $nC_4H_9$, $-CH_2$ phenyl, or $-CH_2$ cyclohexyl
and
$R_6 =$ $-CH_3$, $-CH(CH_3)_2$, $-CH(CH_3)CH_2CH_3$, $-CH_2CH(CH_3)_2$, $-CH_2CONH_2$, $-CH_2CH_2CONH_2$ or -CH(OH)-$CH_3$
and
W = $OR_7$ or $NHR_7$ wherein:
$R_7 =$ $-CH_3$, $-CH_2CH_3$, $-CH(CH_3)_2$, $-C(CH_3)_3$, $-CH_2CH(CH_3)_2$, $-CH_2C(CH_3)_3$, $-CH_2$ phenyl, $-(CH_2)_n$ heterocycle, where n = 0-4 and heterocycle is defined as a stable 5- to 7-membered mono- or bicyclic or a stable 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and may include any bicyclic group in which any of the above defined heterocycles is fused to a benzene ring; $-(CH_2)_n$ substituted heterocycle, where n = 0-4, and substituted with F,Cl,Br,I, $C_1$ - $C_6$ alkoxide, $C_1$ - $C_6$ straight, branched or cyclic alkyl.

**4.** Compounds according to Claim 1 of the formula:

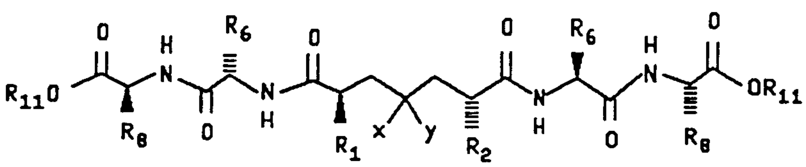

wherein:
X = OH and Y = H or $CH_2OH$, or X and Y taken together are an epoxide
$R_1 = R_2 =$ n-butyl, $-CH_2$ phenyl, or $-CH_2$ cyclohexyl
and
$R_6 =$ $-CH_3$, $-CH(CH_3)_2$, $-CH(CH_3)CH_2CH_3$, $-CH_2CH(CH_3)_2$, $-CH_2CONH_2$, $-CH_2CH_2CONH_2$, or -CH(OH)-$CH_3$ and $R_8 =$ $-CH_2$ phenyl, $-CH_2$(4-hydroxyphenyl), $-CH_3$, $-CH(CH_3)_2$, $-CH(CH_3)CH_2CH_3$, $-CH_2CH$-$(CH_3)_2$, or $-(CH_2)_3N=C(NH_2)_2$,
and $R_{11} =$ $-CH_3$, $-CH_2CH_3$, $-CH(CH_3)_2$, $-C(CH_3)_3$, $-CH_2CH(CH_3)_2$, $-CH_2C(CH_3)_3$, $-CH_2$ phenyl.

**5.** Compounds according to Claim 1 of the formula:

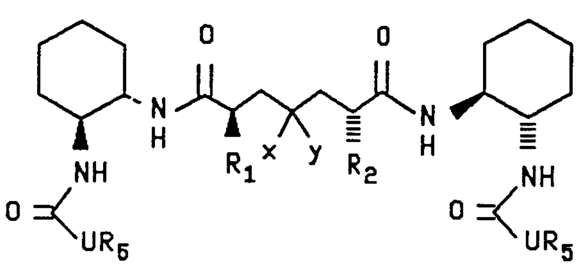

wherein:
X = OH and Y = H or $CH_2OH$ or X and Y taken together are an epoxide;
$R_1 = R_2 = nC_4H_9$, $-CH_2$ phenyl or $-CH_2$ cyclohexyl;

U = O, NH or a single bond; and

$R_5$ = $C_1$ - $C_8$ straight or branched alkyl; $-(CH_2)_n$ cyclic $C_3$ - $C_7$ alkyl, n = 0 - 4; $-(CH_2)_n$-phenyl, n = 0 - 4; $-(CH_2)_n$ substituted phenyl, where n = 0 - 4 and substituted with F,Cl,Br,I, $C_1$ - $C_4$ alkoxide, $C_1$ - $C_6$ straight, branched or cyclic alkyl; $-(CH_2)_n$ 1-napthyl, n = 0 - 4; $-(CH_2)_n$ 1-substituted napthyl, where n = 0 - 4 and substituted with F,Cl,Br,I, $C_1$ - $C_4$ alkoxide, $C_1$ -$C_6$ straight, branched or cyclic alkyl; $-(CH_2)_n$ 2-napthyl, n = 0 - 4; $-(CH_2)_n$ 2-substituted napthyl, where n = 0 - 4 and substituted with F,Cl,Br,I, $C_1$ - $C_4$ alkoxide, $C_1$ - $C_6$ straight, branched or cyclic alkyl; $-(CH_2)_n$ 2-quinoyl, n = 0 - 4; $-(CH_2)_n$ 2-substituted quinoyl, where n = 0 - 4 and substituted with F,Cl,Br,I, $C_1$ - $C_4$ alkoxide, $C_1$ - $C_6$ straight, branched or cyclic alkyl; $-(CH_2)_n$ heterocycle, where n = 0-4 and heterocycle is defined as a stable 5- to 7-membered mono- or bicyclic or a stable 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and may include any bicyclic group in which any of the above defined heterocycles is fused to a benzene ring; $-(CH_2)_n$ substituted heterocycle, where n = 0-4, and substituted with F,Cl,Br,I, $C_1$ - $C_6$ alkoxide, $C_1$ - $C_6$ straight, branched or cyclic alkyl.

**6.** A compound according to Claim 1, [R-(R*,R*)]-N$^{\omega}$,N$^{\omega'}$-[oxiranylidenebis(2-butyl-1-oxo-3,1--propanediyl)]-bis[N-L-leucyl-L-phenylalanine]dimethyl ester; [R-(R*,R*)]-N$^{\omega}$,N$^{\omega'}$-[oxiranylidenebis(2-butyl-1-oxo-3,1-propanediyl)]bis[N-L-alanyl-L-alanine]dimethyl ester; [R-(R*,R*)]-N$^{\omega}$,N$^{\omega'}$-[oxiranylidenebis(2-butyl-1-oxo-3,1-propanediyl)bis[N-L-valyl-L-valine]dimethyl ester; R-(R*,R*)]-N$^{\omega}$,N$^{\omega'}$-(2,6-dibutyl-4-hydroxy-4-(hydroxymethyl,-1,7-dioxo-1,7-heptanediyl]bis[N-L-leucyl-L-phenylalanine] dimethyl ester; [R-(R*,R*)-]--N$^{\omega}$,N$^{\omega'}$-[2,6-dibutyl-4-hydroxy-4-(hydroxymethyl)-1,7--dioxo-1,7-heptanediyl]bis[N-L-alanyl-L-alanine]-dimethyl ester;
[R-(R*,R*)]-N$^{\omega}$,N$^{\omega'}$-[2,6-dibutyl-4-hydroxy-4-(hydroxymethyl)-1,7-dioxo-1,7-heptanediyl]bis[N-L-valyl-L-valine] dimethyl ester; [R-(R*,R*)]-N$^{\omega}$,N$^{\omega'}$-(2,6-dibutyl -4-hydroxy-1,7-heptanediyl)bis[N-L-leucyl-L-phenylalanine]dimethyl ester; [R-(R*,R*)]-N$^{\omega}$,N$^{\omega'}$-(2,6-dibutyl-4--hydroxy-1,7-heptanediyl)bis[N-L-alanyl-L-alanine] dimethyl ester; [R-(R*,R*)]-N$^{\omega}$,N$^{\omega'}$-(2,6-dibutyl-4-hydroxy-1,7-dioxo-1,7-heptanediyl)bis[N-L-valyl-L-valine]dimethyl ester; [2R-[2R*,4R*,5[1S*(1S*)]]and 2S-[2R*,4S*,5[1R*(1R*)]]]-N-[N-[1-[2-hydroxy-5-[[1-[-[[-1-(methoxycarbonyl)-2-methylpropyl]amino]carbonyl]-2-methylpropyl]amino]-5-oxo-4-(phenylmethyl)-pentyl]-L-prolyl]-L-valyl]-L-valine methyl ester; [R-(R*,R*)]-N,-N-[4-hydroxy-1,7-dioxo-2,6-bis-(phenylmethyl)-1,7-heptanediyl]bis[N-L-valyl-L-valine]dimethyl ester; [R-(R*,R*)]-N$^{\omega}$,N$^{\omega'}$-[4-hydroxy-1,7-dioxo-2,6-bis-(phenylmethyl)-1,7-heptanediyl]bis[N-L-leucyl-L-phenylalanine]dimethyl ester; [R-(R*,R*)]-N,N'-bis(1,1-dimethylethyl)-4-hydroxy-2,6-bis(phenylmethyl)heptanediamide; [R-(R*,R*)]-N,N'-dicyclopentyl-4-hydroxy-2,6-bis(phenylmethyl)heptanediamide; [R-(R*,R*)]-N,N'-bis-(cyclohexylmethyl)-4-hydroxy-2,6-bis(phenylmethyl)heptanediamide; [1S-[1α[2R*,6R*,7(1R*,2R*)],2β]-[[4-hydroxy-1,7-dioxo-2,6-bis(phenylmethyl)-1,7-heptanediyl]bis(imino-2,1-cyclohexanediyl)]bis carbamic acid bis(1,1-dimethylethyl)ester; [1S-[1α[2R*,6R*,7(1R*,-2R*)],2β]]-4-hydroxy-2,6-bis(phenylmethyl)-N,N'-bis[2-[(2-quinolinylcarbonyl)amino]cyclohexyl]-heptanediamide; 2,2'-[[2-hydroxy-2-(hydroxymethyl)-1,3-propanediyl]bis[(phenylmethyl)imino]]bis[N-(1,1-dimethylethyl)acetamide]; 2,2'-[(2-hydroxy-1,3-propanediyl)bis[(phenylmethyl)imino]]bis[N-(1,1-dimethylethyl)acetamide]; N-[3-[[2-[(1,1-dimethylethyl)-amino]-2-oxo-ethyl](phenylmethyl)amino]-2-hydroxy-2-(hydroxymethyl)propyl]-N-(phenylmethyl)glycine ethyl ester; N,N'-[2-hydroxy-2-(hydroxymethyl)-1,3-propanediyl]bis[N-(phenylmethyl)glycine] diethyl ester;1,1'-[2-hydroxy-2-(hydroxymethyl)-1,3-propanediyl]-bis[N-(1,1-dimethylethyl)-2-piperidinecarboxamide](Isomer A); 1,1'-[2-hydroxy-2-(hydroxymethyl)-1,3-propanediyl]bis[N(1,1-dimethylethyl)-2-piperidinecarboxamide] (Isomer B); or N,N'-[2-hydroxy-2-(hydroxymethyl)-1,3-propanediyl]bis[N-(cyclohexylmethyl)glycine] diethyl ester.

**7.** Compounds of the formula:

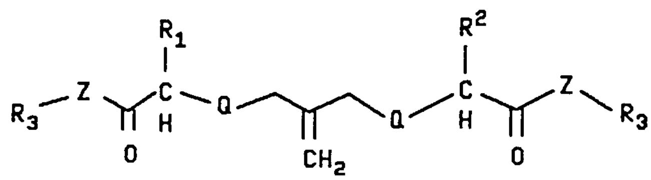

wherein:

Q is the same or different and is selected from a single bond or $NR_9$ or $NR_{10}$;
$R_9$ = H, $C_1$ - $C_7$ straight or branched alkyl; $-(CH_2)_n$ cyclic $C_3$ - $C_7$ alkyl, n = 0 - 4; $-(CH_2)_n$-phenyl, n=0-4; or $-(CH_2)_n$-substituted phenyl, where n=0 - 4 and substituted with F,Cl,Br,I, $C_1$ - $C_4$ alkoxide, $C_1$ - $C_6$ straight, branched or cyclic alkyl or phenyl; or $R_9$ and $R_2$ taken together are $-(CH_2)_m-$, m=3 or 4; or $R_9$ and $R_1$ taken together are $-(CH_2)_m-$, m=3 or 4; or $R_9$ and $R_2$ taken together are

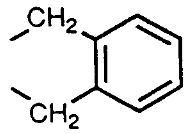

or $R_9$ and $R_1$ taken together are

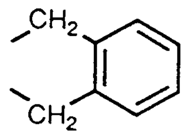

$R_{10}$ = H, $C_1$ - $C_7$ straight or branched alkyl; $-(CH_2)_n$ cyclic $C_3$ - $C_7$ alkyl, n = 0 - 4; $-(CH_2)_n$-phenyl, n=0-4; or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; or $R_{10}$ and $R_2$ taken together are $-(CH_2)_m-$, m=3 or 4; or $R_{10}$ and $R_1$ taken together are $-(CH_2)_m-$, m=3 or 4; or $R_{10}$ and $R_2$ taken together are

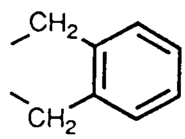

or $R_{10}$ and $R_1$ taken together are

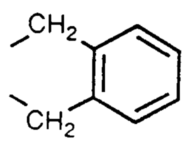

wherein:
$R_1$ = H, $C_1$ - $C_7$ straight or branched alkyl; $-(CH_2)_n$ cyclic $C_3$ - $C_7$ alkyl, n = 0 - 4; $-(CH_2)_n$-phenyl, n=0-4; or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl, or phenyl;
$R_2$ = H, $C_1$ - $C_7$ straight or branched alkyl; $-(CH_2)_n$ cyclic $C_3$ - $C_7$ alkyl, n = 0 - 4; $-(CH_2)_n$-phenyl, n=0-4; or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl, or phenyl;
$R_3Z$ =

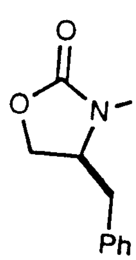

or Z = O or NH and
$R_3$ =

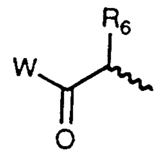

$C_1$ - $C_{10}$ straight or branched alkyl; $-(CH_2)_n$-cyclic $C_3$ - $C_7$ alkyl, n = 0 - 4; or $-(CH_2)_n$ -substituted cyclic $C_3$ - $C_7$ alkyl, where n = 0 - 4 and substituted with $C_1$ - $C_7$ straight, branched or cyclic alkyl, or $NHCOR_4$;
wherein:
$R_4$ = $UR_5$; U = a single bond, oxygen or NH;
$R_5$ = $C_1$ - $C_8$ straight or branched alkyl; $-(CH_2)_n$ cyclic $C_3$ - $C_7$ alkyl, n = 0 - 4; $-(CH_2)_n$-phenyl, n = 0 - 4; $-(CH_2)_n$ substituted phenyl, where n = 0 - 4 and substituted with F,Cl,Br,I, $C_1$ - $C_4$ alkoxide, $C_1$ - $C_6$ straight, branched or cyclic alkyl; $-(CH_2)_n$ 1-napthyl, n = 0 - 4; $-(CH_2)_n$ 1-substituted napthyl, where n = 0 - 4 and substituted with F,Cl,Br,I, $C_1$ - $C_4$ alkoxide, $C_1$ -$C_6$ straight, branched or cyclic alkyl; $-(CH_2)_n$ 2-napthyl, n = 0 - 4; $-(CH_2)_n$ 2-substituted napthyl, where n = 0 - 4 and substituted with F,Cl,Br,I, $C_1$ - $C_4$ alkoxide, $C_1$ - $C_6$ straight, branched or cyclic alkyl; $-(CH_2)_n$ 2-quinoyl, n = 0 - 4; $-(CH_2)_n$ 2-substituted quinoyl, where n = 0 - 4 and substituted with F,Cl,Br,I, $C_1$ - $C_4$ alkoxide, $C_1$ - $C_6$ straight, branched or cyclic alkyl; $-(CH_2)_n$ heterocycle, where n = 0-4 and heterocycle is defined as a stable 5- to 7-membered mono- or bicyclic or a stable 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and may include any bicyclic group in which any of the above defined heterocycles is fused to a benzene ring; $-(CH_2)_n$ substituted heterocycle, where n = 0-4, and substituted with F,Cl,Br,I, $C_1$ - $C_6$ alkoxide, $C_1$ - $C_6$ straight, branched or cyclic alkyl;
$R_6$ = $C_1$ - $C_7$ straight, branched or cyclic alkyl; $(CH_2)_2CONH_2$,$CH_2CONH_2$, $CH_2OH$, or $CH(CH_3)OH$;
W =

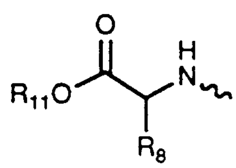

$OR_7$ or $NHR_7$, wherein: $R_7$ = $C_1$ - $C_8$ straight or branched alkyl; $-(CH_2)_n$ cyclic $C_3$ - $C_7$ alkyl, n = 0 - 4; $-(CH_2)_n$-phenyl, n = 0 - 4; $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; $-(CH_2)_n$ heterocycle, where n = 0-4 and heterocycle is as defined before; $-(CH_2)_n$ substituted heterocycle, where n = 0-4, heterocycle is as defined before and substituted with F,Cl,Br,I, $C_1$ - $C_4$ alkoxide, $C_1$ - $C_6$ straight, branched or cyclic alkyl;
$R_8$ = $C_1$ - $C_7$ straight or branched alkyl; $-(CH_2)_n$ cyclic $C_3$ - $C_7$ alkyl, n = 0 - 4; $-(CH_2)_n$ -phenyl, n = 0 - 4; $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; or $-(CH_2)_n-N=C(NH_2)_2$, n = 0 - 4;
$R_{11}$ = $C_1$ - $C_8$ straight, branched or cyclic alkyl;
$-(CH_2)_n$-phenyl, n = 0 - 4, or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl.

**8.** A compound according to Claim 7, [R-(R*,R*)]-2,6-dibutyl-4-methyleneheptanedioic acid bis-(phenylmethyl)ester; [R-(R*,R*)]-$N^{\omega}$,$N^{\omega'}$-(2,6-dibutyl-4-methylene-1,7-dioxo-1,7-heptanediyl)bis[N-L-leucyl-L-phenylalanine]dimethyl ester [R-(R*,R*)]-$N^{\omega}$,-$N^{\omega'}$-(2,6-dibutyl-4-methylene-1,7-dioxo-1,7-heptanediyl)-bis[N-L-alanyl-L-alanine]dimethyl ester; [R-(R*,R*)]--$N^{\omega}$,$N^{\omega'}$-(2,6-dibutyl-4-methylene-1,7-dioxo-1,7-heptanediyl)bis[L-valyl-L-valine]dimethyl ester; [S-(R*,S*)] -1-[2-methylene-5-oxo-5-[2-oxo-4-(phenylmethyl)-3-oxazolidinyl]-4-(phenylmethyl)pentyl]-L-proline methyl ester; [R-(R*,R*,R*)]-N-[N-[1-[5-[[1-[[[1-(methoxycarbonyl)-2-methylpropyl]amino]carbonyl]-2-methylpropyl]amino]-2-methylene-5-oxo-4-(phenylmethyl)pentyl]-L-prolyl]-L-valyl]-L-valine methyl ester; [R-(R*,R*)]-4-methylene-2,6-bis-(phenylmethyl)heptanedioic acid bis(phenylmethyl)ester; [R-(R*,R*)]-$N^{\omega}$,$N^{\omega'}$-[4-methylene-1,7-dioxo-2,6-bis(phenylmethyl)-1,7-heptanediyl]bis[N-L-valyl-L-valine]dimethyl ester; [R-(R*,R*)]-$N^{\omega}$,$N^{\omega'}$-[4-methylene-1,7-dioxo-2,6-bis(phenylmethyl)-1,7-heptanediyl]bis[N-L-leucyl-L-phenylalanine] dimethyl ester; [R-(R*,R*)]-N,N'-bis(1,1-dimethylmethyl)-4-methylene-2,6-bis(phenylmethyl)heptanediamide; [R-(R*,R*)]-N,N'-dicyclopentyl-4-methylene-2,6-bis(phenylmethyl)heptanediamide; [R-(R*,R*)]-N,N'-bis-(cyclohexylmethyl)-4-methylene-2,6-bis(phenylmethyl)heptanediamide; [1S-[1α[2R*,6R*,7(1R*,2R*)],2β]]-N,N'-bis(2-aminocyclohexyl)-4-methylene-2,6-bis(phenylmethyl)heptanediamide; [1S-[1α[2R*,6*,7-(1R*,2R*)],2β]]-[[4-methylene-1,7-dioxo-2,-6-(phenylmethyl)-1,7-heptanediyl]bis(imino-2,1-cyclohexanediyl)]biscarbamic acid bis-(1,1-dimethylethyl)ester; [1S-[1α[2R*,6R*,7(1R*,2R*)],2β]]-4-methylene-2,-6-bis(phenylmethyl)-N,N'-bis[2-[(2-quinolinylcarbonyl)amino]cyclohexyl]heptanediamide; N,N'-(2-methylene-1,-3-propanediyl)bis[N-(phenylmethyl)glycine] diethyl ester; 2,2'-[(2-methylene-1,3-propanediyl)bis[(phenylmethyl)imino]]bis[N-(1,1-dimethylethyl)acetamide]; 1,-1'-(2-methylene-1,3-propanediyl)bis-2-piperidinecarboxylic acid dimethyl ester; 1,1'-(2-methylene-1,3-propanediyl)bis[N-(1,1-dimethylethyl)-2-piperidinecarboxamide]; N,N'-(2-methylene-1,3-propadiyl)bis[N-cyclohexylmethyl)-glycine] diethyl ester; 2,2'-(2-methylene-1,3-propanediyl)bis[1,2,3,4-tetrahydro-3-isoquinolinecarboxylic acid] dimethyl ester; or 2,2'-(2-methylene-1,3-propanediyl)bis[N-(1,1-dimethylethyl)-1,2,3,4-tetrahydro-3-isoquinolinecarboxamide].

**9.** Compounds of the formula:

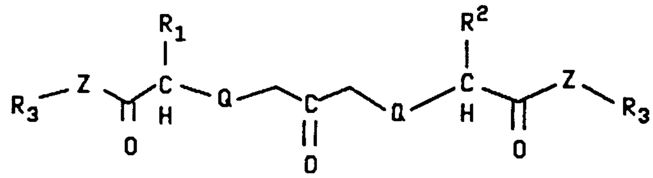

wherein:
Q is the same or different and is selected from a single bond or $NR_9$ or $NR_{10}$;
wherein:
$R_9$ = H, $C_1$ - $C_7$ straight or branched alkyl; $-(CH_2)_n$ cyclic $C_3$ - $C_7$ alkyl, n = 0 - 4; $-(CH_2)n$-phenyl, n = 0-4; or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; or $R_9$ and $R_2$ taken together are $-(CH_2)_m-$, m = 3 or 4; or $R_9$ and $R_1$ taken together are $-(CH_2)_m-$, m = 3 or 4; or $R_9$ and $R_2$ taken together are

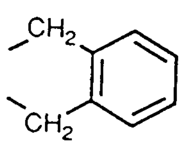

or $R_9$ and $R_1$ taken together are

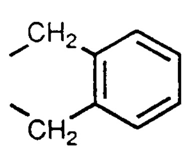

$R_{10}$ = H, $C_1$ - $C_7$ straight or branched alkyl; $-(CH_2)_n$ cyclic $C_3$ - $C_7$ alkyl, n = 0 - 4; $-(CH_2)_n$-phenyl, n = 0-4; or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; or $R_{10}$ and $R_2$ taken together are $-(CH_2)_m-$, m = 3 or 4; or $R_{10}$ and $R_1$ taken together are $-(CH_2)_m-$, m = 3 or 4; or $R_{10}$ and $R_2$ taken together are

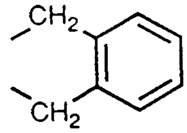

or $R_{10}$ and $R_1$ taken together are

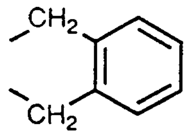

wherein:

Z = O or NH;

$R_1$ = H, $C_1$ - $C_7$ straight or branched alkyl; $-(CH_2)_n$ cyclic $C_3$ - $C_7$ alkyl, n = 0 - 4; $-(CH_2)_n$-phenyl, n=0-4; or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl, or phenyl; $R_2$ = H, $C_1$ - $C_7$ straight or branched alkyl; $-(CH_2)_n$ cyclic $C_3$ -$C_7$ alkyl, n = 0 - 4; $-(CH_2)_n$-phenyl, n=0-4; or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl, or phenyl;

$R_3$ =

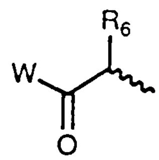

$C_1$ - $C_{10}$ straight or branched alkyl; $-(CH_2)_n$-cyclic $C_3$ - $C_7$ alkyl, n = 0 - 4; or $-(CH_2)_n$ -substituted cyclic $C_3$ - $C_7$ alkyl, where n = 0 - 4 and substituted with $C_1$ - $C_7$ straight, branched or cyclic alkyl, or $NHCOR_4$;

wherein:

$R_4$ = $UR_5$; U = a single bond, oxygen or NH; $R_5$ = $C_1$ - $C_8$ straight or branched alkyl; $-(CH_2)_n$ cyclic $C_3$ - $C_7$ alkyl, n = 0 - 4; $-(CH_2)_n$-phenyl, n = 0 - 4; $-(CH_2)_n$ substituted phenyl, where n = 0 - 4 and substituted with F,Cl,Br,I, $C_1$ - $C_4$ alkoxide, $C_1$ - $C_6$ straight, branched or cyclic alkyl; $-(CH_2)_n$ 1-napthyl, n = 0 - 4; $-(CH_2)_n$ 1-substituted napthyl, where n = 0 - 4 and substituted with F,Cl,Br,I, $C_1$ - $C_4$ alkoxide, $C_1$ - $C_6$ straight, branched or cyclic alkyl; $-(CH_2)_n$ 2-napthyl, n = 0 - 4; $-(CH_2)_n$ 2-substituted napthyl, where n = 0 - 4 and substituted with F,Cl,Br,I, $C_1$ - $C_4$ alkoxide, $C_1$ - $C_6$ straight, branched or cyclic alkyl; $-(CH_2)_n$ 2-quinoyl, n = 0 - 4; $-(CH_2)_n$ 2-substituted quinoyl, where n = 0 - 4 and substituted with F,Cl,Br,I, $C_1$ - $C_4$ alkoxide, $C_1$ - $C_6$ straight, branched or cyclic alkyl; $-(CH_2)_n$ heterocycle, where n = 0-4 and heterocycle is defined as a stable 5- to 7-membered mono- or bicyclic or a stable 7- to 10-membered bicyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and may include any bicyclic group in which any of the above defined heterocycles is fused to a benzene ring; $-(CH_2)_n$ substituted heterocycle, where n = 0-4, and substituted with F,Cl,Br,I, $C_1$ - $C_6$ alkoxide, $C_1$ - $C_6$ straight, branched or cyclic alkyl; $R_6$ = $C_1$ - $C_7$ straight, branched or cyclic alkyl;

$(CH_2)_2CONH_2$,$CH_2CONH_2$, $CH_2OH$, or $CH(CH_3)OH$;

W =

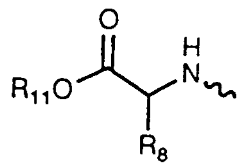

$OR_7$ or $NHR_7$, wherein: $R_7$ = $C_1$ - $C_8$ straight or branched alkyl; $-(CH_2)_n$ cyclic $C_3$ - $C_7$ alkyl, n = 0 - 4; $-(CH_2)_n$-phenyl, n = 0 - 4; $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; $-(CH_2)_n$ heterocycle, where n = 0-4 and heterocycle is as defined before; $-(CH_2)_n$ substituted heterocycle, where n = 0-4, heterocycle is as defined before and substituted with F,Cl,Br,I, $C_1$ - $C_4$ alkoxide, $C_1$ - $C_6$ straight, branched or cyclic alkyl; $R_8$ = $C_1$ - $C_7$ straight or branched alkyl; $-(CH_2)_n$ cyclic $C_3$ - $C_7$ alkyl, n = 0 - 4; $-(CH_2)_n$ -phenyl, n = 0 - 4; $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl; or $-(CH_2)_n-N=C(NH_2)_2$, n = 0 - 4;
$R_{11}$ = $C_1$ - $C_8$ straight, branched or cyclic alkyl; $-(CH_2)_n$-phenyl, n = 0 - 4, or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl.

**10.** A compound according to Claim 9 [R-(R*,R*)]-$N^{\omega}$,$N^{\omega'}$-[2,6-dibutyl-1,4,7-trioxo-1,7-pentanediyl)bis[N-L-leucyl-L-phenylalanine]dimethylester; [R-(R*,R*)]-$N^{\omega}$,$N^{\omega'}$-[2,6-dibutyl-1,4,7-trioxo-1,7-heptanediyl)bis[N-L-alanyl-L-alanine] dimethyl ester; [R-(R*,R*)]-$N^{\omega}$,$N^{\omega'}$-[2,6-dibutyl-1,4,7-trioxo-1,7-pentanediyl)bis[N-L-valyl-L-valine] dimethyl ester; [R-(R*,R*,R*)]-N-[N-[1-[5-[[1-[[[1-(methoxycarbonyl)-2-methylpropyl]amino]-carbonyl]-2-methylpropyl]amino]-2,5-dioxo-4-(phenylmethyl)pentyl]-L-prolyl]-L-valyl]-L-valine methyl ester; [R-(R*,R*)]-$N^{\omega}$,$N^{\omega'}$-[1,4,-7-trioxo-2,6-(phenylmethyl)-1,7-heptanediyl]bis[N-L-valyl-L-valine] dimethyl ester; [R-(R*,R*)]-$N^{\omega}$,$N^{\omega'}$-[1,-4,7-trioxo-2,6-bis(phenylmethyl)-1,7-heptanediyl]bis[N-L-leucyl-L-phenylalanine]dimethyl ester; [R-(R*,R*)]-N,N'-bis(1,1-dimethylethyl)-4-oxo-2,6-bis(phenylmethyl)-heptanediamide; [R-(R*,R*)]-N,N'-dicyclopentyl-4-oxo-2,6-bis(phenylmethyl)heptanediamide; [R-(R*,R*)]-N,N'-bis(cyclohexylmethyl)-4-oxo-2,6-bis(phenylmethyl)heptanediamide; [1S-[1α[2R*,6R*,7(1R*,-2R*)]-,2β]]-[1,4,7-trioxo-2,6-bis(phenylmethyl)-1,7-heptanediyl]bis(imino-2,1-cyclohexanediyl)bis carbamic acid bis(1,1-dimethylethyl)ester; or [1S-[1α[2R*,6R*,7-(1R*,2R*)],2β]]-4-oxo-2,6-bis-(phenylmethyl)-N,N'-bis[2-[(2-quinolinylcarbonyl)amino]cyclohexyl]-heptanediamide.

**11.** Compounds of the formula:

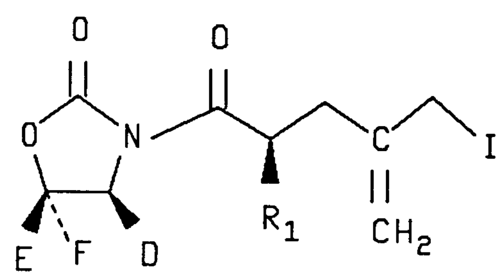

wherein:
D = $C_1$ - $C_7$ alkyl; $-(CH_2)_n$-phenyl, n = 0 - 4; E and F are the same or different and selected from H, $C_1$ - $C_7$alkyl and $-(CH_2)_n$-phenyl, n = 0 - 4;$R_1$ = H, $C_1$ - $C_7$ straight or branched alkyl; $-(CH_2)_n$ cyclic $C_3$ - $C_7$ alkyl, n = 0 - 4; $-(CH_2)_n$-phenyl, n=0-4; or $-(CH_2)_n$-substituted phenyl, where n = 0 - 4 and substituted with F,Cl,Br,I, $C_1$ - $C_4$ alkoxide, $C_1$ - $C_6$ straight, branched or cyclic alkyl or phenyl.

**12.** A compound according to Claim 11, [S-(R*,S*)]-3-[4-(iodomethyl)-1-oxo-2-(phenylmethyl)-4-pentenyl]-4-(phenylmethyl)-2-oxazolidinone.

**13.** Compounds according to Claim 7 of the formula:

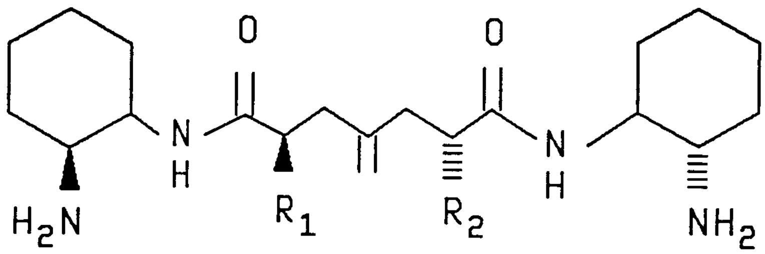

wherein:
$R_1 = R_2 = nC_4H_9$, $-CH_2$ phenyl or $-CH_2$ cyclohexyl.

**14.** A method of inhibiting the protease enzyme of an HIV retrovirus which comprises contacting said enzyme with an effective amount of a compound according to Claim 1.

**15.** A method for treating a mammal infected with an HIV retrovirus which comprises administering an effective amount of a compound according to Claim 1.

**16.** A pharmaceutical composition which comprises an effective amount of a compound according to Claim 1 in association with a pharmaceutically acceptable carrier.

**17.** A method for producing a compound of the formula:

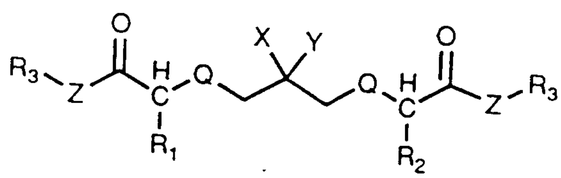

according to Claim 1, wherein X and Y are taken together to be an epoxide $-(CH_2O)-$ which comprises reacting a compound of the formula:

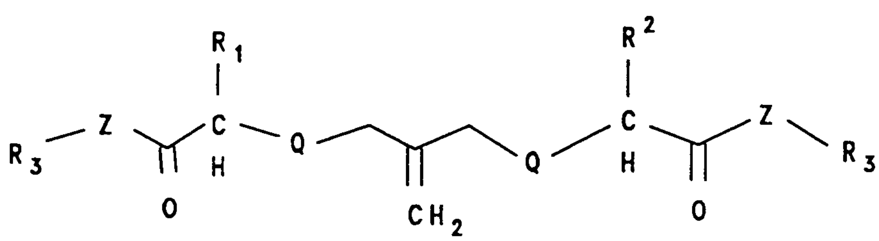

according to Claim 7, with m-chloroperbenzoic acid in methylene chloride at 0°C.

**18.** A method for producing a compound of the formula:

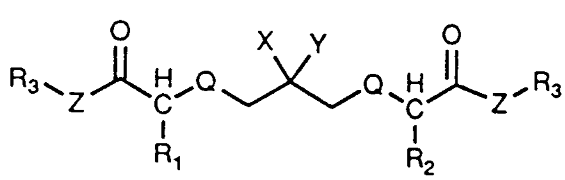

according to Claim 1, wherein X is OH and Y is $-CH_2OH$ which comprises reacting a compound of the formula:

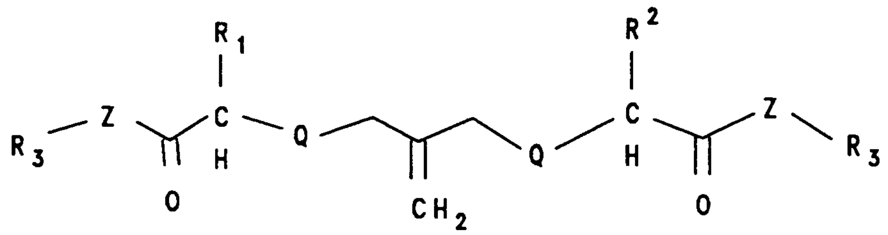

according to Claim 7 with osmium tetroxide.

**19.** A method for producing a compound of the formula:

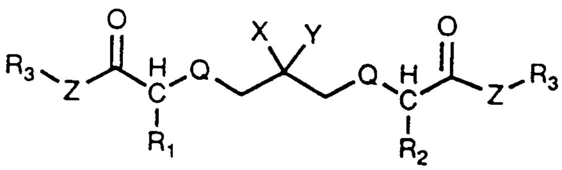

according to Claim 1, wherein X = OH and Y = H which comprises reacting:

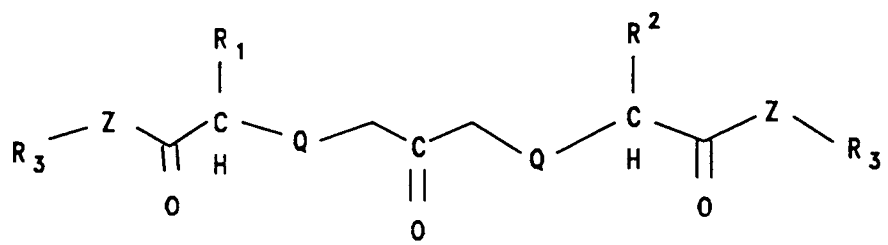

according to Claim 9, with sodium borohydride.

**20.** A method for producing a compound of the formula:

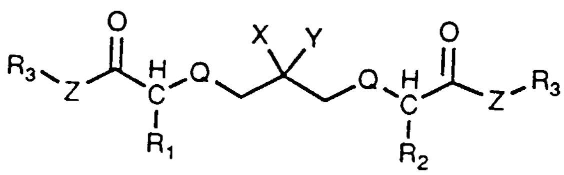

according to Claim 1, wherein X = OH and Y = H which comprises reacting a compound of the formula:

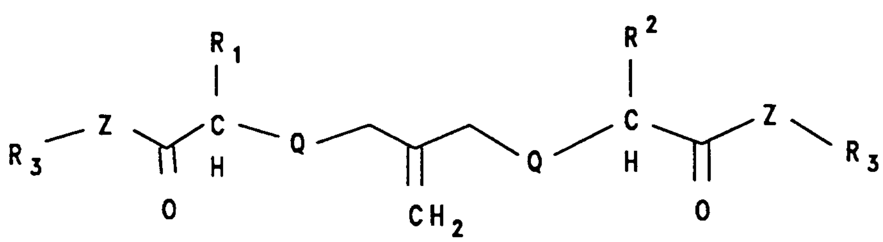

according to Claim 7, with osmium tetroxide in the presence of sodium periodate and reducing the product so formed with sodium borohydride.

**21.** A method for producing a compound of the formula:

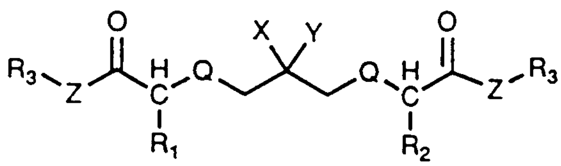

according to Claim 1, wherein X is OH and Y is H which comprises reacting a compound of the formula:

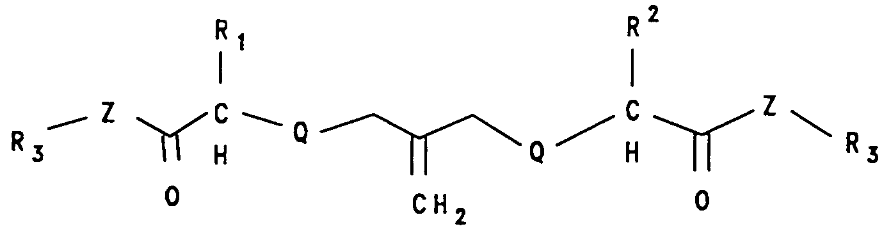

according to Claim 7, with osmium tetroxide and sodium periodate in 1 or 2 steps and reducing the product so formed with sodium borohydride.

**22.** The compounds according to Claims 1, 3, 5, 7 or 9 wherein said heterocycles include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, isoxazoyl, isoxazoyl, isoxazolidinyl, morpolinyl, thiazolyl, thiazolidinyl, isothiazolyl, quinuclidinyl, isothiazolidinyl, indolyl, quinolinyl, isoquinolinyl, benzimidazolyl, quinuclidinyl, isothiazolidinyl, indolyl, quinolinyl, isoquinolinyl, benzimidazolyl, thiadiazolyl, benzopyranyl, benzothiazolyl, benzoxazolyl, furyl, tetrahydrofuryl, tetrahydropyranyl, thienyl, benzothienyl, thiamorpholinyl, thiamorpholinyl sulfoxide, thiamorpholinyl sulfone and oxadiazolyl.